(19) Europäisches Patentamt
European Patent Office
Office européen des brevets

(11) **EP 4 068 292 A1**

(12) **EUROPEAN PATENT APPLICATION**
published in accordance with Art. 153(4) EPC

(43) Date of publication:
**05.10.2022 Bulletin 2022/40**

(21) Application number: **20892087.6**

(22) Date of filing: **25.11.2020**

(51) International Patent Classification (IPC):
*G16H 10/00* (2018.01)

(52) Cooperative Patent Classification (CPC):
**G16H 40/20; G16H 10/20; G16H 20/10;**
**G16H 20/30; G16H 20/60; G16H 50/20**

(86) International application number:
**PCT/CN2020/131561**

(87) International publication number:
**WO 2021/104324 (03.06.2021 Gazette 2021/22)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: 25.11.2019  CN 201911169008
30.09.2020  CN 202011063268
24.11.2020  CN 202011331863

(71) Applicant: **BOE Technology Group Co., Ltd.**
**Beijing 100015 (CN)**

(72) Inventors:
• **WEI, Wei**
**Beijing 100176 (CN)**
• **ZHAO, Lei**
**Beijing 100176 (CN)**
• **HE, Shuo**
**Beijing 100176 (CN)**

(74) Representative: **Hoffmann Eitle**
**Patent- und Rechtsanwälte PartmbB**
**Arabellastraße 30**
**81925 München (DE)**

(54) **MEDICAL INFORMATION PROCESSING METHOD, MEDICAL INFORMATION ACQUISITION METHOD AND MEDICAL INFORMATION EXCHANGE METHOD**

(57) Disclosed is a medical information exchange method, comprising: a medical information processing device displaying at least one navigation tab, and in response to a first operation of a user, displaying disease management information of a patient matching a target navigation tab, and transmitting the disease management information of the patient to a server; a medical information acquisition device acquiring the disease management information of the patient from the server; the medical information acquisition device displaying multiple role selection items; in response to a selection operation of the user regarding a role selection item, displaying a login information box corresponding to a user role, wherein login information boxes corresponding to different roles are different; and in response to login information entered into the login information box, displaying at least one functional tab corresponding to the user role, wherein at least one functional tab corresponding to a medical worker is configured to indicate the display of disease management information of multiple patients, and at least one functional tab corresponding to a patient is configured to indicate the display of disease management information of the patient.

FIG. 1

# Description

This application claims priority to Chinese Patent Application No. 201911169008.X, filed on November 25, 2019, Chinese Patent Application No. 202011063268.1, filed on September 30, 2020, and Chinese Patent Application No. 202011331863.9, filed on November 24, 2020, which are incorporated herein by reference in their entirety.

## TECHNICAL FIELD

The present disclosure relates to the technical field of medical information, and in particular, to a medical information processing method, a medical information acquisition method, and a medical information interaction method.

## BACKGROUND

Chronic diseases mainly refer to a series of diseases represented by cardiovascular and cerebrovascular diseases, diabetes, malignant tumors, etc., and have characteristics of long disease course, high mortality and disability rates, heavy economic burden, etc.

At present, there are approximately hundreds of millions of patients with the chronic diseases in our country. As a proportion of aging population increases year by year, a population base with potential chronic diseases continues to expand. Therefore, disease management has not only become a focus of attention in the medical and health field, but also become a social problem that needs urgent attention.

## SUMMARY

In an aspect, a medical information processing method is provided. The method includes: displaying at least one navigation tab; and in response to a first operation of a user, determining a target navigation tab, and displaying management information of a disease of a patient matched with the target navigation tab, the target navigation tab being one of the at least one navigation tab.

In some embodiments, the at least one navigation tab includes a patient management tab; and in response to the first operation of the user, determining the target navigation tab, and displaying the management information of the disease of the patient matched with the target navigation tab include: displaying at least a patient list in response to the first operation of the user on the patient management tab, the patient list including visit information of each patient of at least one patient and at least one medical operation index item corresponding to the patient, and the at least one medical operation index item including an index item for creating a treatment method of the disease of the patient.

In some embodiments, the at least one medical operation index item includes a health prescription creation index item; and the method further includes: displaying at least a health prescription text box in response to an operation of the user on a health prescription creation index item corresponding to a target patient, the health prescription text box being a blank box; and presenting a medical regimen matched with a disease of the target patient in the health prescription text box in response to a second operation of the user, the medical regimen including at least one of a medication regimen, a diet regimen or an exercise regimen.

In some embodiments, the medical information processing method further includes: displaying an entry sub-tab. Presenting the medical regimen matched with the disease of the target patient in the health prescription text box in response to the second operation of the user includes: presenting a first classification list in response to an operation of the user on the entry sub-tab, the first classification list including a plurality of first-level items and at least one disease type included in each first-level item, and the plurality of first-level items including a medication regimen item, a diet regimen item, and an exercise regimen item; in response to an operation of the user on a disease type corresponding to the disease of the target patient in a first-level item in the first classification list, presenting at least one entry text template corresponding to the disease type in the first-level item; and in response to an operation of the user on an entry text template, presenting content of the entry text template in the health prescription text box.

In some embodiments, the medical information processing method further includes: displaying at least one entry template setup item, the at least one entry template setup item including at least one of an addition item, an editing item, and a deletion item, the addition item being configured to instruct to add a new entry text template, the editing item being configured to instruct to edit at least one entry text template, and the deletion item being configured to instruct to delete at least one entry text template.

In some embodiments, the medical information processing method further includes: displaying a health prescription template sub-tab. Presenting the medical regimen matched with the disease of the target patient in the health prescription text box in response to the second operation of the user includes: presenting a second classification list in response to an operation of the user on the health prescription template sub-tab, the second classification list including at least one disease type; in response to an operation of the user on a disease type corresponding to the disease of the target patient in the second classification list, further presenting at least one medical regimen template corresponding to the disease type; and in response to an operation of the user on a medical regimen template, presenting at least part of the content of the medical regimen template in the health prescription text box.

In some embodiments, the medical information processing method further includes: displaying a health prescription template setup item, the health prescription template setup item being configured to instruct to edit at least one medical regimen template, and to add content of the edited medical regimen template as a new medical regimen template; or to update content of the medical regimen template before editing to the content of the edited medical regimen template.

In some embodiments, the medical information processing method further includes: displaying a historical prescription sub-tab. Presenting the medical regimen matched with the disease of the target patient in the health prescription text box in response to the second operation of the user includes: presenting a historical prescription list associated with the disease of the target patient in response to an operation of the user on the historical prescription sub-tab; presenting content of a historical prescription in the historical prescription list in response to an operation of the user on the historical prescription list; and presenting the content of the historical prescription in the health prescription text box in response to an operation of the user on the historical prescription.

In some embodiments, the medical information processing method further includes: displaying an item of saving as a template, the item of saving as a template being configured to save the medical regimen presented in the health prescription text box as a new medical regimen template.

In some embodiments, the at least one medical operation index item includes a follow-up creation index item, and the medical information processing method further includes: determining a follow-up task matched with a disease of a target patient in response to an operation of the user on a follow-up creation index item corresponding to the target patient and a third operation of the user, the follow-up task including follow-up content matched with the disease of the target patient and a follow-up time corresponding to the follow-up content.

In some embodiments, the follow-up content is a follow-up record form; and the method further includes: in response to an operation of opening a scale making tool interface, displaying the scale making tool interface, the scale making tool interface including a field display region and an editing region, the field display region including a plurality of fields, the editing region being used to make the follow-up record form, a follow-up record form to be made including a plurality of target elements, and each target element being generated by using a field; and making the follow-up record form in the editing region in response to an operation of making the follow-up record form.

In some embodiments, making the follow-up record form in the editing region in response to the operation of making the follow-up record form includes: displaying a selected field in the editing region in response to a field selection operation of the user in the scale making tool interface; and repeatedly responding to the field selection operation until fields corresponding to a plurality of target elements required by the follow-up record form are all displayed in the editing region to generate the follow-up record form.

In some embodiments, the plurality of fields include at least one basic field, and the basic field includes at least one attribute to be edited.

In a case where the field selected through the field selection operation is a basic field, after the selected field is displayed in the editing region, the method further includes:

displaying an attribute menu of the basic field in the editing region in response to the field selection operation, the attribute menu including at least one attribute editing box; and
displaying an edited attribute in each attribute editing box to generate a target element in response to a basic field attribute editing operation entered by the user in the at least one attribute editing box.

In some embodiments, the attribute menu further includes a button of saving as a frequently-used field; and the method further includes: displaying the generated target element in the field display region as a frequently-used field in response to an operation of the user selecting the button of saving as a frequently-used field.

In some embodiments, the plurality of fields include at least two basic fields, the at least two basic fields include first basic fields and second basic fields, a first basic field is configured to generate a first target element, a second basic field is configured to generate a second target element, and there is a corresponding relationship between the first target element and the second target element; and at least one attribute editing box of the first basic field includes a data source attribute editing box; displaying the edited attribute in each attribute editing box in response to the basic field attribute editing operation entered by the user in the at least one attribute editing box includes: displaying a data source attribute representing the corresponding relationship in the data source attribute editing box of the first basic field in response to the basic field attribute editing operation; and the method further includes: displaying a follow-up task performance interface in response to an operation of opening follow-up task performance, the follow-up task performance interface including the follow-up record form included in the follow-up task created for the target patient, the follow-up record form including the first target element and the second target element, and the first target element and the second target element each having an editing box; and receiving a thirteenth operation entered in an editing box of the second target element, the thirteenth operation being used to: in response to an operation of entering target content in the editing box of the second target element, display the target content in the editing box of the second target element, and automatically display content obtained according to the corresponding relationship represented by the data source attribute in an editing box of the first target element.

In some embodiments, the plurality of fields include third basic fields, a third basic field is configured to generate a third target element, and at least one attribute editing box of the third basic field includes a data source attribute editing box; displaying the edited attribute in each attribute editing box in response to the basic field attribute editing operation entered by the user in the at least one attribute editing box includes: displaying a data source attribute linked to a background database in the data source attribute editing box of the third basic field in response to the basic field attribute editing operation; and the method further includes: displaying a follow-up task performance interface in response to an operation of performing the follow-up task, the follow-up task performance interface including the follow-up record form included in the follow-up task created for the target patient, the follow-up record form including the third target element, and the third target element having an editing box; and automatically displaying content in the background database linked to the data source attribute of the third target element in the editing box of the third target element in response to an operation of the user of selecting the editing box of the third target element.

In some embodiments, the plurality of fields include at least one frequently-used field, and the frequently-used field includes at least one edited attribute; and in a case where a field selected through a field selection operation is a frequently-used field, after the selected field is displayed in the editing region, the method further includes: using the frequently-used field displayed in the editing region as a target element in response to the field selection operation of the user.

In some embodiments, the plurality of fields include a plurality of frequently-used fields, and the plurality of frequently-used fields include at least one public frequently-used field and at least one private frequently-used field; and the public frequently-used field may be used as a target element in at least two follow-up record forms to be made, and the private frequently-used field may be used as a target element in one follow-up record form to be made.

In some embodiments, the scale making tool interface further includes a form region and a submission column; and the form region is configured to display a name of a generated follow-up record form, and the submission column includes a form generation button; and the method further includes: displaying a name of a saved follow-up record form in the form region in response to an operation of the user of selecting the form generation button.

In some embodiments, the submission column further includes a preview button; and before the name of the saved follow-up record form is displayed in the form region in response to the operation of the user of selecting the form generation button, the method further includes: displaying a generated form in the editing region in response to an operation of selecting the preview button.

In some embodiments, the operation of selecting the preview button is further configured to select a preview effect of a form, and the preview effect includes a paper effect and an effect displayed on a screen of a terminal; and displaying the generated form in the editing region in response to the operation of the user of selecting the preview button includes: in response to the operation of selecting the preview button, displaying the generated follow-up record form in the editing region in the paper effect, or displaying the generated follow-up record form in the editing region in the effect displayed on a screen of a mobile terminal.

In some embodiments, the form region includes a list sub-region and an operation sub-region; the list sub-region includes a name of at least one saved follow-up record form, and the saved form includes a plurality of set elements; and the operation sub-region includes at least one import reference option, and each saved form corresponds to an import reference option; and the method further includes: in response to an operation of selecting an import reference option, displaying a saved follow-up record form corresponding to the selected import reference option in the editing region, and using the follow-up record form as a basic form; in response to a modification operation of the user, setting modified at least one set element to generate a target element, the modification operation being configured to modify at least one set element of a plurality of set elements in the basic form, and the at least one set element being not the same as the target element in the follow-up record form to be made; and repeatedly responding to the modification operation until the plurality of set elements in the basic form are the same as the plurality of target elements in the follow-up record form to be made to generate a new follow-up record form.

In some embodiments, the medical information processing method further includes: displaying at least one follow-up task template. Determining the follow-up task matched with the disease type in response to the third operation of the user includes: in response to an operation of the user on a follow-up task template, determining the follow-up task template as the follow-up task matched with the disease type.

In some embodiments, the method further includes: creating a follow-up template, the follow-up template including the at least one follow-up task template.

In some embodiments, creating the follow-up template includes:

displaying a follow-up template interface, the follow-up template interface including a template selection box, and the template selection box including a plurality of disease options; displaying a disease option menu in response to an operation of creating the follow-up template; and in response to an operation of the user of selecting a disease option, determining a target disease, and creating a follow-up template matched with the target disease, the follow-up template including a plurality of follow-up record forms and a time interval corresponding to each follow-up record form, each of the plurality of follow-up record forms being a follow-up record form corresponding to the target disease, and the time interval being a time interval between a follow-up date set for the target disease and a date when a follow-up task is created.

In some embodiments, the follow-up template interface further includes a template content column, the template content column includes a new button, and the template content column is configured to display a created follow-up template; and creating the follow-up template matched with the target disease includes: displaying a follow-up template creation dialog box in the follow-up template interface in response to an operation of selecting the new button, the follow-up template creation dialog box including a follow-up record form editing box, a time interval editing box, an OK button and a cancel button, the follow-up record form editing box having at least one form option related to the target disease, and each form option corresponding to a preset follow-up record form; in response to an operation of the user of selecting a form option, displaying a name of a follow-up record form corresponding to the selected form option in the follow-up record form editing box; displaying a set time interval in the time interval editing box in response to an operation of the user of entering a time interval; and displaying the follow-up template in the template content column in response to an operation of the user of selecting the OK button. In some embodiments, the medical information processing method further includes: displaying at least one preset time option and a follow-up content selector corresponding to each preset time option. Determining the follow-up task matched with the disease type in response to the third operation of the user includes: in response to selection of the user of a preset time option and selection of the user of a follow-up content selector corresponding to the preset time option, determining follow-up content in the selected follow-up content selector and a follow-up time in the preset time option as the follow-up task matched with the disease type.

In some embodiments, the medical information processing method further includes: displaying a time selector and a follow-up content selector corresponding to the time selector. Determining the follow-up task matched with the disease type in response to the third operation of the user includes: in response to selection of the user of the time selector and the follow-up content selector corresponding to the time selector, determining follow-up content in the selected follow-up content selector and a follow-up time in the time selector as the follow-up task matched with the disease type.

In some embodiments, the medical information processing method further includes: displaying a disease type selector; and determining the disease type corresponding to the disease of the target patient in response to an operation of the user on the disease type selector.

In some embodiments, the medical information processing method further includes: displaying a follow-up person selector; and associating a follow-up person with the follow-up task in response to an operation of the user on the follow-up person selector.

In some embodiments, the medical information processing method further includes: displaying a card registration and creation index item; displaying at least basic information blank text boxes and a diagnosis information blank text box in response to an operation of the user on the card registration and creation index item, the basic information blank text boxes being configured to present basic information of a new patient, and the diagnosis information blank text box being configured to present diagnosis information of the new patient; and in response to an operation of the user of saving information presented in the basic information blank text boxes and the diagnosis information blank text box, adding at least part of the information presented in the basic information blank text boxes and the diagnosis information blank text box as visit information into the patient list.

In some embodiments, the at least one navigation tab includes a health prescription management tab; in response to the first operation of the user, determining the target navigation tab, and displaying the management information of the disease of the patient matched with the target navigation tab include: displaying a health prescription list in response to the first operation of the user on the health prescription management tab, the health prescription list including at least one line of prescription information, each line of prescription information including visit information of a patient and at least one first medical information operation item, and the at least one first medical information operation item including a second detail index item; and the method further includes: displaying a health prescription text box in response to an operation of the user on the second detail index item, a medical regimen corresponding to the line of prescription information being presented in the health prescription text box, and the medical regimen including at least one of a medication regimen, a diet regimen or an exercise regimen.

In some embodiments, each line of prescription information further includes state information, and the state information includes one of being valid, being deactivated, or being invalid; in a case where state information in a line of prescription information is being valid, at least one first medical information operation item in the line of prescription information further includes a deactivation item and an invalidation item; and the method further includes: in response to an operation of the user on the deactivation item or the invalidation item, changing the state information in the line of prescription information from being valid to being deactivated or being invalid, and hiding the deactivation item and the invalidation item.

In some embodiments, the medical information processing method further includes: displaying a new health prescription creation index item; displaying a patient search box and a health prescription text box in response to an operation of the user on the new health prescription creation index item, the health prescription text box being a blank box; in response to an operation of querying information entered in the patient search box, determining a target patient, and displaying visit information of the target patient; presenting a medical regimen matched with a disease of the target patient in the health prescription text box in response to a second operation of the user; and in response to an operation of the user of saving the medical regimen, adding the visit information of the target patient as part of information in a new line of prescription information into the health prescription list, and associating the medical regimen of the target patient in a second detail index item in the new line of prescription information.

In some embodiments, the medical information processing method further includes: displaying a card registration and creation index item; displaying basic information blank text boxes and a diagnosis information blank text box in response to an operation of the user on the card registration and creation index item, the basic information blank text boxes being configured to present basic information of a new patient, and the diagnosis information blank text box being configured to present diagnosis information of the new patient; and in response to an operation of the user of saving information presented in the basic information blank text boxes and the diagnosis information blank text box, adding part of the information presented in the basic information blank text boxes and the diagnosis information blank text box as visit information into the patient list.

In some embodiments, the at least one navigation tab includes a follow-up management tab; and in response to the first operation of the user, determining the target navigation tab, and displaying the management information of the disease of the patient matched with the target navigation tab include: displaying a follow-up task list in response to the first operation of the user on the follow-up management tab, the follow-up task list including at least one line of follow-up information, and each line of follow-up information including visit information of a patient, a follow-up time, follow-up content, and at least one second medical information operation item.

In some embodiments, each line of follow-up information further includes a follow-up state, and the follow-up state includes one of no follow-up and being completed; in a case where a follow-up state in a line of follow-up information is no follow-up, the at least one second medical information operation item in the line of follow-up information further includes a follow-up performance index item; and the method further includes: displaying a follow-up record form corresponding to follow-up content in response to an operation of the user on the follow-up performance index item; and in response to an operation of saving content entered in the follow-up record form, changing the follow-up state in the line of follow-up information from no follow-up to being completed, and changing the follow-up performance index item to a third detail index item.

In some embodiments, the at least one second medical information operation item further includes a follow-up comparison index item; and the method further includes: in response to an operation of the user on the follow-up comparison index item, acquiring and displaying comparison results of at least two follow-up contents matched with a same disease corresponding to the patient in the line of follow-up information.

In some embodiments, the at least one second medical information operation item further includes a follow-up creation index item; and the method further includes: determining a new follow-up task matched with a disease type corresponding to a disease of a target patient in response to an operation of the user on the follow-up creation index item and a third operation of the user, the new follow-up task including follow-up content matched with the disease of the patient and a follow-up time corresponding to the follow-up content; and adding the new follow-up task into the follow-up task list in response to an operation of the user of saving the new follow-up task.

In some embodiments, the medical information processing method further includes: displaying a new follow-up creation index item; displaying a patient search box in response to an operation of the user on the new follow-up creation index item; in response to an operation of querying information entered in the patient search box, determining a target patient, and displaying visit information of the target patient; determining a new follow-up task matched with the disease of the target patient in response to a third operation of the user, the new follow-up task including follow-up content matched with the disease of the target patient and a follow-up time corresponding to the follow-up content; and adding the visit information of the target patient and the new follow-up task into the follow-up task list in response to an operation of the user of saving the new follow-up task.

In some embodiments, the medical information processing method further includes: displaying a card registration and creation index item; displaying basic information blank text boxes and a diagnosis information blank text box in response to an operation of the user on the card registration and creation index item, the basic information blank text boxes being configured to present basic information of a new patient, and the diagnosis information blank text box being configured to present diagnosis information of the new patient; and in response to an operation of the user of saving information presented in the basic information blank text boxes and the diagnosis information blank text box, adding part of the information presented in the basic information blank text boxes and the diagnosis information blank text box as visit information into a patient list.

In some embodiments, the medical information processing method further includes: displaying a login information box; and

displaying work content and workload of the user on a current working day in response to an operation of logging in login information entered in the login information box.

In some embodiments, the at least one navigation tab includes a disease assessment tab; and in response to the first operation of the user, determining the target navigation tab, and displaying the management information of the disease of the patient matched with the target navigation tab include: displaying a disease assessment information text box in response to an operation of the user on the disease assessment tab; and displaying a disease assessment report corresponding to disease assessment information in response to an operation of saving information entered in the disease assessment information text box.

In some embodiments, the at least one navigation tab includes a smart question and answer tab; and in response to the first operation of the user, determining the target navigation tab, and displaying the management information of the disease of the patient matched with the target navigation tab include: displaying a question search box in response to an operation of the user on the smart question and answer tab; and in response to an operation of querying a question entered in the question search box, displaying at least one answer associated with the question.

In some embodiments, the at least one navigation tab further includes a consultation tab; and in response to the first operation of the user, determining the target navigation tab, and displaying the management information of the disease of the patient matched with the target navigation tab include: displaying a consultation list in response to an operation of the user on the consultation tab, the consultation list including at least one line of consultation information from a server, and each line of consultation information including visit information of a patient, a question summary, a consultation type, and a fourth detail index item; and displaying at least a consultation question corresponding to the line of consultation information in response to an operation of the user on the fourth detail index item.

In some embodiments, each line of consultation information further includes a reply state, and the reply state includes one of being answered and being unanswered; and in a case where a reply state in a line of consultation information is being unanswered, the method further includes: further displaying a reply text box matched with the consultation type in the line of consultation information in response to the operation of the user on the fourth detail index item; presenting an answer matched with the consultation question in response to an operation of the user on the reply text box; and changing the reply state in the line of consultation information from being unanswered to being answered in response to an operation of the user of saving the answer.

In some embodiments, the consultation type includes report interpretation; and the reply text box includes an abnormal question summary text box and a health advice text box; and the method further includes: further displaying at least one examination report in response to the operation of the user on the fourth detail index item. Presenting the answer matched with the consultation question in response to the operation of the user on the reply text box includes: presenting an abnormal question corresponding to the at least one examination report and interpretation text of the abnormal question in response to an operation of the user on the abnormal question summary text box; and presenting a medical regimen matched with the abnormal question in response to an operation of the user on the health advice text box, the medical regimen including at least one of a medication regimen, a diet regimen or an exercise regimen.

In some embodiments, the medical information processing method further includes: displaying an abnormal question template tab. Presenting the abnormal question corresponding to the at least one examination report and the interpretation text of the abnormal question in response to the operation of the user on the abnormal question summary text box includes: displaying a third classification list in response to an operation of the user on the abnormal question template tab, the third classification list including at least one question type; in response to an operation of the user on a question type corresponding to the abnormal question in the third classification list, displaying the abnormal question corresponding to the question type and at least one interpretation text template corresponding to the abnormal question; and in response to an operation of the user on an interpretation text template, presenting content of the interpretation text template and the abnormal question in the abnormal question summary text box.

In some embodiments, the medical information processing method further includes: displaying a health advice template tab. Presenting the medical regimen matched with the abnormal question in response to the operation of the user on the health advice text box includes: displaying an entry sub-tab in response to an operation of the user on the health advice template tab; displaying a first classification list in response to an operation of the user on the entry sub-tab, the first classification list including a plurality of first-level items and at least one disease type included in each first-level item, and the plurality of first-level items including a medication regimen item, a diet regimen item, and an exercise regimen item; in response to an operation of the user on a disease type corresponding to the abnormal question in a first-level item in the first classification list, displaying at least one entry text template corresponding to the disease type in the first-level item; and in response to an operation of the user on an entry text template, presenting content of the entry text template in the health advice text box; and/or presenting the medical regimen matched with the abnormal question in response to the operation of the user on the health advice text box includes: displaying a health prescription template sub-tab in response to the operation of the user on the health advice template tab; displaying a second classification list in response to an operation of the user on the health prescription template sub-tab, the second classification list including at least one disease type; displaying at least one medical regimen template corresponding to the disease type in response to an operation of the user on the disease type corresponding to the abnormal question in the second classification list; and in response to an operation of the user on a medical regimen template, presenting at least part of content of the medical regimen template in the health advice text box.

In some embodiments, the at least one navigation tab includes a statistical tab; and in response to the first operation of the user, determining the target navigation tab, and displaying the management information of the disease of the patient matched with the target navigation tab include: displaying statistical information in response to an operation of the user on the statistical tab, the statistical information being information obtained by performing classified statistics on the patient and the management information of the disease of the patient.

In some embodiments, the medical information processing method further includes: displaying a knowledge base management tab; displaying an abnormal question template tab and a health advice template tab in response to an operation of the user on the knowledge base management tab; displaying a question node setup item and an interpretation text template setup item in response to an operation of the user on the abnormal question template tab, the question node setup item being configured to instruct to set question types, the interpretation text template setup item being configured to instruct to add a new interpretation text template, to instruct to edit the interpretation text template, and/or to instruct to delete the interpretation text template; displaying an entry sub-tab in response to an operation of the user on the health advice template tab; and displaying a first node setup item and an entry text template setup item in response to an operation of the user on the entry sub-tab, the first node setup item being configured to instruct to set item types of health advice and a disease type matched with each item type, the item types of the health advice including a medication regimen item, a diet regimen item, and an exercise regimen item, and the entry text template setup item being configured to instruct to add a new entry text template, to instruct to edit the entry text template, and/or to instruct to delete the entry text template; and/or displaying a health prescription template sub-tab in response to the operation of the user on the health advice template tab; and displaying a second node setup item and a health prescription template setup item in response to an operation of the user on the health prescription template sub-tab, the second node setup item being configured to instruct to set disease types, the health prescription template setup item being configured to instruct to add a new medical regimen template, to instruct to edit the medical regimen template, and/or to instruct to delete the medical regimen template.

In another aspect, a medical information acquisition method is provided. The method includes: displaying a plurality of role selection items, the plurality of role selection items including a medical worker and a patient; in response to an operation of a user of selecting a role selection item, determining a role of the user, and displaying a login information box corresponding to the role of the user, login information boxes corresponding to different roles being different; and displaying at least one function tab corresponding to the role of the user in response to login information of the user entered in the login information box, at least one function tab corresponding to the medical worker being configured to instruct to display management information of diseases of a plurality of patients, and at least one function tab corresponding to the patient being configured to instruct to display management information of a disease of the patient.

In some embodiments, the role of the user is the medical worker, and the at least one function tab includes a work schedule tab; and the method further includes: displaying at least one work item index item of the user in response to an operation of the user on the work schedule tab, the work item index item including at least one of a question consultation index item, a report interpretation index item, and a follow-up plan index item; and in response to an operation of the user on a work item index item, acquiring and displaying work content corresponding to the work item index item of the user from a server.

In some embodiments, the medical information acquisition method further includes: acquiring an amount of work content corresponding to the work item index item of the user on a target working day from the server, and displaying the amount of work content corresponding to the work item index item on the work item index item.

In some embodiments, the medical information acquisition method further includes: displaying a working day option; and determining the target working day in response to an operation of the user on the working day option.

In some embodiments, the at least one function tab further includes a statistical query tab; and the method further includes: acquiring and displaying at least statistical information of follow-up condition of the plurality of patients from the server in response to an operation of the user on the statistical query tab, the statistical information of the follow-up condition of the plurality of patients being statistical information of health condition of the patients obtained after at least one follow-up is performed on the patients.

In some embodiments, the role of the user is the patient, and the at least one function tab includes a disease assessment tab; and the method further includes: displaying at least one disease assessment question in response to an operation of the user on the disease assessment tab; and acquiring and displaying a disease assessment result of the user from a server in response to an answer of the user to each disease assessment question.

In some embodiments, the role of the user is the patient, and the at least one function tab includes a smart question and answer tab; and the method further includes: displaying a question search box in response to an operation of the user on the smart question and answer tab; and in response to an operation of the user of querying a question entered in the question search box, acquiring and displaying at least one answer associated with the question from a server.

In some embodiments, the role of the user is the patient, and the at least one function tab includes a consultation tab; and the method further includes: displaying a basic information text box and a disease condition description text box in response to an operation of the user on the consultation tab; in response to an operation of the user of submitting information of the patient entered in the basic information text box and a consultation question entered in the disease condition description text box, transmitting the information in the basic information text box and the consultation question in the disease condition description text box to a server; and acquiring and displaying an answer matched with the consultation question from the server.

In some embodiments, the method further includes: displaying an attachment addition item; and adding at least one attachment in response to an operation of the user on the attachment addition item. In response to the operation of the user of submitting the information of the patient entered in the basic information text box and the consultation question entered in the disease condition description text box, transmitting the information in the basic information text box and the consultation question in the disease condition description text box to the server includes: in response to an operation of the user of submitting the information of the patient entered in the basic information text box, the consultation question entered in the disease condition description text box, and the at least one attachment, transmitting the information in the basic information text box, the consultation question in the disease condition description text box, and the at least one attachment to the server.

In some embodiments, the method further includes: displaying a doctor selector; and determining a doctor to be consulted in response to an operation of the user on the doctor selector. In response to the operation of the user of submitting the information of the patient entered in the basic information text box and the consultation question entered in the disease condition description text box, transmitting the information in the basic information text box and the consultation question in the disease condition description text box to the server includes: in response to the operation of the user of submitting the information of the patient entered in the basic information text box and the consultation question entered in the disease condition description text box, transmitting the information in the basic information text box, the consultation question in the disease condition description text box, and a doctor to be consulted corresponding to the consultation question to the server.

In some embodiments, the role of the user is the patient, and the at least one function tab includes a health data statistical tab; and the method further includes: acquiring and displaying a classified statistical result of the management information of the disease of the patient from the server in response to an operation of the user on the health data statistical tab.

In some embodiments, the plurality of role selection items further include a manager, and the at least one function tab includes a statistical query tab; and the method further includes: displaying a query item in response to an operation of the user on the statistical query tab, the query item being configured to instruct to query the classified statistical result, and the classified statistical result being obtained according to classified statistics of data related to the patient and a doctor stored in the server.

In a third aspect, a medical information interaction method is provided. The method includes: displaying, by a medical information processing apparatus, at least one navigation tab, and in response to a first operation of a user, determining a target navigation tab, and displaying management information of a disease of a patient matched with the target navigation tab; transmitting, by the medical information processing apparatus, the management information of the disease of the patient to a server; acquiring, by a medical information acquisition apparatus, the management information of the disease of the patient from the server; and displaying, by the medical information acquisition apparatus, a plurality of role selection items, the plurality of role selection items including a medical worker and a patient; in response to an operation of the user of selecting a role selection item, determining, by the medical information acquisition apparatus, a role of the user, and displaying, by the medical information acquisition apparatus, a login information box corresponding to the role of the user, login information boxes corresponding to different roles being different; and displaying, by the medical information acquisition apparatus, at least one function tab corresponding to the role of the user in response to login information of the user entered in the login information box, at least one function tab corresponding to the medical worker being configured to instruct to display management information of diseases of a plurality of patients, and at least one function tab corresponding to the patient being configured to instruct to display management information of a disease of the patient.

In a fourth aspect, a medical information processing apparatus is provided. The medical information processing apparatus includes a first display and a first processor coupled to the first display. The first processor is configured to: control the first display to display at least one navigation tab; and in response to a first operation of a user, determine a target navigation tab, and control the first display to display management information of a disease of a patient matched with the target navigation tab, the target navigation tab being one of the at least one navigation tab.

In some embodiments, the medical information processing apparatus further includes a first input device coupled to the first processor, and the first input device is configured to enter the first operation of the user.

In a fifth aspect, a medical information acquisition apparatus is provided. The medical information acquisition apparatus includes a second display and a second processor coupled to the second display. The second processor is configured to: control the second display to display a plurality of role selection items, the plurality of role selection items including a medical worker and a patient; in response to an operation of a user of selecting a role selection item, determine a role of the user, and control the second display to display a login information box corresponding to the role of the user, login information boxes corresponding to different roles being different; and control the second display to display at least one function tab corresponding to the role of the user in response to login information of the user entered in the login information box, at least one function tab corresponding to the medical worker being configured to instruct to display management information of diseases of a plurality of patients, and at least one function tab corresponding to the patient being configured to instruct to display management information of a disease of the patient.

In some embodiments, the medical information acquisition apparatus further includes a second input device coupled to the second processor, and the second input device is configured to enter the operation of the user of selecting a role selection item, and to enter the login information of the user.

In a sixth aspect, a computer equipment is provided. The computer equipment includes a memory and a processor. The memory stores computer programs that, when executed on the processor, cause the processor to implement the medical information processing method, the medical information acquisition method, and/or the medical information interaction method described above.

In a seventh aspect, a non-transitory computer-readable storage medium is provided. The non-transitory computer-readable storage medium stores computer programs that, when executed on the processor, cause the processor to perform one or more steps in the medical information processing method according to any one of the above embodiments, one or more steps in the medical information acquisition method according to any one of the above embodiments, and/or one or more steps in the medical information interaction method according to any one of the above embodiments.

## BRIEF DESCRIPTION OF THE DRAWINGS

In order to describe technical solutions in the present disclosure more clearly, accompanying drawings to be used in some embodiments of the present disclosure will be introduced briefly below. Obviously, the accompanying drawings to be described below are merely accompanying drawings of some embodiments of the present disclosure, and a person of ordinary skill in the art may obtain other drawings according to these drawings. In addition, the accompanying drawings to be described below may be regarded as schematic diagrams, and are not limitations on actual sizes of products, actual processes of methods and actual timings of signals to which the embodiments of the present disclosure relate.

FIG. 1 is a flow diagram of a medical information processing method, in accordance with some embodiments;

FIG. 2 is a schematic diagram of a patient list interface, in accordance with some embodiments;

FIG. 3 is a schematic diagram of a health prescription creation interface, in accordance with some embodiments;

FIG. 4 is a schematic diagram of another health prescription creation interface, in accordance with some embodiments;

FIG. 5 is a schematic diagram of yet another health prescription creation interface, in accordance with some embodiments;

FIG. 6 is a schematic diagram of a follow-up creation interface, in accordance with some embodiments;

FIG. 7 is a schematic diagram of another follow-up creation interface, in accordance with some embodiments;

FIG. 8 is a schematic diagram of a medical record card creation interface, in accordance with some embodiments;

FIG. 9 is a schematic diagram of a health prescription management interface, in accordance with some embodiments;

FIG. 10 is a schematic diagram of yet another health prescription creation interface, in accordance with some embodiments;

FIG. 11 is a schematic diagram of a follow-up management interface, in accordance with some embodiments;

FIG. 12 is a schematic diagram of a follow-up task performance interface, in accordance with some embodiments;

FIG. 13 is a schematic diagram showing a follow-up comparison result, in accordance with some embodiments;

FIG. 14 is a schematic diagram of another follow-up comparison result, in accordance with some embodiments;

FIG. 15 is a schematic diagram of yet another follow-up creation interface, in accordance with some embodiments;

FIG. 16 is a schematic diagram of a homepage interface, in accordance with some embodiments;

FIG. 17 is a schematic diagram of a first disease assessment interface, in accordance with some embodiments;

FIG. 18 is a schematic diagram of a disease assessment report, in accordance with some embodiments;

FIG. 19 is a schematic diagram of a smart question and answer interface, in accordance with some embodiments;

FIG. 20 is a schematic diagram of a consultation management interface, in accordance with some embodiments;

FIG. 21 is a schematic diagram of an image-text consultation detail interface, in accordance with some embodiments;

FIG. 22 is a schematic diagram of a report interpretation interface, in accordance with some embodiments;

FIG. 23 is a schematic diagram of a knowledge base management interface, in accordance with some embodiments;

FIG. 24 is a schematic diagram of a statistical management interface, in accordance with some embodiments;

FIG. 25 is a schematic diagram of another statistical management interface, in accordance with some embodiments;

FIG. 26 is a schematic diagram of yet another statistical management interface, in accordance with some embodiments;

FIG. 27 is a schematic diagram of yet another statistical management interface, in accordance with some embodiments;

FIG. 28 is a schematic diagram of yet another statistical management interface, in accordance with some embodiments;

FIG. 29 is a flow diagram of a medical information acquisition method, in accordance with some embodiments;

FIG. 30 is a schematic diagram of another homepage interface, in accordance with some embodiments;

FIG. 31A is a schematic diagram of a function navigation interface where a user is a medical worker, in accordance with some embodiments;

FIG. 31B is a schematic diagram of a function navigation interface where a user is a patient, in accordance with some embodiments;

FIG. 32 is a schematic diagram of a schedule interface, in accordance with some embodiments;

FIG. 33 is a schematic diagram of another schedule interface, in accordance with some embodiments;

FIG. 34 is a schematic diagram of a statistical interface, in accordance with some embodiments;

FIG. 35 is a schematic diagram of a second disease assessment interface, in accordance with some embodiments;

FIG. 36 is a schematic diagram of an assessment result interface, in accordance with some embodiments;

FIG. 37 is a schematic diagram of an FAQ smart question and answer interface, in accordance with some embodiments;

FIG. 38 is a schematic diagram of a consultation interface, in accordance with some embodiments;

FIG. 39 is a schematic diagram of a question detail interface, in accordance with some embodiments;

FIG. 40 is a schematic diagram showing a structure of a medical information processing apparatus, in accordance with some embodiments;

FIG. 41 is a schematic diagram showing a structure of a medical information acquisition apparatus, in accordance with some embodiments;

FIG. 42A is a schematic diagram of a follow-up record form to be made, in accordance with some embodiments;

FIG. 42B is a schematic diagram of another follow-up record form to be made, in accordance with some embodiments;

FIG. 42C is a schematic diagram of yet another follow-up record form to be made, in accordance with some embodiments;

FIG. 43A is a schematic diagram of a scale making tool interface, in accordance with some embodiments;

FIG. 43B is a schematic diagram of another scale making tool interface, in accordance with some embodiments;

FIG. 43C is a schematic diagram of yet another scale making tool interface, in accordance with some embodiments;

FIG. 43D is a schematic diagram of yet another scale making tool interface, in accordance with some embodiments;

FIG. 43E is a schematic diagram of yet another scale making tool interface, in accordance with some embodiments;

FIG. 43F is a schematic diagram of yet another scale making tool interface, in accordance with some embodiments;

FIG. 43G is a schematic diagram of yet another scale making tool interface, in accordance with some embodiments;

FIG. 44A is a schematic diagram of a follow-up template interface, in accordance with some embodiments;

FIG. 44B is a schematic diagram of another follow-up template interface, in accordance with some embodiments; and

FIG. 45 is a diagram showing a preview effect of a follow-up record form made on a mobile phone, in accordance with some embodiments.

## DETAILED DESCRIPTION

Technical solutions in some embodiments of the present disclosure will be described clearly and completely with reference to the accompanying drawings below. Obviously, the described embodiments are merely some but not all embodiments of the present disclosure. All other embodiments obtained by a person of ordinary skill in the art based on the embodiments of the present disclosure shall be included in the protection scope of the present disclosure.

Unless the context requires otherwise, throughout the description and the claims, the term "comprise" and other forms thereof such as the third-person singular form "comprises" and the present participle form "comprising" are construed as open and inclusive, i.e., "including, but not limited to". In the description, the terms such as "one embodiment", "some embodiments", "exemplary embodiments", "example", "specific example" or "some examples" are intended to indicate that specific features, structures, materials or characteristics related to the embodiment(s) or example(s) are included in at least one embodiment or example of the present disclosure. Schematic representations of the above terms do not necessarily refer to the same embodiment(s) or example(s). In addition, the specific features, structures, materials, or characteristics may be included in any one or more embodiments or examples in any suitable manner.

Hereinafter, the terms such as "first", "second" and "third" are used for descriptive purposes only, and are not to be construed as indicating or implying the relative importance or implicitly indicating the number of indicated technical features. Thus, a feature defined with "first", "second" or "third" may explicitly or implicitly include one or more of the features. In the description of the embodiments of the present disclosure, the term "a plurality of" means two or more unless otherwise specified.

In the description of some embodiments, the term "coupled" and extensions thereof may be used. For example, the term "coupled" may be used in the description of some embodiments to indicate that two or more components are in direct physical or electrical contact with each other. However, the term "coupled" may also mean that two or more components are not in direct contact with each other, but still cooperate or interact with each other. The embodiments disclosed herein are not necessarily limited to the content herein.

The use of the term "applicable to" or "configured to" means an open and inclusive meaning, which does not exclude devices that are applicable to or configured to perform additional tasks or steps.

In addition, the use of the phrase "based on" herein is meant to be open and inclusive, since a process, step, calculation or other action that is "based on" one or more of the stated conditions or values may, in practice, be based on additional conditions or values exceeding those stated.

Some embodiments of the present disclosure provide a medical information processing method, and an implementation subject of the method is a first electronic device. The first electronic device is, for example, a mobile phone, a tablet computer, a handheld computer, a notebook computer, a personal computer (PC), or any other product or component with a display and a processor, and the embodiments of the present disclosure do not specifically limit a type of the first electronic device.

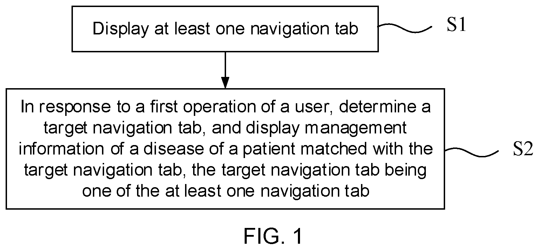

The medical information processing method will be described below by taking an example in which the implementation subject is the PC. As shown in FIG. 1, the medical information processing method includes S1 and S2.

In S1, the PC displays at least one navigation tab 11.

In S2, in response to a first operation of a user, the PC determines a target navigation tab, and displays management information of a disease of a patient matched with the target navigation tab, the target navigation tab being one of the at least one navigation tab.

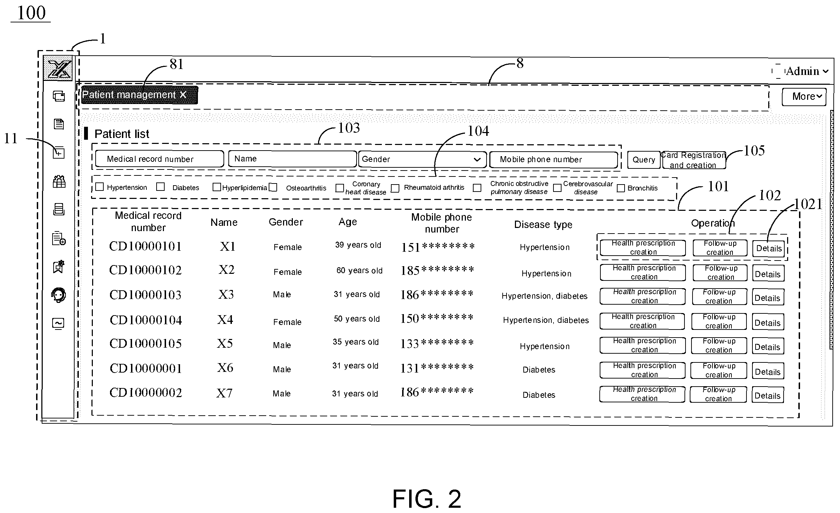

In some examples, referring to FIGS. 2 to 12 and 15 to 19, the PC displays a navigation bar 1, which is configured to present the at least one navigation tab 11. Of course, the at least one navigation tab 11 may further be presented at any other position in an interface displayed by the PC, and the embodiments of the present disclosure do not limit a display position of the at least one navigation tab 11.

For example, the at least one navigation tab 11 is presented in a form of a first-level tab, for example, in a form of at least one icon in the navigation bar 1 shown in FIG. 2, in which one icon represents one navigation tab 11. For another example, the at least one navigation tab 11 is presented in a form of a second-level tab or a lower-level sub-tab. For example, when the user clicks on an icon presented in the navigation bar 1, the icon further displays a second-level tab or a lower-level sub-tab after the icon is expanded to represent the at least one navigation tab. Of course, the at least one navigation tab 11 may also be displayed in other manners, and the display manner of the at least one navigation tab 11 is not specifically limited in the embodiments of the present disclosure.

For example, in a case where there are a plurality of navigation tabs 11, the plurality of navigation tabs 11 may be displayed simultaneously. For example, the plurality of navigation tabs 11 are simultaneously presented in the navigation bar 1 shown in FIG. 2. For another example, the plurality of navigation tabs 11 are not displayed simultaneously. For example, a part of the navigation tabs 11 are first presented in the navigation bar 1 shown in FIG. 2, and a remaining part of the navigation tabs 11 are displayed in sequence according to needs of the user during processing of the medical information. A display order of the plurality of navigation tabs 11 is not specifically limited in the embodiments of the present disclosure.

For another example, in a case where the plurality of navigation tabs 11 are not displayed simultaneously, the plurality of navigation tabs 11 may be displayed on a same interface or on different interfaces. A display position of the plurality of navigation tabs 11 is not specifically limited in the embodiments of the present disclosure.

In S2, the PC determines the target navigation tab in response to the first operation of the user, that is, in response to the first operation of the user on the navigation bar 1, which may be understood as clicking on one of the at least one navigation tab 11, the PC determines the clicked navigation tab 11 as the target navigation tab. According to the determined target navigation tab, a function interface matched with the target navigation tab is displayed, and the function interface is configured to present the management information of the disease of the patient.

It will be noted that, the navigation bar 1 may be displayed in each function interface matched with the target navigation tab appearing below, which facilitates the user to determine different target navigation tabs through the navigation bar, so as to display different function interfaces. In addition, the function interfaces may be a same interface or different interfaces, which is not specifically limited in the embodiments of the present disclosure. In order to facilitate understanding, a description will be given below by taking an example in which the function interfaces are different interfaces.

In the medical information processing method in the embodiments of the present disclosure, the medical information refers to medical data of the patient related to medicine, which includes basic information, diagnosis information, assessment information of the patient, and prescription information and follow-up information that are created by a medical worker for the patient. The user refers to a doctor, a nurse or other medical workers. The management information of the disease of the patient includes medical record card information, health prescription information, and follow-up task information of the patient, disease assessment information, questions, solutions, and consultation information that are matched with the disease of the patient, and statistical information obtained by performing statistical analysis on the above information, which will be described below.

Based on this, the medical information processing method provided by the embodiments of the present disclosure may effectively solve an archiving problem of paper documents, reduce a risk of data loss, greatly improve a working efficiency of the medical worker, and improve a patient visiting efficiency.

In some embodiments, the at least one navigation tab 11 includes a patient management tab. S2 includes S2A.

In S2A, the PC displays at least a patient list in response to the first operation of the user on the patient management tab, the patient list including visit information for each of at least one patient and at least one medical operation index item corresponding to each patient, and the at least one medical operation index item including an index item created for a treatment method of the disease of the patient.

In some examples, as shown in FIG. 2, the PC displays a patient list interface 100 in response to the first operation of the user on the patient management tab. The patient list 101 is presented in the patient list interface 100, and the patient list 101 includes visits information of a plurality of patients and at least one medical operation index item 102 corresponding to each patient. Herein, the first operation may be understood as that the user clicks on an icon representing a patient management tab in the navigation bar 1.

In some embodiments, referring to FIG. 2, the at least one medical operation index item 102 includes a health prescription creation index item. Herein, the health prescription creation index item is configured as an index item that instructs the user to create a health prescription for the patient. Based on this, after S2A, the medical information processing method further includes S22 and S26.

In S22, the PC displays a health prescription text box in response to an operation of the user on a health prescription creation index item corresponding to a target patient, the health prescription text box being a blank box.

In S26, the PC presents a medical regimen matched with a disease of the target patient in the health prescription text box in response to a second operation of the user, the medical regimen including at least one of a medication regimen, a diet regimen, or an exercise regimen.

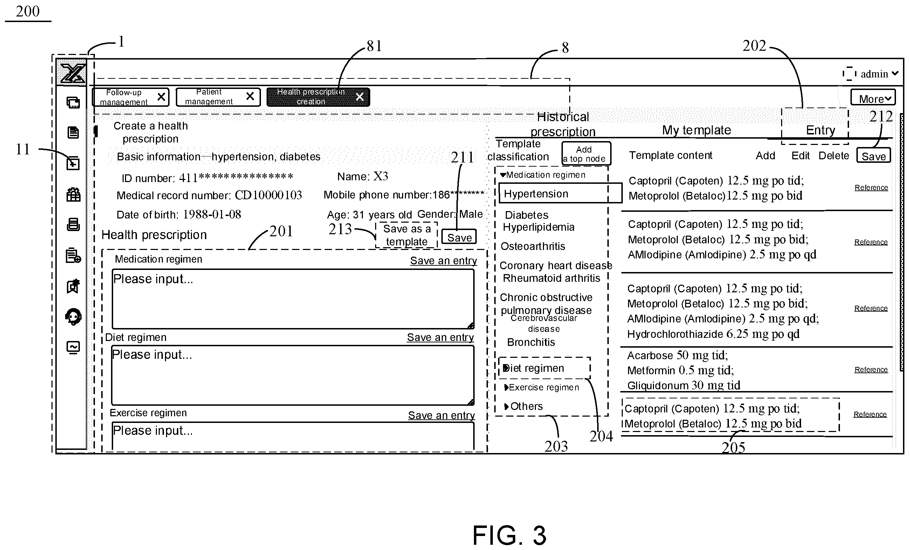

Referring to FIG. 3, in S22, the PC displays a health prescription creation interface 200 in response to the operation of the user on the health prescription creation index item corresponding to the target patient, the health prescription creation interface 200 including a blank health prescription text box 201. Herein, the operation of the user on the health prescription creation index item corresponding to the target patient may be understood as that the user clicks on the health prescription creation index item corresponding to the target patient in the patient list 101.

For example, the user determines that the target patient is a patient whose medical record number is CD10000103 in the patient list 101 shown in FIG. 1, and clicks on a health prescription creation index item corresponding to the patient, and the health prescription creation index item is configured to instruct the user to create a health prescription for the patient whose medical record number is CD10000103.

In S26, the PC presents the medical regimen matched with the disease of the target patient in the blank health prescription text box 201 in response to the second operation of the user. Herein, the second operation of the user may be understood as that the user fills in the medical regimen in the blank health prescription text box 201, or the user presents the medical regimen matched with the disease of the target patient in the blank health prescription text box 201 in a way of referencing an entry text template, referencing a medical regimen template, or reusing a historical prescription described below (i.e., S261 to S263, S264 to S266 or S267 to S269 described below).

Of course, the user may also present the medical regimen matched with the disease of the target patient in the blank health prescription text box 201 in other ways. For example, a part of content of the health prescription text box 201 is presented in a filled-in way, and a remaining part of the content is presented in the way of referencing the entry text template. Herein, the filled-in way may be a way of entering text through a keyboard or by touching a screen, and may also be a voice entry way, etc. The second operation of the user is not specifically limited in the embodiments of the present disclosure, as long as the medical regimen matched with the disease of the target patient may be displayed in the blank health prescription text box 201.

In some examples, after S22, the medical information processing method further includes S23.

In S23, the PC displays an entry sub-tab 202. For example, as shown in FIG. 3, the entry sub-tab 202 is presented in the health prescription creation interface 200 in a form of a word (e.g., entry).

Based on this, S26 includes S261 to S263.

In S261, referring to FIG. 3, the PC presents a first classification list 203 in response to an operation of the user on the entry sub-tab 202, the first classification list 203 including a plurality of first-level items 204 and at least one disease type included in each first-level item 204, and the plurality of first-level items 204 including a medication regimen item, a diet regimen item, and an exercise regimen item.

In S262, referring to FIG. 3, in response to an operation of the user on a disease type corresponding to the disease of the target patient in a first-level item 204 in the first classification list 203, the PC presents at least one entry text template 205 corresponding to the disease type in the first-level item 204.

In S263, in response to an operation of the user on an entry text template 205, the PC presents content of the entry text template 205 in the health prescription text box 201.

In some examples, as shown in FIG. 3, in response to the operation of the user on the entry sub-tab 202, for example, clicking on the text "entry", the PC presents the first classification list 203 in the health prescription creation interface 200. If the disease of the target patient is hypertension, then in response to an operation of the user on a disease type of hypertension, for example, clicking on the disease type of hypertension in the medication regimen item in the first classification list 203, the PC presents a plurality of entry text templates 205 corresponding to hypertension in the health prescription creation interface 200. In response to an operation of the user on an entry text template 205, for example, clicking on a reference button corresponding to the entry text template 205 (as shown in FIG. 3), or dragging the entry text template 205 into the health prescription text box 201, the PC presents content of the entry text template 205 in a text sub-box corresponding to the medication regimen in the health prescription text box 201.

In some other examples, the diet regimen, exercise regimen, or other regimens in the medical regimen may be created in a same way as the medication regimen.

Herein, the content of the entry text template 205 is pre-stored. In this way, the user may directly reference the content of the pre-stored entry text template 205 when creating the health prescription, which improves a working efficiency of a doctor.

Based on this, in some embodiments, the medical information processing method further includes S60.

In S60, the PC displays a first node setup item and an entry text template setup item, the entry text template setup item including at least one of an addition item, an editing item, and a deletion item, the addition item being configured to instruct to add a new entry text template 205, the editing item being configured to instruct to edit at least one entry text template 205, and the deletion item being configured to instruct to delete at least one entry text template.

In some examples, as shown in FIG. 3, in the health prescription creation interface 200, in a case where the entry text template 205 is presented, the addition item, the editing item, and the deletion item are also presented. S6 includes S601 to S603.

In S601, the PC presents an entry text input box in the health prescription creation interface 200 in response to an operation of the user of clicking on the addition item; and in response to an operation of saving content added in the entry text input box, the PC adds the content in the entry text input box as a new entry text template 205. Herein, the operation of saving the content added in the entry text input box may be understood as that the user enters the content in the entry text input box, and then clicks on a first save button 212 presented above the entry text input box.

In S602, the PC changes a state of one of the at least one entry text template 205 to an edited state in response to an operation of the user of clicking on the editing item. For example, the user firstly clicks on a third entry text template corresponding to hypertension in the medication regimen shown in FIG. 3, and then clicks on the editing item, and then the third entry text template is changed from a non-edited state to an edited state. In response to an operation of saving content of the edited entry text template 205, the PC adds the content of the edited entry text template 205 as a new entry text template 205. Herein, the operation of saving the content of the edited entry text template 205 may be understood as that the user modifies the third entry text template in the edited state, and then clicks on the first save button 212 presented above the entry text input box after completing the modification.

In S603, the PC changes a state of one of the at least one entry text template 205 to an edited state in response to an operation of the user of clicking on the deletion item. For example, the user firstly clicks on the third entry text template corresponding to hypertension in the medication regimen shown in FIG. 3, and then clicks on the deletion item, and then the third entry text template 205 is changed from the non-edited state to the edited state. In response to operations of the user of deleting and saving content of an entry text template 205, the PC deletes the content of the entry text template 205. Herein, the operations of deleting and saving the content of the entry text template 205 may be understood as that the user deletes the content of the third entry text template in the edited state, and then clicks on the first save button 212 presented above the entry text input box.

Through S601 to S603, the user may add, modify and delete the entry text template 205 to update the pre-stored entry text template 205.

In some other examples, after S22, the medical information processing method further includes S24.

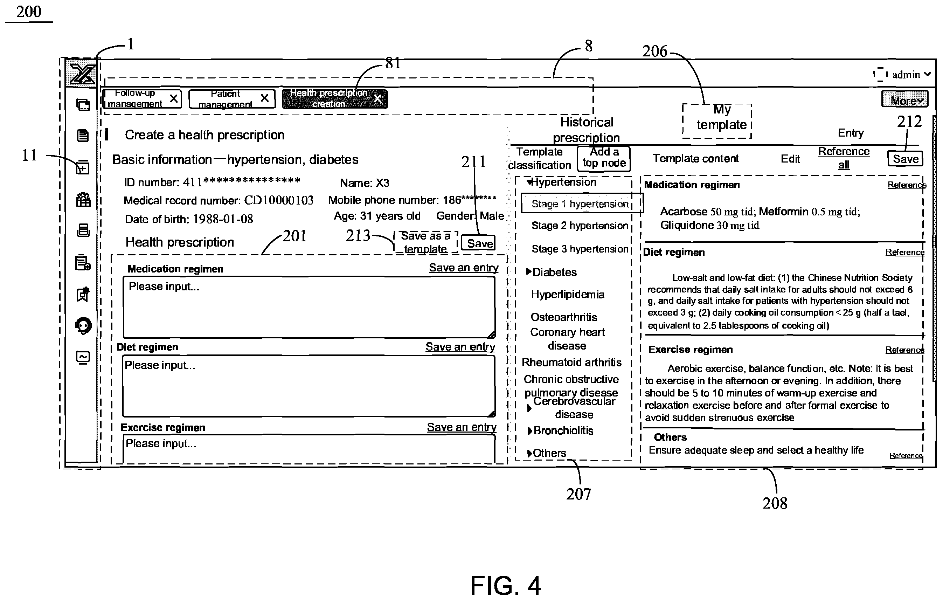

In S24, the PC displays a health prescription template sub-tab. For example, as shown in FIG. 4, the health prescription template sub-tab 206 is presented in the health prescription creation interface 200 in a form of words (e.g., my template).

Based on this, S26 includes S264 to S266.

In S264, referring to FIG. 4, the PC presents a second classification list 207 in response to an operation of the user on the health prescription template sub-tab 206, the second classification list 207 including a plurality of disease types.

In S265, referring to FIG. 4, in response to an operation of the user on a disease type corresponding to the disease of the target patient in the second classification list 207, the PC further presents at least one medical regimen template 208 corresponding to the disease type.

In S266, in response to an operation of the user on a medical regimen template 208, the PC presents at least part of content of the medical regimen template 208 in the health prescription text box 201.

In some examples, as shown in FIG. 4, in response to the operation of the user on the health prescription template sub-tab 206, for example, clicking on the text "my template", the PC presents the second classification list 207 in the health prescription creation interface 200. The second classification list 207 includes a plurality of first-level diseases, such as hypertension, diabetes, hyperlipidemia, osteoarthritis, coronary heart disease and other diseases. The first-level disease type of hypertension further includes second-level disease types, i.e., stage 1 hypertension, stage 2 hypertension, and stage 3 hypertension. If the disease of the target patient is the stage 1 hypertension, then in response to an operation of the user on the stage 1 hypertension, for example, clicking on hypertension in the second classification list 207, and then clicking on the stage 1 hypertension in a secondary menu corresponding to hypertension, the PC further presents a medical regimen template 208 corresponding to the stage 1 hypertension on a side of the second classification list 207.

As shown in FIG. 4, the medical regimen template 208 includes a medication regimen, a diet regimen, an exercise regimen, or others. Of course, the medical regimen template is not limited thereto, FIG. 4 is merely an example, and this is not specifically limited in the embodiments of the disclosure.

For example, all reference buttons corresponding to the medical regimen template 208 are further presented in the health prescription creation interface 200. In response to an operation of the user on a medical regimen template 208, for example, the user clicks on all reference buttons corresponding to the medical regimen template 208, the PC presents entire content of the medical regimen template 208 in the health prescription text box 201.

For another example, reference buttons corresponding to the medication regimen, the diet regimen, the exercise regimen and others are further presented in the health prescription creation interface 200. In S703, in response to an operation of the user on a medical regimen template 208, for example, the user clicks on a reference button corresponding to a diet regimen in the medical regimen template 208, the PC presents content of the diet regimen in a text sub-box corresponding to the diet regimen in the health prescription text box 201. Of course, the user may also present content of a medication regimen, an exercise regimen, or others in corresponding text sub-boxes in the health prescription text box 201 in a same way.

Herein, similar to the entry text template 205, the medical regimen template 208 is also pre-stored. In this way, the user may directly reference the content of the pre-stored medical regimen template 208 when creating the health prescription, which improves the working efficiency of the doctor.

Based on this, in some embodiments, the medical information processing method further includes S8.

In S8, the PC displays a health prescription template setup item, the health prescription template setup item being configured to instruct to edit at least one medical regimen template 208, and to add content of the edited medical regimen template 208 as a new medical regimen template 208, or to update content of the medical regimen template 208 before editing to the content of the edited medical regimen template 208.

In some examples, as shown in FIG. 4, in a case where the medical regimen template 208 is presented in the health prescription creation interface 200, the editing item is further presented in the health prescription creation interface 200. S8 includes S801.

In S801, the PC changes a state of one of the at least one medical regimen template to an edited state in response to an operation of the user of clicking on the editing item, for example, the user firstly clicks on the medical regimen template 208 corresponding to the stage 1 hypertension shown in FIG. 4, and then clicks on the editing item, and then the medical regimen template 208 is changed from a non-edited state to an edited state. In response to an operation of saving content of the edited medical regimen template 208, the PC adds the content of the edited medical regimen template 208 as a new medical regimen template 208. Herein, the operation of saving the content of the edited medical regimen template 208 may be understood as that the user modifies the medical regimen template 208 in the edited state, and then clicks on a first save button 212 presented above the medical regimen template 208 after completing the modification.

Through S801, the user may modify the medical regimen template 208 to update the pre-stored medical regimen template 208.

In yet some examples, after S22, the medical information processing method further includes S25.

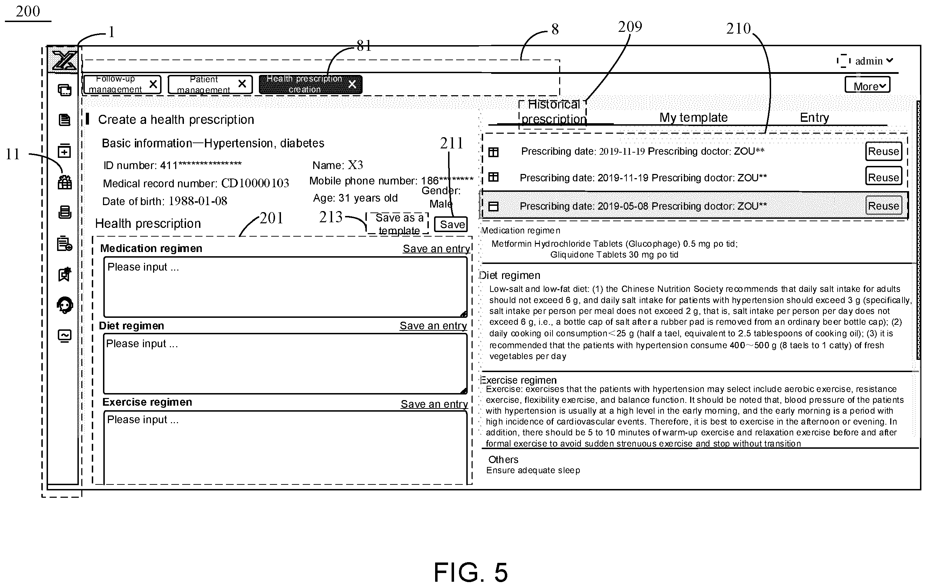

In S25, the PC displays a historical prescription sub-tab. For example, as shown in FIG. 5, the historical prescription sub-tab 209 is presented in the health prescription creation interface 200 in a form of words (e.g., historical prescription).

Based on this, S26 includes S267 to S269.

In S267, referring to FIG. 5, in response to an operation of the user on the historical prescription sub-tab 209, for example, clicking on the text "historical prescription", the PC presents a historical prescription list 210 associated with the disease of the target patient, the historical prescription list 210 including information of three historical prescriptions, for example, the information includes prescribing date(s) and prescribing doctor(s).

In S268, referring to FIG. 5, in response to an operation of the user on the historical prescription list 210, for example, clicking on information of a third historical prescription in the historical prescription list 210, the PC presents content of the third historical prescription in the health prescription creation interface 200.

In S269, in response to an operation of the user on the historical prescription, for example, clicking on a reuse button corresponding to the historical prescription, the PC presents content of the historical prescription in the health prescription text box 201.

Herein, the content of the historical prescriptions in the historical prescription list 210 associated with the disease of the target patient are medical regimens created by the doctor(s) for health condition of the target patient before a current visit date. In this way, the doctor may reuse one of the historical prescriptions according to the health condition of the target patient, which improves the working efficiency of the doctor.

In some embodiments, in a case where the health prescription text box 201 is presented in the health prescription creation interface 200, a health prescription save item 211, for example, a second save button on a side of the health prescription text box 201 in FIGS. 3 to 5, is further presented in the health prescription creation interface 200. After S26, the method further includes S27.

In S27, in response to an operation of the user of saving the content of the health prescription text box 201, for example, the user clicks on the health prescription save item 211, the PC adds a name of the user and clicking time into the historical prescription list 210 of the target patient as new historical prescription information, and associates the content presented in the health prescription text box 201 with the new historical prescription information.

In some embodiments, as shown in FIGS. 3 to 5, in the case where the health prescription text box 201 is presented in the health prescription creation interface 200, an item 213 of saving as a template is further presented in the health prescription creation interface 200. Herein, the item 213 of saving as a template is configured to instruct to add a new medical regimen template.

In some embodiments, in a case where part or all of the content of the entry text template 205, the medical regimen template 208, or the historical prescription are presented in the health prescription text box 201, the medical worker may also modify the content presented in the health prescription text box 201 according to actual health condition of the target patient, so as to make the medical regimen more suitable for the target patient. Based on this, S8 further includes S802.

In S802, in response to the modification of the user to the content presented in the health prescription text box 201 and an operation of the user of saving the modified content, the PC saves the modified content as a new medical regimen template 208. Herein, the operation of saving the modified content may be understood as that the user clicks on the item 213 of saving as a template after completing the modification.

In some other examples, in a case where the user fills in the medical regimen in the blank health prescription text box 201, S8 further includes S803.

In S803, in response to a filling-in operation of the user in the blank health prescription text box 201 and an operation of the user of saving filled-in content, the PC saves the filled-in content as a new medical regimen template 208. Herein, the operation of saving the filled-in content may be understood as that the user clicks on the item 213 of saving as a template after completing the filling-in in the blank health prescription text box 201.

It will be noted that, the entry sub-tab 202, the health prescription template sub-tab 206, and the historical prescription sub-tab 209 may further be presented in any other interface in different forms, for example, they are presented in the navigation bar 1 in forms of icons.

It will also be noted that, the entry sub-tab 202, the health prescription template sub-tab 206, and the historical prescription sub-tab 209 may or may not be displayed simultaneously (as shown in FIGS. 3 to 5), which is not limited in the embodiments of the present disclosure.

In some embodiments, visit information of the target patient, such as the diagnosed disease type, a name, an age and a gender of the target patient, is further presented in the health prescription creation interface 200, which facilitates the doctor to view basic information and the disease type of the target patient when creating a health prescription, and facilitates the doctor to create a health prescription that is more suitable for the target patient based on the information such as the age and the gender of the target patient. For example, symptoms and treatment methods of chronic diseases such as high blood pressure, hyperlipidemia, and hyperglycemia are closely related to ages and genders of the patients, since there are great differences in dietary patterns and living habits of the patients of different ages and genders, and factors such as the dietary patterns and the living habits are crucial to treatments of the above diseases.

In some embodiments, referring to FIG. 2, the at least one medical operation index item 102 includes a follow-up creation index item. Herein, the follow-up creation index item is configured as an index item that instructs the user to create a follow-up task for the patient. After S2A, the medical information processing method further includes S31 and S36.

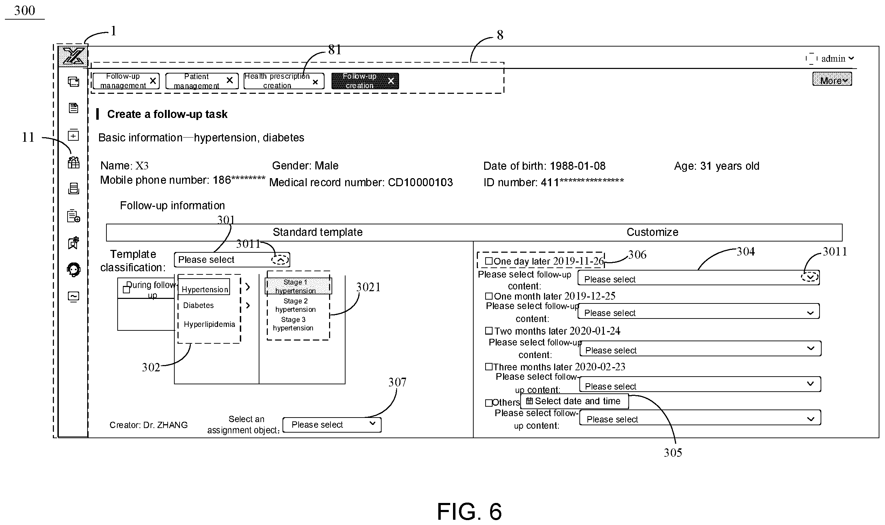

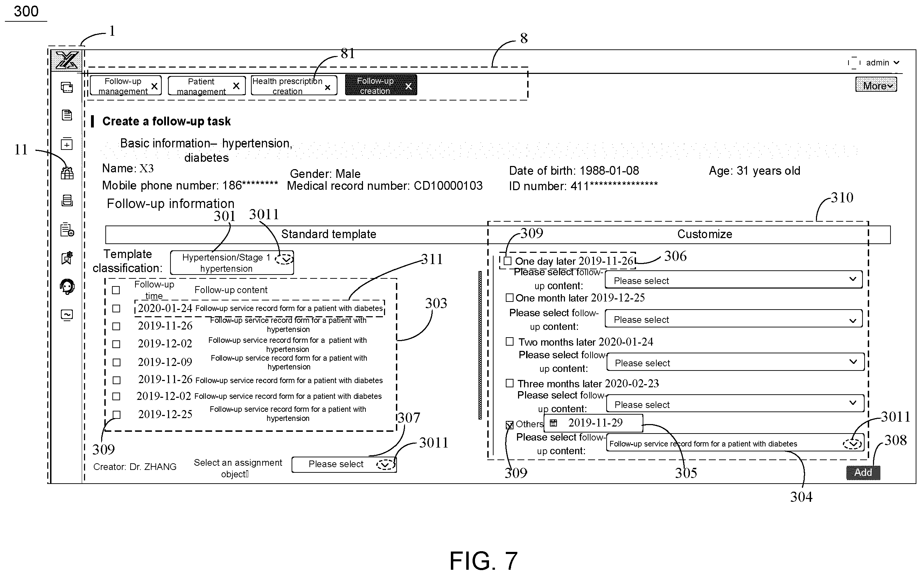

In S31, as shown in FIGS. 6 and 7, the PC displays a follow-up creation interface 300 in response to an operation of the user on a follow-up creation index item corresponding to the target patient. Herein, the operation of the user on the follow-up creation index item corresponding to the target patient may be understood as that the user clicks on the health prescription creation index item corresponding to the target patient in the patient list 101 in the patient management interface 100.

In S36, the PC determines follow-up tasks matched with the disease type in response to a third operation of the user, each follow-up task including follow-up content matched with the disease of the target patient and a follow-up time corresponding to the follow-up content.

Herein, the third operation of the user may be understood as that the user selects a preset follow-up task template 311 in a follow-up task template region 303 in the follow-up creation interface 300 (i.e., S361 described below), or the user creates a follow-up task in a custom way in a custom region 310 in the follow-up creation interface 300 (i.e., S362 or S363 described below).

In some examples, after S31, the medical information processing method further includes S33.

In S33, at least one follow-up task template is displayed.

In some examples, as shown in FIG. 7, the follow-up creation interface 300 includes the follow-up task template region 303, in which a plurality of follow-up task templates 311 are presented. Each follow-up task template 311 includes a follow-up time and follow-up content matched with a disease type. Herein, the plurality of follow-up task templates 311 include a plurality of follow-up tasks 311 matched with a plurality of disease types.

For example, the follow-up content of each follow-up task template 311 is a preset follow-up record form matched with a disease type. The follow-up record form enables the medical worker to know the health condition of the patient through content of the follow-up record form when following up the patient. The follow-up content of each follow-up task template 311 shown in FIG. 7 is merely a name of the follow-up record form. Of course, the follow-up content may also be other content, as long as the content may achieve a purpose that the medical worker may know the health condition of the patient when following up the patient, which is not limited in the embodiments of the present disclosure.

For example, the follow-up time displayed in each follow-up task template 311 may be understood as a date when the follow-up task is performed determined by the PC according to a preset time interval in the preset follow-up task template 311 and a current date when the follow-up task is created. For example, if the preset time interval in the preset follow-up task template 311 is seven days, and the current date when the follow-up task is created is November 19, 2019, then the follow-up time displayed in the follow-up task template in the follow-up creation interface 300 is November 26, 2019.

Based on this, S36 includes S361.

In S361, in response to an operation of the user on a follow-up task template 311, the PC determines the follow-up task template 311 as a follow-up task matched with the disease type.

For example, as shown in FIG. 7, in response to an operation of the user on a follow-up task template 311, which may be understood as selecting an option box 309 on a side of the follow-up task template 311, and then clicking on an add button 308 presented at a lower right corner of the follow-up creation interface 300. In this way, the PC determines the follow-up task template 311 as the follow-up task created for the target patient in response to this operation.

Of course, the user may also select option boxes 309 on sides of a plurality of follow-up task templates 311 simultaneously, and then click on the add button 308. Then, the PC determines the plurality of follow-up task templates 311 as a plurality of follow-up tasks created for the target patient in response to this operation.

In some examples, the preset follow-up record form may be quickly created through a visual operation by an editor. For example, form layout is performed through grids, a nested form layout may be used, Flex is supported, and complex form layout scenarios may be basically covered. By providing the custom region and custom style extension, the user's own follow-up record form may be easily and conveniently customized.

For example, a specific implementation is that: enter a designer page, and by clicking on or dragging an encapsulated field control in a component field region, the field control is laid out in an editing region; in the editing region, the user may drag the field control arbitrarily to change a position of the field control, and add and delete a control tailored to the user's own business characteristics, and the control may be freely reused by copying; the user may autonomously set field attributes and form attributes in a configuration region; "field identification" is configured to bond data; the form layout is performed through a basic 1 / 24 column, the nested form layout may be used, and Flex is supported; and if form styles are insufficient, the styles may be modified through a "custom class" configuration.

Due to importance and diversity of the follow-up record form, for a traditional follow-up record form, it has no standard development interface and has a loose structure; there are a large number of codes to be written, and complex business logic is laborious; a form function is rigid, and cannot be adapted to more business scenarios; maintenance and upgrading difficulty is high; form operation is single, and cannot interact with a process, etc.; business changes quickly, but form update cannot be timely in place; and the codes are written manually, and cannot be flexibly used. In the embodiments of the present disclosure, the creation of the follow-up record form page may be quickly completed through the visual operation of the editor. The form layout is performed through the grids, the nested form layout may be used, Flex is supported, and the complex form layout scenarios may be basically covered. By providing the custom region and the custom style extension, the follow-up record form may be easily and conveniently customized.

For another example, form elements may be added through dragging and clicking. Field attributes addition custom label width, whether to hide a label, a custom class and operation attributes. The operation attributes include data bonding, hiding, and using an arrow to select time, display a password, or display a score. Fields include uploading files, sub-forms, and label pages; a toolbar includes undoing and redoing; generating a form includes a function of uploading a logo; and previewing includes a printing function.

In some other examples, after S31, the medical information processing method further includes S34.

In S34, at least one preset time option and a follow-up content selector corresponding to each preset time option are displayed.

In some examples, as shown in FIG. 7, the follow-up creation interface 300 includes the custom region 310, in which a plurality of preset time options 306 and the follow-up content selector 304 corresponding to each preset time option are presented.

For example, the preset time option 306 is a time interval from a current date when the follow-up task is created, such as one day later, one month later, or two months later. In addition, a follow-up time corresponding to the time interval is also displayed after the time interval, which facilitates the medical worker to determine a specific follow-up date. The follow-up time has a same meaning as the follow-up time in each follow-up task template displayed in S33, each of which may be understood as that the PC determines the date when the follow-up content is performed according to the preset time interval in the preset follow-up task template 311 and the current date when the follow-up task is created.

For example, the follow-up content selector 304 is configured to instruct the user to select the follow-up content matched with the disease of the target patient. The follow-up content has a same meaning as the follow-up content in each follow-up task template displayed in S33, each of which may be understood as a preset follow-up record form matched with a disease type.

Based on this, S36 includes S362.

In S362, in response to selection of the user of a preset time option 306 and selection of the user of a follow-up content selector 304 corresponding to the preset time option 306, the PC determines follow-up content in the follow-up content selector 304 and a follow-up time in the preset time option 306 as the follow-up task matched with the disease type.

For example, as shown in FIG. 7, in response to selection of the user of a preset time option 306 and selection of the user of a follow-up content selector 304 corresponding to the preset time option 306, which may be understood as selecting an option box 309 on a side of the preset time option 306, clicking on an expanded item 3011 in the follow-up content selector 304 corresponding to the preset time option 306, and selecting the follow-up content matched with the disease of the target patient in a follow-up content list displayed on a lower side of the follow-up content selector 304, and then clicking on the add button 308. In this way, the PC determines the selected follow-up time corresponding to the preset time option 306 and the selected follow-up content as the follow-up task created for the target patient in response to the selection.

Of course, the user may also select option boxes 309 on sides of a plurality of preset time options 306 simultaneously, and select corresponding follow-up content in the follow-up content selector 304 corresponding to each preset time option 306, and then click on the add button 308. Then, in response to this operation, the PC determines the selected follow-up time corresponding to each preset time option 306 and the corresponding follow-up content as a plurality of follow-up tasks created for the target patient.

In yet some examples, after S31, the medical information processing method further includes S35.

In S35, a time selector and a follow-up content selector corresponding to the time selector are displayed.

As shown in FIG. 7, in the custom region 310 of the follow-up creation interface 300, the time selector 305 and the follow-up content selector 304 corresponding to the time selector are presented. Herein, the follow-up content selector 304 is the same as the follow-up content selector 304 in S34, which will not be repeated herein.

For example, the time selector 305 is configured to instruct the user to select any date.

Based on this, S36 includes S363.

In S363, in response to selection of the user of the time selector 305 and the follow-up content selector 304 corresponding to the time selector 305, the PC determines selected follow-up content in the follow-up content selector 304 and a follow-up time in the time selector 305 as the follow-up task matched with the disease type. For example, the PC displays a date table in response to the user clicking on the time selector 305, and the user may select any date in the date table as the follow-up time.

In yet some examples, the plurality of follow-up task templates 311, the plurality of preset time options, follow-up content selectors corresponding to each preset time option, time selectors, and follow-up content selectors corresponding to the time selectors are presented in the follow-up creation interface 300 simultaneously. According to the health condition of the patient, the user may determine a suitable follow-up time through the follow-up task template 311 in S33 and the preset time option 306 in S34. In a case where there is no suitable follow-up time in both the follow-up task template 311 and the preset time option 306, the user may also determine a suitable follow-up time through the time selector 305 in S35. Of course, the user may also determine the follow-up time in other ways, which is not limited in the embodiments of the present disclosure.

In some other embodiments, in S31, as shown in FIG. 6, in response to the operation of the user on the follow-up creation index item corresponding to the target patient, the PC displays the follow-up creation interface 300, and presents a disease type selector in the follow-up creation interface 300. Based on this, after S31 and before S36, the medical information processing method further includes S32.

In S32, as shown in FIG. 6, in response to an operation of the user on the disease type selector 301, the PC displays a disease type list 302, and determines the disease type corresponding to the disease of the target patient. Herein, the operation of the user on the disease type selector 301 may be understood as clicking on an expanded item 3011 in the disease type selector 301. Determining the disease type corresponding to the disease of the target patient may be understood as clicking on the disease type in the disease type list 302 corresponding to the disease of the target patient. For example, if the disease of the target patient is hypertension, a degree of which is the first level, then the user clicks on hypertension in the disease type list 302 to display a secondary list 3021 corresponding to hypertension, and clicks on the stage 1 hypertension in the secondary list 3021. The disease type list 302 and the secondary list 3021 are a plurality of preset disease types.

In some examples, in response to the determined disease type corresponding to the disease of the target patient, the PC displays only the follow-up task template 311 matched with the disease type in the plurality of follow-up task templates 311 in S33.

For example, the disease type of the disease of the target patient is hypertension, in response to the determined disease type corresponding to the disease of the target patient being the stage 1 hypertension, the PC displays only one or more follow-up task templates 311 matched with the stage 1 hypertension in the follow-up task template region 303.

In some other examples, in response to the determined disease type corresponding to the disease of the target patient, the PC displays only the follow-up content matched with the disease type in the follow-up content selector in S34 and S35.

For example, the disease type of the disease of the target patient is hypertension, and in response to the determined disease type corresponding to the disease of the target patient being the stage 1 hypertension, the PC displays only the follow-up content matched with the disease type in the follow-up content list displayed on the lower side of the follow-up content selector 304.

In some embodiments, after S36, the medical information processing method further includes S37 and S38.

In S37, as shown in FIGS. 6 and 7, the PC displays a follow-up person selector 307. Herein, the follow-up person selector 307 is configured to instruct the user to associate each follow-up task with a medical worker who performs the follow-up task, so that it may be possible to avoid a problem that the follow-up task is not performed due to an unclear designation of a person who performs the follow-up task.

In S38, the PC associates a follow-up person with a follow-up task in response to an operation of the user on the follow-up person selector 307. For example, the PC displays a follow-up personnel list in response to the user clicking on an expanded item 3011 in the follow-up person selector 307; and in response to the user clicking on a follow-up person in the follow-up personnel list, the PC associates the follow-up person with the follow-up task. For example, the user determines a follow-up task through S361, S362, or S363, and before clicking on the add button 308, the user first clicks on the follow-up person selector 307 to determine a follow-up person, and then clicks on the add button 308, so that the follow-up person may be associated with the follow-up task.

In some embodiments, similar to the health prescription creation interface 200, the visit information of the target patient, such as the diagnosed disease type, the name, the age and the gender of the target patient, is further presented in the follow-up creation interface 300, which facilitates the doctor to view the basic information and the disease type of the target patient when creating a follow-up task, and facilitates the doctor to create a follow-up task that is more suitable for the target patient based on the information such as the age and the gender of the target patient.

In some embodiments, the above follow-up content is a preset follow-up record form. Based on this, the medical information processing method further includes S1-1 to S1-1.

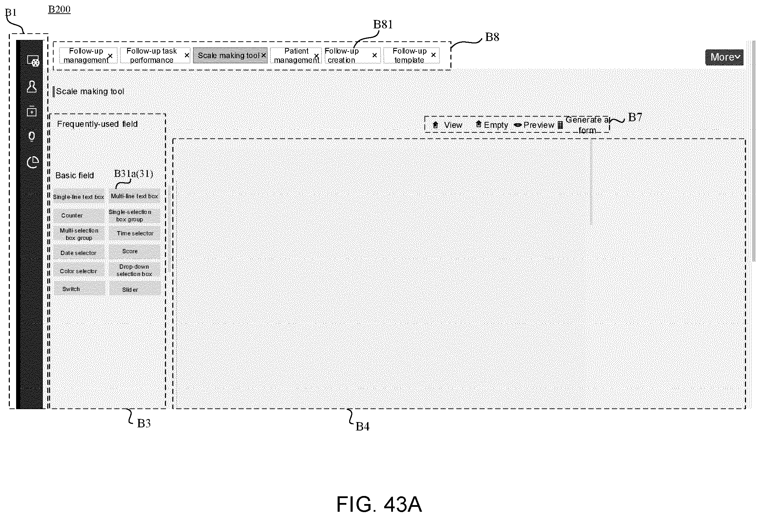

In S1-1, as shown in FIGS. 43A to 43E, in response to an operation of opening the scale making tool interface, the scale making tool interface B200 is displayed. The operation of opening the scale making tool interface B200 is, for example, selecting an icon B91 corresponding to the scale making tool interface B200 in a main menu bar B9 on a left side.

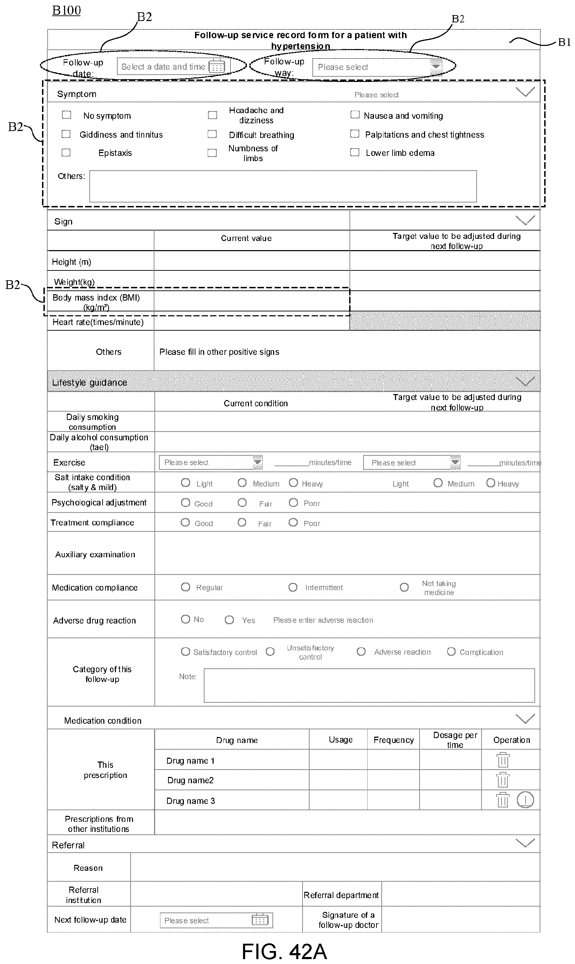

In S1-2, in response to an operation of making a follow-up record form, a follow-up record form B100 is made in the scale making tool interface B200, and the made follow-up record form B100 is saved. The follow-up record form B100 is, for example, a follow-up service record form for a patient with hypertension shown in FIGS. 42A to 42C.

The following focuses on steps of making the follow-up record form in S1-2.

Referring to FIGS. 43A to 43E, the scale making tool interface B200 includes a field display region B3 and an editing region B4. The field display region B3 includes a plurality of fields B31, and the editing region B4 is configured to make a follow-up record form B100. The follow-up record form B100 to be made includes a plurality of target elements, and each target element is generated by using a field.

It will be noted that, the follow-up record form B100 includes a plurality of "elements", which refer to basic components of the follow-up record form B100, and are essentially carriers of various information or data in the follow-up record form B100, and information of a follow-up object (the patient) may be collected through the elements. The "target elements" refer to "elements" to be generated in the follow-up record form B100 to be made, i.e., carriers of information or data to be generated. The "fields" included in the field display region B3 refer to basic components for generating the "target elements", which may be understood as unedited initial states of the "target elements".

For example, the follow-up record form B100 shown in FIG. 42A includes a header B1 and a plurality of elements B2, and the header B1 is a title of the follow-up record form B100. The follow-up record form B100 includes a "follow-up date" element, a "follow-up way" element, a "symptom" element, a "body mass index" element, and other elements. These elements are basic components of the follow-up record form B100, for example, the "body mass index" element is a carrier of body mass index data of the follow-up object. Each element has set attributes, which represent properties or characteristics of the element, and are configured to define a name, filling-in setup, a data source and other characteristics of the target element. In a case where the follow-up record form to be made is the follow-up record form B100 shown in FIG. 1A, the target elements are the elements to be generated in the follow-up record form B100 to be made, i.e., the "follow-up date" element, the "follow-up way" element, the "symptom" element, the "body mass index" element, and other elements that are to be generated.

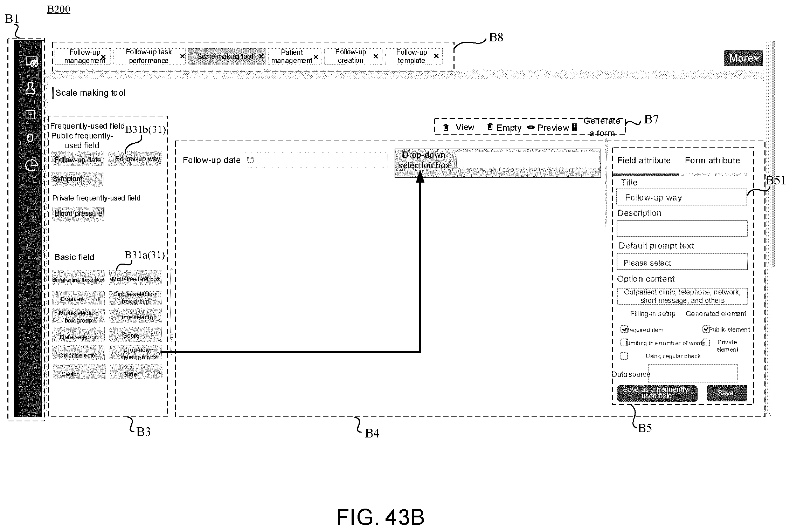

For example, as shown in FIGS. 43A and 43B, the fields B31 include basic fields B31a, or the fields B31 include the basic fields B31a and frequently-used fields B31b. The basic fields B31a and the frequently-used fields B31b will be introduced hereinafter.

The follow-up record form B100 in FIG. 42A may also be understood as a follow-up record form made by using the method of making a follow-up record form B100 in S1-2. The follow-up record form includes a plurality of elements B2. Each element B2 in the follow-up record form B100 shown in FIG. 42A includes editing box(es), and the editing box(es) are blank, which means that the element is in a state where content thereof is not filled in, that is, the element has no content. For example, an editing box of the "follow-up date" element is not filled with a specific date, an editing box of the "follow-up way" element is not filled with a specific way, and in editing boxes of the "symptom" element, editing boxes of a plurality of symptoms are not checked, and an editing box of the "body mass index" element is blank. That is, after the follow-up record form B100 is created, the content of each element B2 is in a to-be-filled-in state, and the user needs to fill in the editing box(es) of each element B2 in the follow-up record form B100, so as to achieve collection of the medical information.

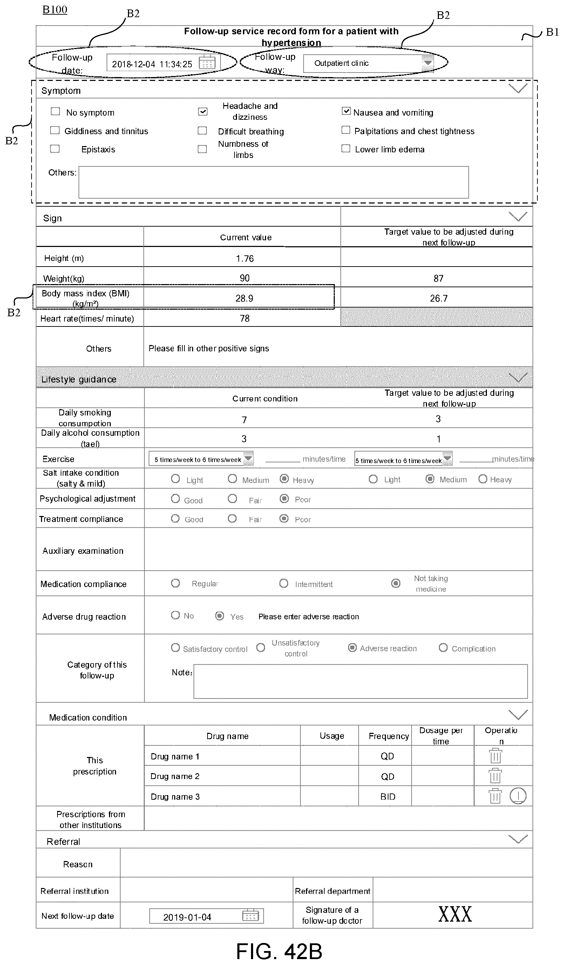

Referring to FIG. 42B, FIG. 42B shows a state where the editing box(es) of each element B2 in the made follow-up record form are filled in, i.e., a state where data collection is completed. For example, the editing box of the "follow-up way" element is filled with outpatient clinic, options of "headache and dizziness" and "nausea and vomiting" are checked in the editing boxes of the "symptom" element, and the editing box of the "body mass index" element is filled in with 28.9. For example, the follow-up record form B100 shown in FIG. 42B may be considered as a follow-up record form B100 in which the doctor fills in the content of each element according to a follow-up result after performing a follow-up service on the patient with hypertension, i.e., after performing a follow-up task.

In the present disclosure, a user who makes the follow-up record form B100 is referred to as a first user, and a user who fills in the content of each element 2 of the follow-up record form B100 is referred to as a second user, that is, a user who performs the follow-up task is the second user. In an example where the follow-up record form B100 is the follow-up record form for the patient with hypertension, the first user may be, for example, a doctor, a nurse, or an operation manager, and the second user may be, for example, a doctor, a nurse, or a patient. For example, the first user and the second user may be a same person or different persons.

In S1-1, the scale making tool interface B200 is first displayed, and the first user creates a form in the scale making tool interface B200. In the scale making tool interface B200, the plurality of fields B31 are configured to generate target elements, and the editing region B4 is configured to create a follow-up record. At a beginning of creating a new follow-up record form, for example, the editing region B4 is blank.

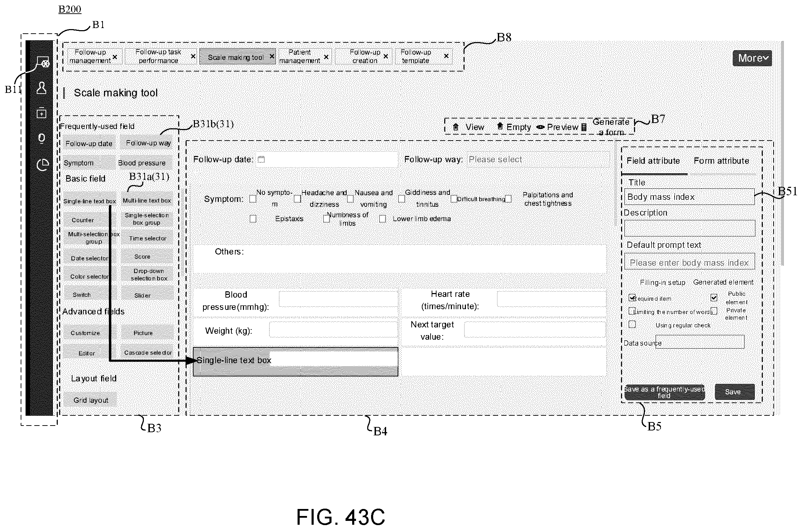

In S1-21, as shown in FIGS. 43B and 43C, a field selection operation entered in the scale creation tool interface B200 is received, and the field selection operation is configured to select one basic field B31a of the plurality of fields B31.

It will be noted that, the "operations" involved in the present disclosure all refer to operations performed by the user. For example, the "operations" may be operations such as clicking, double-clicking, and dragging with a mouse, or may be in forms of touching a screen, gestures, and voice.

For example, in S1-21, the field selection operation is to select one basic field B31a of the plurality of fields B31 through double-clicking with the mouse, and to display the field B31 at a target position in the editing region B4 by clicking on the target position in the editing region B4 with the mouse. Or, the field selection operation is to select one basic field B31a of the plurality of fields B31, and to drag the field B31 to the target position in the editing region B4. The selected field B31 is, for example, a single-line text box.

In S1-22, as shown in FIGS. 43B and 43C, the selected field B31 is displayed in the editing region B4 in response to the field selection operation.

For example, the selected field B31 is displayed at the target position in the editing region B4 in response to the field selection operation. For example, a drop-down selection box is displayed at the target position in the editing region B4.

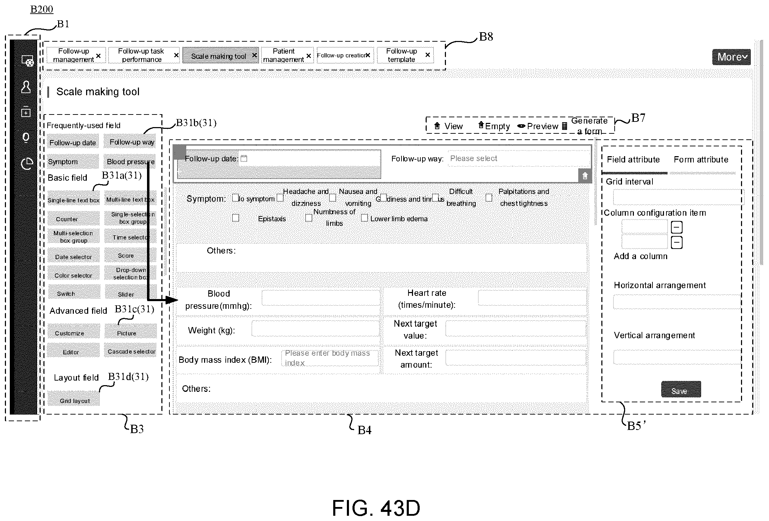

It will be noted that, since each target element B2 in the follow-up record form B100 to be made is generated by using a field B31, after the selected field B31 is displayed in the editing region B4, operations described later in the present disclosure (e.g., S1-23' to S1-25) also need to be performed on the field B31 to generate a corresponding target element by using the field. For example, as shown in FIGS. 43B and 43D, at the target position in an upper right corner of the editing region B4, a target element generated by using the drop-down selection box is the "follow-up way" element.

In S1-260, the field selection operation is repeatedly received and responded to until the fields B31 corresponding to the plurality of target elements required by the follow-up record form B100 are all displayed in the editing region B4 to generate the follow-up record form B100.

For example, S1-260 includes S1-26 and S1-27.

In S1-26, it is determined whether the fields B31 corresponding to the plurality of target elements required by the follow-up record form B100 are all displayed in the editing region B4; if not, return to S1-21 to continue to perform the operations in S1-21 and S1-22; and if so, S1-27 is performed.

In S1-27, a follow-up record form is generated.

For example, the target elements included in the follow-up record form to be created include twenty target elements such as the "follow-up date" element, the "follow-up way" element, and the "symptom" element. By repeating the operations in S1-21 and S1-22, twenty fields B31 in the field display region B3 are selected in sequence, and the fields B31 are displayed in sequence in the editing region B4, so as to generate corresponding target elements B2 to finally generate the required follow-up record form B100.

In this way, the user may design and make the form according to his or her own needs, and may customize the content of the form through corresponding operations, which has a high flexibility, improves an efficiency of making the form, and reduces a software operation and maintenance cost.

In some embodiments, as shown in FIGS. 43A to 43D, the plurality of fields B31 include at least one basic field B31a, and the basic field B31a includes at least one attribute to be edited.

The "basic field" is a basic component whose attributes have not been edited and that are used to generate a target element. The basic field is, for example, a basic component preset by a software engineer. After the attributes of the basic field B31a are edited and saved, a target element is generated by using the basic field B31a. The "attributes" represent the properties or characteristics of the element, and the attributes are configured to define the name, the filling-in setup, the data source and other characteristics of the element or field. The "attributes to be edited" refer to items that need to be edited in the basic field.

For example, as shown in FIGS. 43B and 43C, the field display region B3 of the scale making tool interface B200 includes a plurality of basic fields B31a, which are, for example, a single-line text box, a multi-line text box, a multi-selection box group, a single-selection box group, the drop-down selection box, a multi-level drop-down box, a time selector, a date selector, and a color selector. Each basic field B31a includes at least one attribute to be edited, and different basic fields B31a include different attributes. For example, attributes of the single-line text box include a title attribute, a description attribute, a default prompt text attribute, filling-in setup attributes (whether it is a required item, whether there is a word limit, whether there is a regular check, etc.), and a data source attribute. Attributes of date and time include a title attribute, a description attribute, data type attributes (date, time, date and time), title and time attributes displayed by default, filling-in setup attributes (the filling-in setup attributes including whether it is a required item, whether to limit an optional range of date, whether to limit an optional range of time, and whether to be enterable), and a data source attribute. By displaying an attribute menu B5 corresponding to each basic field B31a in the editing region, and filling in content of attribute editing boxes B51 in the attribute menu B5, the attributes of the basic field may be edited. Before the attributes of the basic field B31a are edited, the attribute editing boxes in the attribute menu B5 of the basic field B31a are blank.

In a case where the field B31 selected through the field selection operation is a basic field B31a, S1-21 is S1-21'.

In S1-21', the field selection operation entered in the scale making tool interface B200 is received, and the field selection operation is configured to select one basic field B31a of the plurality of fields B31. S1-22 is S1-22'.

Based on this, S1-22 is S1-22'.

In S1-22', the selected basic field B31 is displayed in the editing region B4 in response to the field selection operation. For example, as shown in FIG. 43C, the selected basic field B31a is the single-line text box, and the selected single-line text box is displayed in the editing region B4.

In the above case, after S1-22', S1-22 further includes S1-23' to S1-25.

In S1-23', the attribute menu B5 of the basic field B31a is displayed in the editing region in response to the field selection operation, the attribute menu B5 including at least one attribute editing box B51.

For example, as shown in FIG. 43C, an attribute menu B5 of the single-line text box is displayed in the editing region B4 in response to the field selection operation. The field selection operation is, for example, a way of double-clicking the single-line text box displayed in the editing region B4. That is to say, the field selection operation includes an operation of selecting the single-line text box and dragging the single-line text box to the target position in the editing region B4 with the mouse, and further includes an operation of double-clicking the single-line text box displayed in the editing region B4. Or, after the single-line text box is dragged to the target position in the editing region B4, the attribute menu B5 of the single-line text box is automatically displayed without the double-clicking operation.

The attribute menu B5 includes at least one attribute editing box B51, for example, the at least one attribute of the single-line text box includes the title attribute, the description attribute, the default prompt text attribute, the filling-in setup attributes (whether it is a required item, whether there is a word limit, whether there is a regular check, etc.), and the data source attribute. Then, the attribute menu B5 includes a title attribute editing box B51, a description attribute editing box B51, a default prompt text attribute editing box B51, and filling-in setup attribute editing boxes B51. In this case, the plurality of attribute editing boxes B51 displayed in the attribute menu B5 are all blank and in a state to be edited.

In S1-24, a basic field attribute editing operation entered in the at least one attribute editing box B51 is received, the basic field attribute editing operation being configured to edit an attribute of the basic field in each attribute editing box B51.

For example, the basic field attribute editing operation may be an operation in which the first user selects an attribute editing box B51 through clicking with the mouse, and enters corresponding content in the attribute editing box B51 with a keyboard.

In a case where the selected basic field B31a is the drop-down selection box, as shown in FIG. 43B, for example, if "follow-up way" is filled in in a title attribute editing box B51 in an attribute menu B5 of the drop-down selection box, then the target element to be generated by using the drop-down selection box is the "follow-up way" element. "Please select" is filled in in a default prompt text attribute editing box B51, and "outpatient clinic, telephone, network, short message, and others" are filled in in an option content attribute editing box B51. A description attribute is used to specify some additional explanations for the basic field B31a, which are generally used to guide the second user to fill in the follow-up record form B100, and the additional explanations may also not be filled in. For example, no content is filled in a description attribute editing box B51 herein. Filling-in setup attributes include whether it is a required item, whether there is a word limit, and whether there is a regular check. Corresponding content may be checked, for example, if a check box corresponding to the required item is checked, it means that in the generated form, the target element is an element that requires the second user to have to fill in content. A "data source attribute" refers to an attribute linking a generated element to a data source, and the attribute makes it possible to automatically generate content of the element when the content of the element is filled in. The data source refers to a mathematical formula, a mapping relationship, a background database, etc. Herein, for example, the data source is not bound, that is, content of the data source attribute is not set.

In a case where the selected basic field B31a is the single-line text box, as shown in FIG. 43C, for example, if "body mass index" is filled in the title attribute editing box B51 in the attribute menu B5 of the single-line text box, then a target element to be generated by using the single-line text box is the "body mass index" element. "Please enter body mass index" is filled in the default prompt text attribute editing box B51. No content is filled in the description attribute editing box B51. The filling-in setup attributes include whether it is a required item, whether there is a word limit, and whether there is a regular check, and a check box corresponding to the required item is checked. The data source attribute represents some related information linked to a target element generated by using the frequently-used field B31b. Content of the element may be directly obtained according to the data source attribute. Herein, for example, a calculation formula of the body mass index may be filled in the data source attribute editing box B51. When the second user fills in the content of the follow-up record form B100, if a height and a weight of the patient are known, a system will automatically calculate the body mass index of the patient according to the filled-in height and weight, and the bound calculation formula of the body mass index, and display the body mass index in the editing box of the "body mass index" element of the follow-up record form B100.

In S1-25, an edited attribute is displayed in each attribute editing box to generate a target element in response to a basic field attribute editing operation.

After the first user edits the attribute in each attribute editing box B51 of the basic field B31a through the basic field attribute editing operation, the edited attribute is displayed in each attribute editing box B51 in response to the basic field attribute editing operation. For example, as shown in FIG. 43C, the text "body mass index" is displayed in the title attribute editing box B51 in the attribute menu B5 of the single-line text box, and the text "please enter body mass index" is displayed in the default prompt text attribute editing box B51. Finally, a target element is generated by using the basic field B31a with set attribute content. As shown in FIG. 43D, for example, the "follow-up way" element is generated by using the drop-down selection box, and the "body mass index" element is generated by using the single-line text box.

In some embodiments, as shown in FIGS. 43B and 43C, the attribute menu B5 further includes a save button. The basic field attribute editing operation is further configured to select the save button in addition to editing the attribute of the basic field in each attribute editing box B51, so as to save the edited attribute to generate the target element.

In some embodiments, as shown in FIGS. 43B and 43C, the attribute menu B5 further includes a button of saving as a frequently-used field. Based on this, the medical information processing method further includes S1-251 and S1-252.

In S1-251, an operation of selecting the button of saving as a frequently-used field entered in the attribute menu is received.

For example, as shown in FIGS. 43B and 43C, the attribute menu B5 of the basic field B31a includes the "save" button and the button of "saving as a frequently-used field". The operation of selecting the button of saving as a frequently-used field is an operation performed by the first user. For example, after the target element is generated by using the base field B31a in S1-25, by clicking on the button of "saving as a frequently-used field", the generated target element may be saved as a frequently-used field B31b.

In S1-252, the generated target element is displayed in the field display region B3 as the frequently-used field B21b in response to an operation of selecting the button of saving as a frequently-used field.

As shown in FIGS. 43B and 43C, the field display region B3 includes a plurality of basic fields B31a and a plurality of frequently-used fields B31b. A frequently-used field B31b includes at least one edited attribute. For example, after the plurality of attributes of the single-line text box are set, the "follow-up way" element is generated, that is, the target element is generated by using the basic field B31a; and in response to the operation of selecting the button of saving as a frequently-used field, the target element is saved as the frequently-used field B31b, and a "follow-up way" frequently-used field is displayed in the field display region B3.

In the present disclosure, the basic field B31a includes at least one attribute to be edited, the frequently-used field B31b includes at least one edited attribute, and the target element B2 includes at least one edited attribute. The above attributes are, for example, the title attribute, the description attribute, the default prompt text attribute, the filling-in setup attributes and the data source attribute. The "data source" represents a source of content of an element, or a source of data collected by the element. For example, for the "body mass index" element, a "data source" thereof is obtained according to the height and the weight of the follow-up object and a corresponding relationship among the height, the weight and the body mass index. The "data source attribute" refers to an attribute linking the generated element to the data source, and the attribute makes it possible to automatically generate the content of the element when the content of the element is filled in. The data source refers to the mathematical formula, the mapping relationship, the background database, etc. For a way of setting the data source attribute included in the basic field B31a, there are some implementations as follows.

In some embodiments, the plurality of fields B31 include at least two basic fields B31a, and the at least two basic fields B31a include first basic fields B31a and second basic fields B31a. A first basic field B31a is used to generate a first target element, a second basic field B31a is used to generate a second target element, and there is a corresponding relationship between the first target element and the second target element. At least one attribute editing box of the first basic field includes a data source attribute editing box.

For example, the first basic field B31a is the single-line text box, and the second basic fields B31a are single-line text boxes, the number of which is two. The first target element generated by using the first basic field B31a is, for example, the "body mass index" element. A second target element generated by using one of the second basic fields B31a is a "height" element, and a second target element generated by using the other second basic field B31a is a "weight" element, and a corresponding relationship between the "body mass index" element and both the "height" element and "weight" ele-

$$BMI = \frac{W}{H^2}$$

ment is _____, where BMI is the body mass index, W is the weight, a unit of which is kg, and H is the height, a unit of which is m. Then, there is the above corresponding relationship between the "body mass index" element and both the "height" element and the "weight" element.

Or, the first basic field B31a is the single-line text box, and the second basic field B31a is the single-line text box. The first target element generated by using the first basic field B31a is, for example, an "age" element, and the second target element generated by using the second basic field B31 is a "date of birth" element, and a corresponding relationship between the "age" element and the "date of birth" element is: $T=T_1-T_2$, where $T$ is the age, $T_1$ is a current date, and $T_2$ is the date of birth. Then, there is the above corresponding relationship between the "age" element and the "date of birth" element.

For another example, the first basic field B31a is the single-line text box, and the second basic field B31a is the single-line text box. The first target element generated by using the first basic field B31a is, for example, an "expected delivery date" element, and the second target element generated by using the second basic field B31a is a "last menstrual date" element, and a corresponding relationship between the "expected delivery date" element and the "last menstrual date" element is that the expected delivery date is a date obtained by adding nine months to or subtracting three months from a month that the last menstrual date is in, and then adding seven days. Then, there is the above corresponding relationship between the "expected delivery date" element and the "last menstrual date" element.

In S1-24, an eleventh operation entered in the at least one attribute editing box B51 is received. The eleventh operation is used to edit the attribute of the basic field in each attribute editing box B51, and then the eleventh operation is to fill in the above formula in the data source attribute editing box B51. For example, in a case where the first target element generated by using the first basic field B31a is, for example, the "body mass index" element, the eleventh operation is to fill in the formula representing the above corresponding relationship in the data source attribute editing box B51.

In S1-25, the edited attribute is displayed in each attribute editing box B51 in response to the operation of selecting the button of saving as a frequently-used field, which includes S1-251.

In S1-251, a data source attribute representing the corresponding relationship is displayed in the data source attribute editing box B51 of the first basic field in response to the operation of selecting the button of saving as a frequently-used field.

For example, as shown in FIG. 43C, in the attribute menu B5 of the single-line text box, the corresponding formula is entered in the data source attribute editing box B51 to set content of the data source attribute of the single-line text box.

The medical information processing method further includes S1-3.

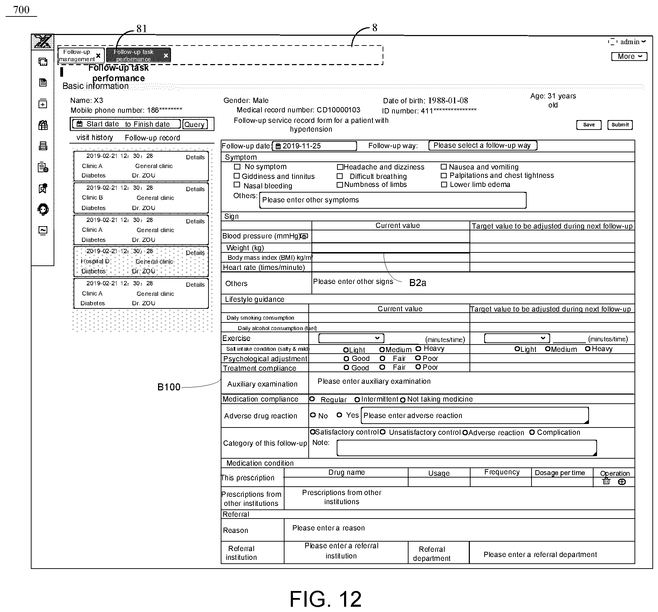

In S1-3, as shown in FIG. 12, a follow-up task performance interface 700 is displayed in response to an operation of opening follow-up task performance. The follow-up task performance interface 700 includes the follow-up record form B100 included in the follow-up task created for the target patient. The follow-up record form B100 includes first target elements and second target elements. The first target element and the second target element each have an editing box. Herein, the operation of opening the follow-up task performance is the same as an operation of the user on a follow-up performance index item described below, and they are both configured to display the follow-up task performance interface 700. For example, the operation of opening the follow-up task performance is to click on the follow-up performance index item in a follow-up task list 601.

For example, as shown in FIG. 12, in the follow-up record form B100, the first target element B2 is the "body mass index" element, the second target element B2 is the "weight" element, and the first target element B2 and the second target element each have an editing box B21.

S1-3, in which the follow-up task is performed in response to the operation of performing the follow-up task, includes S1-31 to S1-32.

In S1-31, an operation of the user of entering target content in an editing box of the second target element B2 is received. For example, the height and the weight of the patient are entered in editing boxes 2a of the "height" element and the "weight" element, respectively.

In S1-32, in response to the operation of the user of entering the target content in the editing box of the second target element, the target content is displayed in the editing box of the second target element, and content obtained according to the corresponding relationship represented by the data source attribute is automatically displayed in an editing box of the first target element.

When the attributes of the basic field for generating the first target element are edited, the corresponding relationship between both the height and the weight, and the body mass index is edited in the data source attribute editing box, and the content of the data source attribute is set in advance. In this way, in a process of performing the follow-up task, when the second user fills in the follow-up record form B100, only by entering the corresponding content into the editing boxes B2a of the "height" element and the "weight" element, the content of the "body mass index" element may be automatically calculated and displayed in the editing box without manual calculation, which improves efficiency and accuracy, and saves time. For example, as shown in FIG. 42B, a current value of the "height" element is 1.76 and a current value of the "weight" element is 90, and then a current value of the "body mass index" element may be automatically calculated to be 28.9.

In some other embodiments, the plurality of fields include third basic fields, a third basic field is used to generate a third target element, and at least one attribute editing box of the third basic field includes a data source attribute editing box.

S1-25, in which the edited attribute is displayed in each attribute editing box B51 in response to the basic field attribute editing operation, includes S1-251'.

In S1-251', in response to the basic field attribute editing operation, a data source attribute linked to the background database is displayed in the data source attribute editing box B51 of the third basic field, the background database storing content of the target element corresponding to the basic field B31a.

For example, the background database is, for example, a database of an electronic medical record system or a health management system of each hospital. After the data source attribute of the basic field B31a is bound to the background database, when the second user fills in the content of the element generated by using the basic field B31a, corresponding content may be automatically retrieved from the background database without manual filling-in, thereby improving the efficiency and accuracy.

The medical information processing method further includes S1-3'.

In S1-3', as shown in FIG. 12, the follow-up task performance interface 700 is displayed in response to the operation of performing the follow-up task. The follow-up task performance interface 700 includes the follow-up record form B100 in the follow-up task created for the target patient, the follow-up record form B100 includes the third target element, and the third target element has an editing box.

For example, as shown in FIG. 12, in the follow-up record form B100, the third target element B2 is the "follow-up date" element, and the third target element has an editing box B2a.

S1-3', in which the follow-up task is performed in response to the operation of performing the follow-up task, includes S1-31' to S1-32'.

In S1-31', an operation of the user of selecting the editing box B2a of the third target element is recieved.

For example, the operation of selecting the editing box B2a of the third target element is to select the editing box 2a of the "follow-up date" element.

In S1-32', in response to the operation of the user of selecting the editing box B2a of the third target element, the content in the background database linked to the data source attribute of the third target element is automatically displayed in the editing box of the third target element.

For example, considering an example in which the third basic field B31a is date and time, content of the title attribute is set to the "follow-up date" in an attribute menu, and then the third target element generated by using the basic field B31a is the "follow-up date" element. Content of the data source attribute is set to be bound to the background database in the attribute menu. For example, there are a first visit time of the patient, a set follow-up interval, and a current date and time stored in the background database. According to the first visit time and the set follow-up interval, and the current date and time, specific content of the follow-up date may be obtained. For example, in FIG. 42B, the content of the "follow-up date" in the follow-up record form B100 is automatically filled in as 2018-12-04 11:34:25, without the manual filling-in by the second user.

For another example, considering an example in which the third basic field B31a is the single-line text box, content of the title attribute is set to "name" in the attribute menu, and then the third target element generated by using the basic field B31a is a "name" element. The content of the data source attribute is set to be bound to the background database in the attribute menu. For example, the background database is a database of a medical data processing system, and there are the name, gender, age, height, weight and other information of the patient stored in the database of the medical data processing system. When the second user fills in the form, content of the "name" element may be loaded directly without manual entering.

In some embodiments, as shown in FIGS. 43B to 43D, in the field display region B3 of the scale making tool interface B200, the plurality of fields B31 include at least one frequently-used field B31b, and the frequently-used field B31b includes at least one edited attribute.

For example, as shown in FIG. 43D, the frequently-used fields B31b include a "follow-up way" frequently-used field B31b, a "follow-up date" frequently-used field B31b, and a "symptom" frequently-used field B31b. Each frequently-used field B31b includes at least one edited attribute. Considering an example in which the frequently-used field B31b is the "follow-up way" frequently-used field B31b, the "follow-up way" frequently-used field B31b includes a title attribute, a default prompt text attribute, an option content attribute, a description attribute and filling-in setup attributes. Content of the plurality of attributes has been edited and saved. The frequently-used field B31b may be directly used as a target element.

In a case where the field B31 selected through the field selection operation is a frequently-used field B31b, S1-21 is S1-21".

In S1-21", the field selection operation entered in the scale making tool interface B200 is received, the field selection operation being configured to select a frequently-used field B31b in the plurality of fields B31.

Based on this, S1-22 is S1-22".

In S1-22", the selected frequently-used field B31a is displayed in the editing region B4 in response to the field selection operation. For example, as shown in FIG. 43D, the selected frequently-used field B31b is a "blood pressure" frequently-used field B31b.

In the above case, after S1-22", the method further includes S1-23".

In S1-23", the frequently-used field B31b displayed in the editing region B4 is used as the target element B2 in response to the field selection operation.

For example, the field selection operation is, for example, to select a frequently-used field B31b and to drag the frequently-used field B31b to a target position in the editing region B4. In response to the field selection operation, the frequently-used field B31b displayed in the editing region B4 is used as the target element B2. Since the frequently-used field B31b includes at least one edited attribute, the frequently-used field B31b may be directly used as the target element. For example, as shown in FIG. 12, a "blood pressure" frequently-used field is set in the editing region to generate a "blood pressure" element.

In some embodiments, as shown in FIG. 43B, the plurality of fields B31 includes a plurality of frequently-used fields B31b, and the plurality of frequently-used fields B31b include at least one public frequently-used field B31b and at least one private frequently-used field B31b.

The public frequently-used field B31b may be used as a target element in at least two follow-up record forms to be made, and the private frequently-used field B31b may be used as a target element in one follow-up record form B100 to be made. That is to say, the public frequently-used field B31b may be reused in at least two different follow-up record forms B100, and the private frequently-used field B31b may only be used in one follow-up record form B100 to be made, and may not be reused in other follow-up record forms B100.

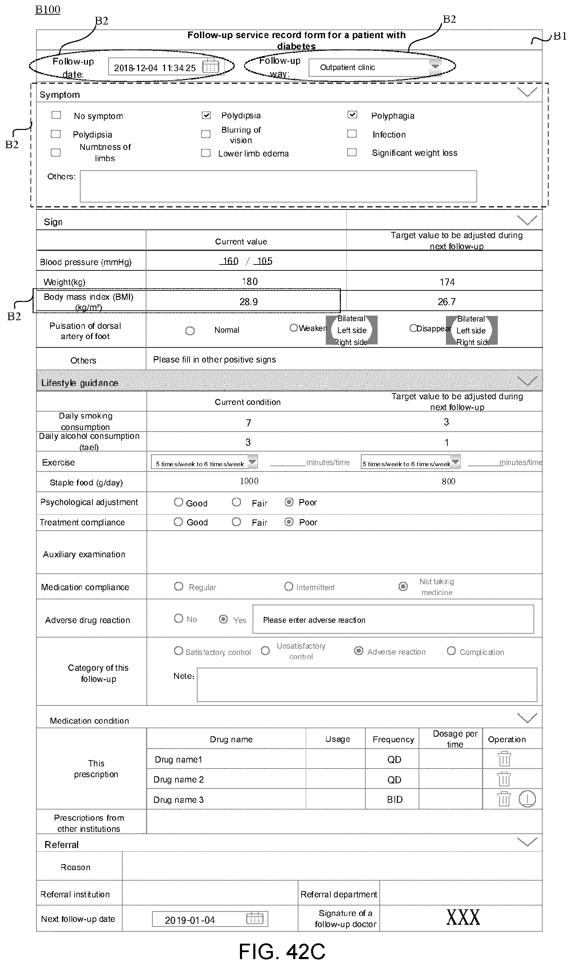

For example, considering the follow-up service record form for the patient with hypertension shown in FIG. 42A and a follow-up service record form for a patient with type 2 diabetes shown in FIG. 42C as examples, the two follow-up record forms B100 include a plurality of common target elements, such as a "follow-up way" target element, and a "follow-up date" target element. Each follow-up record form B100 further includes its own unique elements, for example, the follow-up service record form for the patient with type 2 diabetes shown in FIG. 42C further includes a "heart rate" element, a "salt intake condition" element, and other elements that are unique.

As shown in FIG. 12, a "height" frequently-used field B31b, a "weight" frequently-used field B31b, a "follow-up way" frequently-used field B31b, and a "follow-up date" frequently-used field B31b are all public frequently-used fields B31b. Content of an edited attribute of the public frequently-used field B31b has universality and may be used as a target element in at least two follow-up record forms B100 to be created, and may be reused in different forms. For example, the "follow-up way" frequently-used field B31b may be reused in the follow-up service record form for the patient with hypertension shown in FIG. 42A, and may also be reused in the follow-up service record form for the patient with type 2 diabetes shown in FIG. 42C.

For example, as shown in FIG. 12, the "heart rate" frequently-used field B31b is a private frequently-used field B31b, and may only be used in the follow-up service record form for the patient with type 2 diabetes shown in FIG. 42C, but cannot be used in the follow-up service record form for the patient with hypertension shown in FIG. 42A.

The plurality of frequently-used fields B31b are divided into at least one public frequently-used field B31b and at least one private frequently-used field B31b according to use ranges, which facilitates management of the plurality of frequently-used fields B31b, and facilitates the first user to quickly select a required frequently-used field B31b from a corresponding public frequently-used field B31b set and corresponding private frequently-used fields B31b when creating a follow-up record form, so as to set the frequently-used field B31b in the editing region B4 to generate a target element B2, thereby facilitating rapid making of the follow-up record form and improving efficiency.

In some embodiments, referring to FIGS. 43A to 43E, after S1-22, the method further includes S1-23 to S1-25.

In S1-23, the attribute menu B5 of the basic field B31a is displayed in the editing region B4 in response to the field selection operation, the attribute menu B5 including at least one attribute editing box B51.

For example, as shown in FIG. 43C, the attribute menu B5 of the single-line text box is displayed in the editing region B4 in response to the field selection operation.

In S1-24, the basic field attribute editing operation entered in the at least one attribute editing box B51 is received, the basic field attribute editing operation being configured to edit the attribute of the basic field in each attribute editing box B51.

For example, as shown in FIG. 43C, the basic field attribute editing operation may be an operation in which the first user clicks on and select an attribute editing box B51 with the mouse, and enters corresponding content into the attribute editing box B51 with the keyboard. For example, the plurality of attributes such as the title attribute, the description attribute, the default prompt text attribute, the filling-in setup attributes, and the data source attribute in the attribute menu B5 of the single-line text box are edited. For details, reference may be made to the above description, which will not be repeated herein.

In S25, in response to the basic field attribute editing operation, the edited attribute is displayed in each attribute editing box B51 to generate a target element.

For example, as shown in FIG. 43C, the edited attribute is displayed in each attribute editing box B51, and the "body mass index" element is generated by using the single-line text box. In this case, S1-26, in which it is determined whether the plurality of target elements required by the follow-up record form B100 are all set in the editing region B4, is performed; if not, return to S1-21 to continue to perform the operations in S1-21 to S1-25; and if so, S1-27, in which the follow-up record form is generated, is performed.

Through the operations in S1-21 to S1-25, corresponding target elements may be sequentially generated by using the basic fields B31a, so that a part of the content of the follow-up record form to be made may be formed by using the basic fields B31a to generate the target elements.

After the operations in S1-21 to S1-25 are completed each time, the generated target element may also be saved as the frequently-used field through S1-251 and S1-252 for next use.

In S1-251, the operation of selecting the button of saving as a frequently-used field entered in the attribute menu is received.

For example, as shown in FIGS. 43B and 43C, the attribute menu B5 of the basic field B31a includes the "save" button and the button of "saving as a frequently-used field". The operation of selecting the button of saving as a frequently-used field is the operation performed by the first user. For example, after the "follow-up way" target element is generated by using the basic field B31a in S1-25, the generated target element may be saved as the frequently-used field B31b by clicking on the button of "saving as a frequently-used field".

In S1-252, the generated target element is displayed in the field display region B3 as the frequently-used field in response to the operation of selecting the button of saving as a frequently-used field.

For example, as shown in FIGS. 43B and 43C, the "follow-up way" frequently-used field B31b is displayed in the field display region B3 of the scale making tool interface B200.

Through the operations in S1-251 and S1-252, the target element generated by using the basic field B31a may be saved as the frequently-used field B31b, so that when the follow-up record form is made this time, the saved frequently-used field B31b may be used directly when the target element is generated by using the frequently-used field B31b in S1-21; or, when a new follow-up record form is created next time, the saved frequently-used field B31b may be directly used to facilitate the making of the follow-up record form.

S1-2 further includes S1-21" to S1-23".

In S1-21", the field selection operation entered in the scale making tool interface B200 is received, the field selection operation being configured to select a frequently-used field B31b in the plurality of fields B31. For example, the field selection operation is received, and the frequently-used field B31b selected through the field selection operation is the "follow-up way" frequently-used field B31b.

For example, the frequently-used field B31b may also be the frequently-used field B31b saved in the field display region B3 through S1-33 and S1-34 after the target element is generated by using the basic field B31a in the process of creating the follow-up record form. For example, the "follow-up way" frequently-used field B31b selected through the field selection operation is the "follow-up way" frequently-used field B31b saved in the field display region B3 through S1-33 and S1-34.

In S22", the selected frequently-used field B31a is displayed in the editing region B4 in response to the field selection operation.

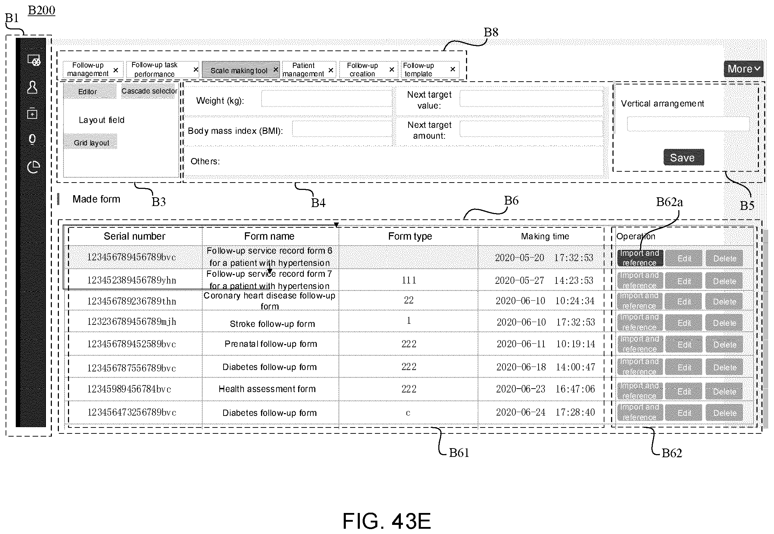

As shown in FIG. 43E, the "follow-up way" frequently-used field is displayed in the editing region B4.

In S23", the frequently-used field B31b displayed in the editing region B4 is used as the target element B2 in response to the field selection operation.

For example, the "follow-up way" frequently-used field B31b is used as the "follow-up way" element. In this case, S1-26, in which it is determined whether the plurality of target elements required by the follow-up record form B100 are all set in the editing region B4, is performed; if not, return to S1-21" to continue to perform the operations in S1-21" to S1-23"; and if so, S1-27, in which the follow-up record form is gereated, is performed.

Through the operations in S1-21" to S1-23", corresponding target elements may be sequentially generated by using the frequently-used fields B31b, so that a part of the content of the follow-up record form to be created may be formed by using the frequently-used fields B31b to generate the target elements.

In the above embodiments, as shown in FIG. 42A, some of the plurality of target elements included in the follow-up record form to be made may be generated by using the frequently-used fields B31b, and the other target elements may be generated by using the basic fields B31b. After the target elements are generated by using the basic fields B31b, the target elements may also be saved as the frequently-used fields to facilitate direct use next time.

S1-21 to S1-25 and S1-21" to S1-23" are in no particular order. According to a determination result in S1-26, S1-21 to S1-25 and S1-21" to S1-23" may be performed for multiple times until the plurality of target elements required by the follow-up record form are all set in the editing region B4.

For example, the follow-up record form to be created is the follow-up service record form for the patient with hypertension shown in FIG. 42A, and the target elements included in the form are the "follow-up date" element, the "follow-up way" element, the "symptom" element, the "body mass index", etc. In the process of creating the follow-up record form B100, the first user first searches the plurality of frequently-used fields B31b in the field display region B3 to see whether there are the corresponding frequently-used fields B31b. For example, as shown in FIG. 43E, the plurality of frequently-used fields B31b include the "follow-up date" frequently-used field B31b, the "follow-up way" frequently-used field B31b, and the "symptom" frequently-used field B31b. Then, the frequently-used fields B31b may be sequentially set in the editing region B4 through S1-21" to S1-23" to generate the corresponding target elements. For the "body mass index" element, the single-line text box in the plurality of basic fields B31a may be selected to perform the operations in S1-21 to S25, and the "body mass index" element generated by using the single-line text box is set in the editing region B4. When creating a form, the first user may set the form according to target elements B2 required by the form and types of fields B31 included in a field display region, which has a high degree of flexibility.

In this way, by combining a way of generating the target element B2 by using the basic field B31a and a way of generating the target element B2 by using the frequently-used field B31b, the creation of the follow-up record form may be quickly completed according to needs of the first user. As a result, the time consumed by the creation of the follow-up record form is shortened, the working efficiency of the first user is improved, and there is no need to adjust internal procedures when different follow-up record forms are created, which reduces a maintenance cost.

In some embodiments, as shown in FIGS. 43D and 43E, the scale making tool interface B200 further includes a form region B6 and a submission column B7. Pages shown in FIGS. 43D and 43E are both the scale making tool interface B200, and the pages shown in FIGS. 43D and 43E may be selected to be displayed through a scroll bar on a right side. As shown in FIG. 43D, the submission column B7 includes a view button, a clear button, a preview button, and a form generation button. The view button is used to view content of elements in the editing region B4, the clear button is used to delete all elements set in the editing region B4, the preview button is used to preview the generated form, and the form generation button is used to submit and save the generated form. As shown in FIG. 43E, the form region B6 includes a plurality of generated forms, and the form region is used to display names of the generated forms.

S1-2 further includes S1-28 and S1-29.

In S1-28, an operation of selecting the form generation button entered by the user in the submission column B7 is received.

For example, after the creation of the follow-up record form B100 is completed, the first user clicks on a form generation option in the submission column B7 to submit and save the generated form.

In S1-29, in response to the operation of selecting the form generation button, the generated follow-up record form B100 is saved, and a name of the saved follow-up record form B100 is displayed in the form region.

The generated follow-up record form B100 is saved in the form region in response to the operation of selecting the form generation button. For example, the generated follow-up record form B100 is the follow-up service record form for the patient with hypertension shown in FIG. 42A, the follow-up record form B100 is saved, and the name of the saved follow-up record form B100, i.e., the "follow-up service record form for the patient with hypertension", is displayed in the form region.

In some examples, before S1-28, the method further includes S1-271 and S1-272.

In S1-271, an operation of selecting the preview button entered in the submission column B7 is received.

For example, after the creation of the follow-up record form B100 is completed, the first user clicks on a preview option in the submission column B7 to preview the generated follow-up record form B100.

In S1-272, the generated follow-up record form B100 is displayed in the editing region B4 in response to the operation of selecting the preview button.

The generated follow-up record form B100 is displayed in the editing region B4, and a style of the follow-up record form B100 may be viewed to determine whether the generated follow-up record form B100 is the same as the follow-up record form B100 to be created, and if not, a position, a format, content, etc. of each element in the generated follow-up record form B100 may be modified in the editing region B4 until satisfaction is achieved.

In some embodiments, as shown in FIG. 43D, the field display region further includes a layout field B31d, which is, for example, a grid layout field B31d, and after S1-272, the method further includes S1-273.

In S1-273, a grid layout menu B5' is displayed in the editing region in response to an operation of selecting at least two target elements B2 in the editing region B4 and in response to an operation of selecting the grid layout field B31d, the grid layout menu B5' including a plurality of layout editing boxes; and in response to an operation of editing the plurality of layout editing boxes, edited content is displayed in the plurality of layout editing boxes, and positions of the selected at least two target elements B2 are adjusted according to the edited content.

For example, as shown in FIG. 43D, the operation of selecting at least two target elements B2 in the editing region B4 by the first user is that, through the mouse and a ctrl key, the first user selects the "follow-up date" target element and the "follow-up way" target element, and then selects the grid layout field B31d to display the grid layout menu B5' in the editing region. The plurality of layout editing boxes included in the grid layout menu B5' are, for example, a grid interval editing box, and column configuration item editing boxes. By editing the plurality of layout editing boxes, the positions, intervals, layouts, etc. of the selected target elements B2 may be adjusted to make a layout of the follow-up record form B100 achieve a desired effect.

In some embodiments, the operation of selecting the preview button is also configured to select a preview effect of the form, and the preview effect includes a paper effect and a display effect on a screen of a mobile terminal.

S1-272, in which the generated follow-up record form is displayed in the editing region B4 in response to the operation of selecting the preview button, includes S1-2721.

In S1-2721, in response to the operation of selecting the preview button, the generated follow-up record form is displayed in the editing region B4 in the paper effect, or in the display effect on a screen of a terminal. Herein, the terminal is the above electronic device, such as the PC or the mobile phone.

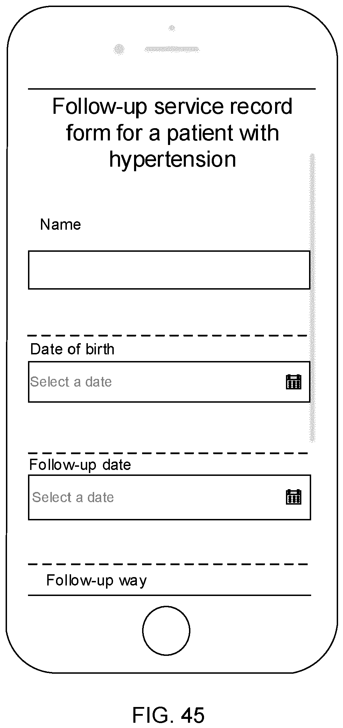

The paper effect refers to, for example, an effect of an entire follow-up record form displayed on a computer screen, which may be presented in a size of a piece of A4 paper or any other piece of paper. For example, effects of the forms shown in FIGS. 42A to 42C are paper effects. The effect displayed on the screen of the terminal refers to, for example, an effect of the follow-up record form displayed on a screen of the mobile phone. For example, an effect of the follow-up record form shown in FIG. 45 is the effect displayed on the screen of the mobile terminal.

In a case where the generated follow-up record form is displayed in the editing region B4 in the display effect on the screen of the mobile terminal, due to a small screen of the mobile phone, the elements included in the generated follow-up record form cannot all be displayed on one page. Then, the plurality of elements may be displayed in sequence according to serial numbers generated by default when the elements are inserted, or the plurality of elements may be set with specific serial numbers and displayed in sequence according to the set serial numbers.

In some embodiments, as shown in FIG. 43E, the form region B6 includes a list sub-region B61 and an operation sub-region B62. The list sub-region B61 includes a name of at least one saved follow-up record form, and the saved follow-up record form includes a plurality of set elements. The operation sub-region B62 includes at least one import reference option B62a, and each saved follow-up record form corresponds to an import reference option B62a. A set element refers to an element whose attributes are all set.

For example, considering an example in which the follow-up record form to be created is the follow-up service record form for the patient with type 2 diabetes shown in FIG. 42C, S1-2 further includes S1-291 and S1-295.

In S1-291, an operation of selecting an import reference option entered in the operation sub-region B62 is received.

For example, the operation of selecting an import reference option is to select an import reference option B62a corresponding to a first form, i.e., the "follow-up record form for the patient with hypertension" in the operation sub-region B62 through clicking with the mouse.

In S1-292, in response to the operation of selecting an import reference option, a saved follow-up record form corresponding to the selected import reference option is displayed in the editing region B4, and the follow-up record form is used as a basic form.

In response to the operation of selecting an import reference option, the follow-up record form for the patient with hypertension is displayed in the editing region B4, and the follow-up record form for the patient with hypertension is used as the basic form. For example, the follow-up record forms shown in FIGS. 43A and 43E are the follow-up record form for the patient with hypertension.

In S1-293, a modification operation entered in the scale making tool interface B200 is received, the modification operation being configured to modify at least one of the plurality of set elements in the basic form, and the at least one set element being not the same as the target element in the follow-up record form to be made.

For example, in the follow-up record form for the patient with hypertension (the basic form) shown in FIG. 42A and the follow-up service record form for the patient with type 2 diabetes (the follow-up record form to be created) shown in FIG. 42C, the "follow-up date" elements of the two forms are the same, the "follow-up way" elements of the two forms are the same, but the "symptom" elements of the two forms are not the same. For example, attributes of the "symptom" element include a title attribute, a description attribute, an option content attribute, and a data source attribute. In the two follow-up record forms, content of the title attributes are both symptom, while in the follow-up record form for the patient with hypertension, the option content attribute of the "symptom" element is "no symptom, headache and dizziness, nausea and vomiting, etc."; and in the follow-up service record form for the patient with type 2 diabetes, the option content attribute of the "symptom" element is "no symptom, polydipsia, polyphagia, etc." Therefore, there is a need to modify at least one of the plurality of set elements in the basic form through a seventh operation, e.g., to modify the option content attribute of the "symptom" element.

In S1-294, in response to the modification operation, the modified at least one set element is set to generate a target element.

For example, in response to the modification operation, the "symptom" element in the follow-up record form for the patient with hypertension is modified to the "symptom" element in the follow-up service record form for the patients with type 2 diabetes.

In S1-295, the modification operation is repeatedly received and responded to until the plurality of set elements in the basic form are the same as the plurality of target elements in the follow-up record form to be made to generate a new follow-up record form.

Through the above operations, the saved follow-up record may be used as the basic form. In a case where the content of the basic form is similar to that of the follow-up record form to be made, by modifying the elements in the basic form that are not the same as the elements in the follow-up record form to be made, a new follow-up record form may be quickly obtained. Thus, there is no need to create a new follow-up record form from scratch by sequentially setting the basic fields B31a and the frequently-used fields B31b to generate the target elements in sequence, which improves a making efficiency of the follow-up record form and saves the time of the user.

In some embodiments, S1-2 further includes: displaying the selected field B31 in the editing region B4 in response to the field selection operation. After the target element is generated by using the field B31, S1-2 further includes: numbering the generated target element, different target elements having different serial numbers.

For example, considering an example in which the target elements in the follow-up record form to be made include a "whether there is a family history of hypertension" element, a "whether there is a family history of diabetes" element, a "people with a family history of diabetes" element, and a "people with a family history of hypertension" element; the "whether there is a family history of hypertension" element and the "whether there is a family history of diabetes" element each have two options, which are a "yes" option and a "no" option; and the "people with a family history of diabetes" element and the "people with a family history of hypertension" element each have six options, which are a grandfather option, a grandmother option, a maternal grandfather option, a maternal grandmother option, a father option, and a mother option.

The above four elements are numbered, for example, serial numbers of the "whether there is a family history of hypertension" element, the "whether there is a family history of diabetes" element, the "people with a family history of diabetes" element, and the "people with a family history of hypertension" element are 1, 2, 3 and 4 respectively.

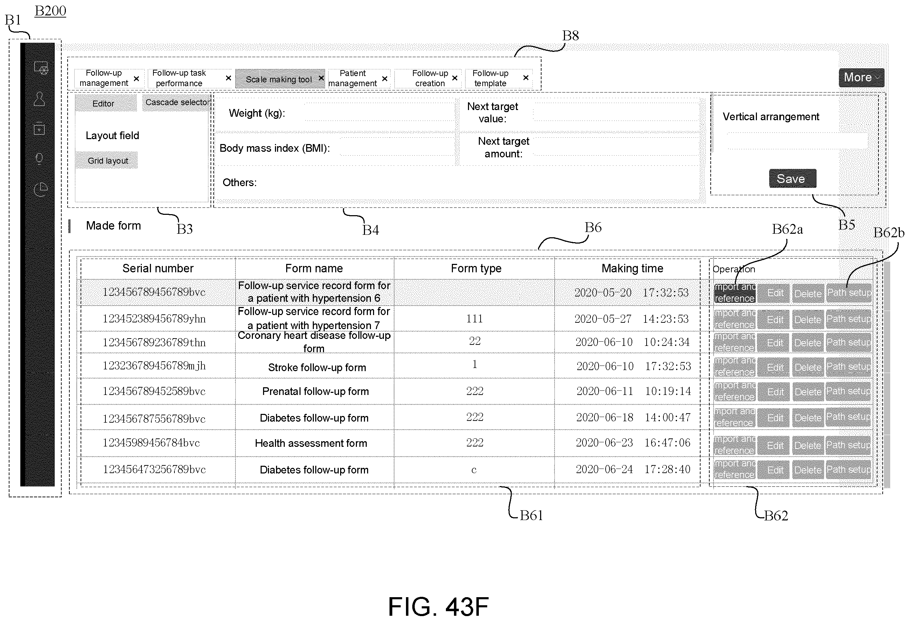

As shown in FIG. 43F, the form region B6 includes the list sub-region B61 and the operation sub-region B62, the list sub-region B61 includes at least one saved follow-up record form B100, and the saved follow-up record form B100 includes a plurality of set elements. The operation sub-region B62 includes at least one path setup option B62b, and each saved follow-up record form B100 corresponds to a path setup option B62b.

S1-2 further includes S1-291' to S1-295'.

In S1-291', a fourth operation entered in the scale making tool interface B200 is received, the fourth operation being configured to select a path setup option B62b in the operation sub-region B62.

For example, the fourth operation is to select a path setup option B62b corresponding to the first follow-up record form B100 in the operation sub-region B62.

In S1-292', in response to the fourth operation, a saved follow-up record form B100 corresponding to the selected path setup option is displayed in the editing region B4, and serial numbers of a plurality of set elements in the follow-up record form B100 are displayed, the plurality of set elements including a first element and a plurality of second elements, and the first element having at least two options.

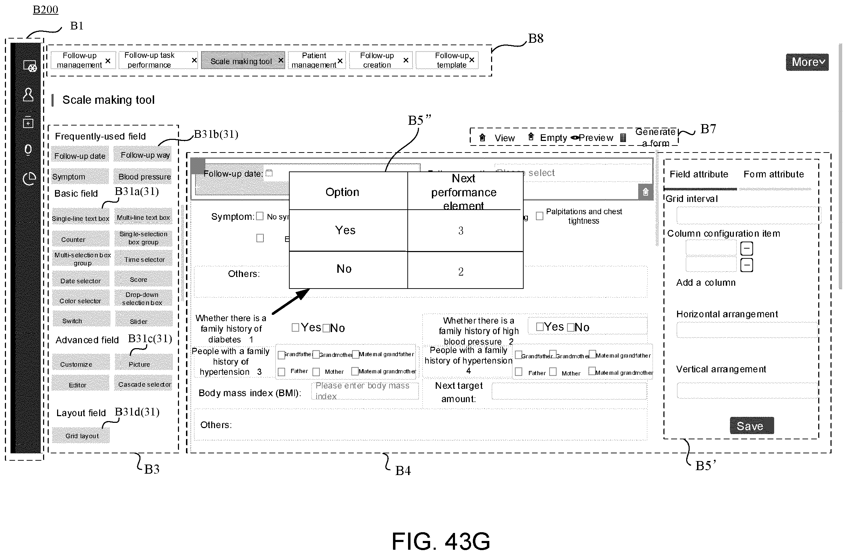

As shown in FIG. 43G, the first element is the "whether there is a family history of diabetes" element, and the second elements are the "whether there is a family history of hypertension" element and the "people with a family history of diabetes" element. Serial numbers of the three elements are 1, 2 and 3. The first element has at least two options, which are the "yes" option and the "no" option.

In S1-292', a fifth operation entered in the scale making tool interface B200 is received, the fifth operation being used to select the first element.

For example, the fifth operation is to double-click on the "whether there is a family history of diabetes" element with the mouse.

In S1-293', a performance path menu of the first element is displayed in response to the fifth operation, the performance path menu including an option list item and a next performance element list item, the option list item including at least two option boxes of the first element, the next performance element list item including at least two path editing boxes, and an editing box corresponding to an option box.

As shown in FIG. 43G, the performance path menu B5" is displayed in the editing region B4 in response to the fifth operation. The performance path menu B5" includes the option list item and the next performance element list item. The two options that the first element has are the "yes" option and the "no" option, and then the option list item includes two option boxes, which are a "yes" option box and a "no" option box. Each option box corresponds to a path editing box. In this case, the path editing box is blank.

In S1-294', a sixth operation entered in the at least two path editing boxes is received, the sixth operation being used to edit a serial number of a next performance element of the first element under different options in each path editing box.

For example, as shown in FIG. 43G, corresponding to the "yes" option box, a path editing box is edited to "3", and corresponding to the "no" option box, a path editing box is edited to "2". That is, if a selection result of the "whether there is a family history of diabetes" element is set to "yes", the "people with a family history of diabetes" element will be performed; and if the selection result of the "whether there is a family history of diabetes" element is "no", the "people with a family history of hypertension" element will be performed.

In S1-295', the edited serial number is displayed in each path editing box in response to the sixth operation.

Through S1-291' to S1-295', setup of element paths between the first element and the second elements is achieved. As a result, when the follow-up task is performed, when the second user fills in the follow-up record form B100, if the "yes" option of the first element is selected, it may be possible to automatically jump to a third element, and then the user fills in the third element; and if the "no" option of the first element is selected, it may be possible to automatically jump to a second element, and then the user fills in the second element. Therefore, there is no need to manually determine a corresponding option, and determine which element is the next performance element, which saves determination time and may facilitate to quickly complete the filling-in of the follow-up record form B100.

In some embodiments, considering a hospital as an example, follow-up record forms B100 used by different departments in an organization are different, and even follow-up record forms used by different persons are also different. In order to avoid confusion, the method further includes: authorizing a right of use of the follow-up record form B100 after the making of the follow-up record form B100 is completed. The authorization may be based on departments or persons, and the follow-up record form B100 is issued after the authorization. In this way, when the follow-up record form B100 is used, for example, when a follow-up task is created, different persons may see corresponding follow-up record forms B100. For example, doctors responsible for the two diseases of hypertension and diabetes may see corresponding follow-up record forms B100.

In some embodiments, the method further includes S1-4.

In S1-4, a follow-up template is created, the follow-up template including a plurality of follow-up task templates.

Based on this, S1-4 includes S1-41 to S1-44.

In S1-41, a follow-up template interface B500 is displayed in response to an operation of the user on a follow-up template tab. Herein, the follow-up template tab is one of the navigation tabs 11, may be presented in the navigation bar 1 in a form of an icon. Of course, the follow-up template tab may further be presented in any other interface of the PC. The embodiments of the present disclosure do not limit a display manner and a display position of the follow-up template tab, as long as a purpose of displaying the follow-up template interface can be achieved.

As shown in FIGS. 45A and 45B, the follow-up template interface B500 includes a template classification selection box B501. The template classification selection box B501 has a disease option menu B502. The disease option menu B502 includes a plurality of disease options. Each disease option corresponds to at least one follow-up template, and each follow-up template includes a plurality of follow-up record forms B100 and a time interval corresponding to each follow-up record form B100. The time interval is a time interval between a current date when the follow-up task is created and a target return visit date. Herein, the disease options in the disease option menu B502 are the same as the disease types included in the disease type selector 301 in the follow-up creation interface described above.

Each disease option may correspond to only one disease, for example, a hypertension disease option corresponds to only the disease of hypertension; or each disease option may correspond to at least two diseases, for example, a stage 1 hypertension disease option corresponds to the two diseases of hypertension and diabetes.

In S1-42, the disease option menu B502 including the plurality of disease options is displayed in response to the operation of creating a follow-up template.

The operation of creating a follow-up template is, for example, that the user clicks on the template classification selection box B501 to make the disease option menu B302 including the plurality of disease options pop up in the template classification selection box 101.

In S1-43, in response to an operation of the user of selecting a disease option, a target disease is determined, and a follow-up template matched with the target disease is created, the follow-up template including a plurality of follow-up record forms and a time interval corresponding to each follow-up record form, each of the plurality of follow-up record forms being a follow-up record form corresponding to the target disease, and the time interval being a time interval between a follow-up date set for the target disease and a date when a follow-up task is created.

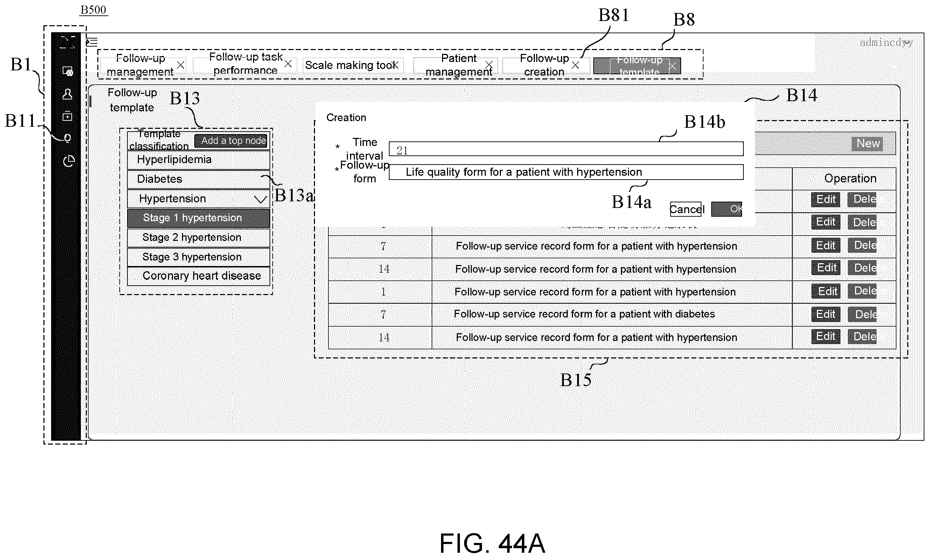

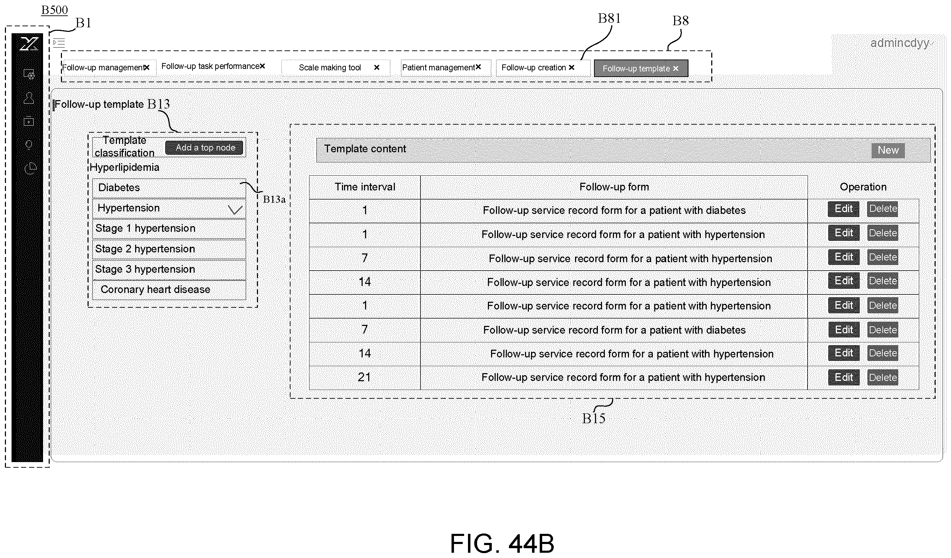

As shown in FIG. 44, a corresponding disease option is selected according to the disease of the target patient. For example, in a case where diseases of the target patient are hypertension and diabetes, the stage 1 hypertension disease option should be selected.

For example, FIG. 45B shows the follow-up template interface B500, template content in the follow-up template interface B500 is, for example, a follow-up template corresponding to the stage 1 hypertension disease option. The follow-up template includes a plurality of follow-up record forms B100 and a time interval corresponding to each follow-up record form B100. For example, a time interval corresponding to a follow-up service record form for the patient with hypertension is 1, and a time interval corresponding to a life quality form for the patient with hypertension is 21.

In S1-44, a plurality of follow-up tasks are created according to the determined target disease. Each follow-up task includes a follow-up record form B100 and a corresponding follow-up time. The follow-up record form B100 is a follow-up record form B100 in the follow-up template corresponding to the selected stage 1 hypertension disease option, and the follow-up time is obtained according to a date when the follow-up task is created and a time interval corresponding to the follow-up record form B100. As shown in FIG. 4B, considering an example in which the date when the follow-up task is created is July 13, 2020, according to the plurality of follow-up record forms B100 and the time interval corresponding to each follow-up record form B100 that are included in the follow-up template in FIG. 5B, the follow-up time corresponding to each follow-up record form B100 may be obtained. For example, in the follow-up template, time intervals corresponding to two follow-up service record forms for the patient with hypertension are both 1, and a time interval corresponding to a follow-up service record form for the patient with diabetes is 1; and then in the plurality of follow-up tasks displayed in a standard template region 10, first three tasks are: a follow-up record form B100 being a follow-up service record form B100 for the patient with hypertension, and a corresponding follow-up time being July 14th, 2020; a follow-up record form B100 being a follow-up service record form B100 for the patient with diabetes, and a corresponding follow-up time being July 14th, 2020; and a follow-up record form B100 being a follow-up service record form B100 for the patient with hypertension, and a corresponding follow-up time being July 14th, 2020. Other tasks may be deduced by analogy, and details will not be repeated herein.

In some embodiments, as shown in FIGS. 44A and 44B, the follow-up template interface B500 further includes a template content column B15, the template content column B15 includes a new button, and the template content column B15 is configured to display a created follow-up template.

Based on this, S1-44 further includes S1-441 to S1-444.

In S1-441, a follow-up template creation dialog box B14 is displayed in the follow-up template interface B500 in response to an operation of selecting the new button, the follow-up template creation dialog box B14 including a follow-up record form editing box, a time interval editing box B14b, an OK button and a cancel button, the follow-up record form editing box B14a having at least one form option related to the target disease, and each form option corresponding to a preset follow-up record form B100.

For example, in a case where the determined target disease is the stage 1 hypertension disease option B13a, the follow-up record form editing box B14a has at least one form option related to the stage 1 hypertension disease option B13a, and each form option corresponds to a generated follow-up record form B100. The follow-up record form B100 may be, for example, a "life quality form for the patient with hypertension", or a "life quality form for the patient with diabetes".

In S1-442, in response to an operation of the user of selecting a form option, a name of a follow-up record form corresponding to the selected form option is displayed in the follow-up record form editing box.

For example, as shown in FIG. 44A, the operation of selecting a form option is to select an option corresponding to the "life quality form for the patient with hypertension" from the at least one form option, and to display the name of the follow-up record form B100 corresponding to the selected form option, i.e., the "life quality form for the patient with hypertension", in the follow-up record form editing box B14a.

In S1-443, an operation of the user of entering a time interval in the time interval editing box, and a set time interval is displayed in the time interval editing box B14b. Herein, the operation of entering the time interval is configured to set a time interval corresponding to a target disease option.

For example, as shown in FIG. 44A, the time interval set in the time interval editing box B14b is displayed as 21 days.

In S1-444, the follow-up template is displayed in the template content column in response to an operation of the user of selecting the OK button.

As shown in FIG. 44B, in a last column in the template content region B15, a record of the created follow-up template is displayed. That is, the time interval is 21 days, and the follow-up record form is a record of the "life quality form for the patient with hypertension". A plurality of records constitute the follow-up template corresponding to the stage 1 hypertension disease option. In some embodiments, referring to FIG. 2, the at least one medical operation index item 102 includes a first detail index item 1021. Herein, the first detail index item 1021 is configured to instruct the user to view the basic information and the diagnosis information of the patient. Based on this, after S2A, the medical information processing method further includes S41.

In S41, in response to an operation of the user on the first detail index item 1021 corresponding to the target patient, for example, clicking on the first detail index item 1021, the PC displays a medical record card interface, and the medical record card information that the target patient filled in when registering the medical record card, such as the basic information, emergency contact information, and the diagnosis information, is presented in the medical record card interface, which facilitates the medical worker to know the detailed information of the patient or to modify the medical record card information of the target patient.

In some embodiments, referring to FIG. 2, the patient list interface 100 further includes a patient search box 103. The patient search box 103 is configured to instruct the user to search for the target patient or other patients. For example, as shown in FIG. 2, the patient search box 103 includes a medical record number input box, a name input box, a mobile phone number input box, and other input boxes. The method further includes determining the target patient or other patients by the user in response to an operation of querying information entered in the patient search box 103. In this way, the user may directly determine the target patient through the search patient box 103 instead of searching one by one in the patient list 101, which improves the working efficiency of the user.

In some other embodiments, referring to FIG. 2, the patient list interface 100 further includes a disease type option box 104, which is configured to instruct the user to filter a patient through a disease type to improve an efficiency of the user in searching for the patient.

In some embodiments, as shown in FIG. 2, a card registration and creation index item 105 is further presented in the patient list interface 100, and the card registration and creation index item 105 is configured to instruct the user to create a medical record card for a new patient. Based on this, after S2A, the medical information processing method further includes S41 and S41.

In S41, the PC displays at least basic information blank text boxes and a diagnosis information blank text box in response to an operation of the user on the card registration and creation index item 105, the basic information blank text boxes being configured to present basic information of the new patient, and the diagnosis information blank text box being configured to present diagnosis information of the new patient.

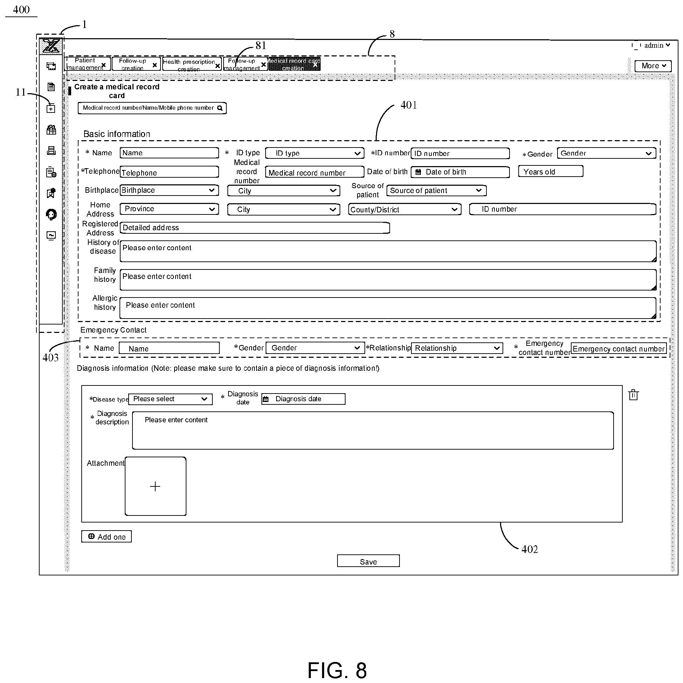

For example, as shown in FIG. 8, the operation of the user on the card registration and creation index item 105 may be understood as that the user clicks on the card registration and creation index item 105. The PC displays a medical record card creation interface 400 in response to the operation. The medical record card creation interface 400 includes a plurality of basic information blank text boxes 401, an emergency contact blank text box 403, and a diagnosis information blank text box 402.

As shown in FIG. 8, the plurality of basic information blank text boxes 401 are configured to enter the basic information of the new patient, such as a name, an ID type, an ID number, a gender, a phone number and other required information, as well as a home address, a disease history, a family history, an allergy history, and other optional information, which facilitates the medical worker to know the basic condition of the new patient; moreover, the required information in the basic information is configured to distinguish different patients. For example, when the user searches for the new patient through the search box 103 in the above embodiments, information in the patient search box may include part of the required information that is in unique correspondence with the new patient, such as the name, the ID number or the phone number.

The emergency contact blank text box 403 is configured to enter information of an emergency contact who is in an intimate relationship with the new patient, such as a parent, a spouse or a child of the new patient.

The diagnosis information blank text box 402 is configured to enter the diagnosis information of the new patient, which includes a diagnosis date, a diagnosed disease type, and a diagnosis description to record the disease information of the new patient. Herein, there is one diagnosis information blank text box 402. Of course, in a case where the new patient has two or more diseases, the diagnosis information blank text boxes may be added to record other disease information of the patient.

In S42, in response to an operation of the user of saving the information presented in the basic information blank text boxes 401 and the diagnosis information blank text box 402, the PC adds at least part of the information presented in the basic information blank text boxes 401 and the diagnosis information blank text box 402 as visit information into the patient list 101.

Herein, the operation of the user of saving the information presented in the basic information blank text boxes 401 and the diagnosis information blank text box 402 may be understood as that the user clicks on a save button in the medical record card creation interface 400 after filling in the basic information, emergency contact information, and diagnosis information of the patient. For example, as shown in FIG. 2, the visit information of the patient in the patient list 101 includes the medical record number, name, gender, age, mobile phone number, and disease type. Then, in response to the saving operation, the PC adds the medical record number, name, gender, age, and mobile phone number presented in the basic information blank text boxes 401, as well as the diagnosed disease type presented in the diagnosis information blank text box 402 as the visit information into the patient list 101 (e.g., being presented in a first line in the patient list 101), and associates all information presented in the medical record card creation interface 400 in the first detail index item 1021 corresponding to the new patient in the patient list 101, so as to facilitate the user to view the medical record card information of the patient through the first detail index item 1021.

It will be noted that, the medical record number uniquely corresponding to the patient may be manually entered by the user when the medical record card is created, or may be automatically generated by the PC in response to the saving operation.

In some embodiments, the at least one navigation tab 11 includes a card registration and creation tab, and S2 further includes: displaying, by the PC, the medical record card creation interface 400 in response to a first operation of the user on the card registration and creation tab (e.g., the user clicking on an icon representing the card registration and creation tab in the navigation bar 1). Herein, the medical record card creation interface 400 is the same as the medical record card creation interface 400 displayed in S41. Based on this, the medical information processing method further includes S41 and S42.

In some embodiments, the at least one navigation tab 11 includes a health prescription management tab. S2 includes S2B.

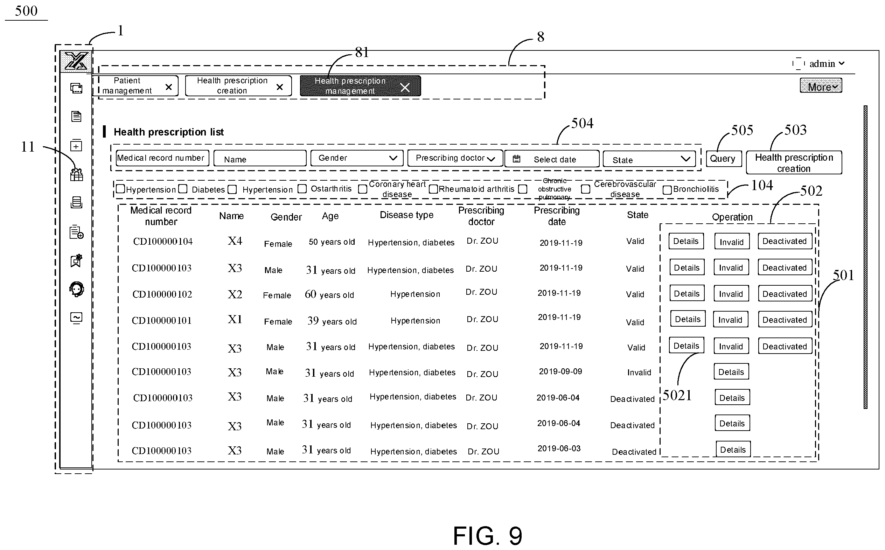

In S2B, as shown in FIG. 9, the PC displays a health prescription management interface 500 in response to a first operation of the user on the health prescription management tab, a health prescription list 501 being presented in the health prescription management interface 500, the health prescription list 501 including a plurality of lines of prescription information, each line of prescription information including visit information of a patient, a prescribing doctor, a prescribing date, and a first medical information operation item 502, and the first medical information operation item 502 including a second detail index item 5021.

Herein, the first operation of the user on the health prescription management tab may be understood as that the user clicks on an icon representing the health prescription management tab in the navigation bar 1. Patients in a plurality lines of prescription information may be a same patient. For example, as shown in FIG. 9, a plurality lines of prescription information includes six different pieces of prescription information of the same patient X3.

Based on this, the medical information processing method further includes S51.

In S51, the PC displays a health prescription text box 201 in response to an operation of the user on the second detail index item 5021, a medical regimen corresponding to the line of prescription information being displayed in the health prescription text box 201, and the medical regimen including at least one of a medication regimen, a diet regimen, or an exercise regimen.

In some examples, the PC displays the health prescription creation interface 200 in response to an operation of the user of clicking on a second detail index item 5021 in the health prescription list 501. What is different from the health prescription creation interface 200 displayed in S26 (i.e., the health prescription creation interface 200 shown in FIGS. 3 to 5) is that, the created medical regimen is presented in the health prescription text box 201 in the health prescription creation interface 200, that is to say, the second detail index item 5021 is configured to instruct the user to view the created medical regimen corresponding to the line of prescription information.

In some embodiments, as shown in FIG. 9, each line of prescription information further includes state information, and the state information is being valid, being deactivated, or being invalid. In a case where state information in a line of prescription information is being valid, a first medical information operation item 502 in the line of prescription information further includes a deactivation item and an invalidation item. For example, state information of first five lines of prescription information in the health prescription list 501 shown in FIG. 9 are all being valid, and then a first medical information operation item corresponding to each of the five lines of prescription information operation item 502 includes a second detail index item 5021, a deactivation item and an invalidation item. That is to say, in a case where state information in a certain line of prescription information is being valid, the certain line of prescription information may be invalidated or deactivated. In addition, in response to an operation of the user of clicking on a second detail index item 5021 in one of the five lines of prescription information, the PC displays a medical regimen corresponding to the line of prescription information, and the medical regimen is in an editable state, and may be modified by the user.

Based on this, the medical information processing method further includes S52.

In S52, in response to an operation of the user on the deactivation item or the invalidation item, the PC changes the state information in the line of prescription information from being valid to being deactivated or being invalid, and hides the deactivation item and the invalidation item. For example, in response to an operation of the user of clicking on a deactivation item in a last line of prescription information in the health prescription list 501 shown in FIG. 9, the PC changes state information in the line of prescription information from being valid to being deactivated, and hides a deactivation item and an invalidation item in a first medical information operation item 502, and displays only a second detail index item 5021.

In addition, the PC displays a medical regimen corresponding to the line of prescription information in response to an operation of the user of clicking on the second detail index item 5021 in the line of prescription information, and the medical regimen is in a non-edited state, and the user may only view the medical regimen.

In some embodiments, as shown in FIG. 9, a prescription information search box 504 and a query item 505 are further presented in the health prescription management interface 500. The prescription information search box 504 includes a medical record number input box, a name input box, a prescribing doctor selection item and a prescribing date selection item. The prescription information search box 504 is configured to present search information, which includes a medical record number, a name, a prescribing doctor, or a prescribing date. The query item 505 is configured to instruct search for the search information presented in the prescription information search box 504. For example, if the user needs to search for a health prescription of the patient X3, then the user enters a name or a medical record number of the patient X3 in the prescription information search box 504, and the PC responds to the name or the medical record number of the patient X3 presented in the prescription information search box 504.

In some other embodiments, referring to FIG. 9, the health prescription management interface 500 further includes a disease type option box 104, which is configured to instruct the user to filter prescription information through a disease type to improve an efficiency of the user in searching for a prescription. For example, if the user needs to find health prescription information about hypertension created by Dr. Zhang on November 19, 2019, then the user selects Dr. Zhang in the prescribing doctor selection item in the prescription information search box 504, selects November 19, 2019 in the prescribing date selection item, and checks an option box corresponding to hypertension in the disease type option box 104; then, the PC displays only the health prescription information matched with hypertension created by Dr. Zhang on November 19, 2019 in the health prescription list 501, in response to operations of querying Dr. Zhang and November 19, 2019 presented in the prescription information search box 504, and hypertension selected in the disease type option box 104.

In some embodiments, as shown in FIG. 9, a new health prescription creation index item 503 is further presented in the health prescription management interface 500. Based on this, the medical information processing method further includes S53 to S56.

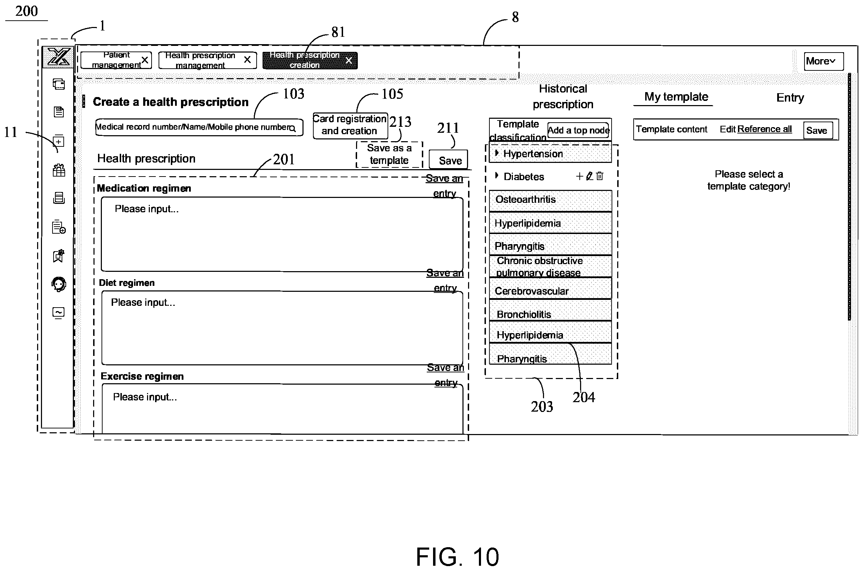

In S53, as shown in FIG. 10, the PC displays the health prescription creation interface 200 in response to an operation of the user on the new health prescription creation index item 503. What is different from the health prescription creation interface 200 shown in S26 (i.e., the health prescription creation interface 200 shown in FIGS. 3 to 5), the health prescription creation interface 200 herein further includes a patient search box 103.

In S54, in response to an operation of querying information entered in the patient search box 103, the PC determines the target patient, and displays the visit information of the target patient. For example, the user enters a name X3 and a medical record number CD10000103 in the patient search box 103, and in response to an operation of querying the name X3 and the medical record number CD10000103 entered in the patient search box 103, the PC determines that the target patient is X3, and displays visit information of the target patient X3 in the health prescription creation interface 200 shown in FIG. 10.

In S55, the PC presents the medical regimen matched with the disease of the target patient in the health prescription text box 201 in response to the second operation of the user.

Herein, S55 is the same as S26, and the second operation of the user may also be understood as that the user fills in the medical regimen in the blank health prescription text box 201, or the user presents the medical regimen matched with the disease of the target patient in the blank health prescription text box 201 in the way of referencing an entry text template, referencing a medical regimen template, or reusing a historical prescription described above (i.e., S261 to S263, S264 to S266 or S267 to S269).

In S56, in response to an operation of the user of saving the medical regimen, the PC adds the visit information of the target patient as part of a new line of prescription information into the health prescription list 501 in the health prescription management interface 500, and associates the medical regimen of the target patient in a second detail index item 5021 in the new line of prescription information.

In some embodiments, the at least one navigation tab 11 includes a new health prescription creation tab. S2 includes S2C.

In S2C, the PC displays the health prescription creation interface 200 in response to a first operation of the user on the new health prescription creation tab (e.g., the user clicks on an icon representing the new health prescription creation tab in the navigation bar 1). The health prescription creation interface 200 displayed herein is the same as the health prescription creation interface 200 displayed in S53. Based on this, the medical information processing method further includes S54 to S56.

In some embodiments, referring to FIG. 10, a card registration and creation index item 105 is further presented in the health prescription creation interface 200. Herein, the card registration and creation index item 105 is the same as the card registration and creation index item 105 in the patient list interface 100 shown in FIG. 2, both of which are configured to instruct the user to create the medical record card for the new patient. Based on this, the medical information processing method further includes S41 and S42, which will not be repeated herein.

It will be noted that, the card registration and creation index item may further be displayed in a form of a tab, for example, it is displayed in the navigation bar 1 in a form of a navigation tab 11, or may be displayed in other ways. The embodiments of the present disclosure do not limit this, as long as a purpose of registering medical record card information for the new patient may be achieved.

In some embodiments, if in S54, the PC does not determine the target patient in response to the operation of querying the information entered in the patient search box 103, the target patient is considered to be the new patient. Based on this, the PC first performs S41 and S42 to create the medical record card for the new patient, and then re-performs S54 to S56 in response to an operation of returning to the health prescription creation interface 200; or, the PC first performs S2A, in which case information in the first line in the patient list 101 is the information of the new patient, and then the PC performs S22 and S26 to create a health prescription for the new patient.

Herein, the operation of returning to the health prescription creation interface 200 may be understood as that the user clicks on an interface tab 81 appearing in a navigation pane 8 in interface tabs 81 in the navigation pane 8 when the PC performs S54, for example, a health prescription creation interface tab in the navigation pane 8 shown in FIG. 10; or, the user clicks on an icon representing the health prescription creation interface tab in the navigation bar 1. Of course, it may also be any other operation, which is not enumerated in the embodiments of the present disclosure, as long as through the operation, the health prescription text box 201 may be displayed, and the health prescription may be created for the patient in the health prescription text box 201.

It will be noted that, the above interfaces each include a navigation pane 8. The navigation pane 8 includes at least one interface tab 81, an interface tab 81 corresponds to an interface, and a display state of an interface tab 81 corresponding to a current interface is different from display states of interface tabs 81 corresponding to other interfaces. For example, referring to FIG. 10, an interface tab 81 corresponding to a current interface displayed on the PC is the health prescription creation interface tab, which means that a display color of the health prescription creation interface tab 81 is darker than display colors of other interface tabs 81, thereby facilitating the user to distinguish different interfaces through the interface tabs to achieve switching between different interfaces.

In some embodiments, the at least one navigation tab 11 includes a follow-up management tab. S2 includes S2D.

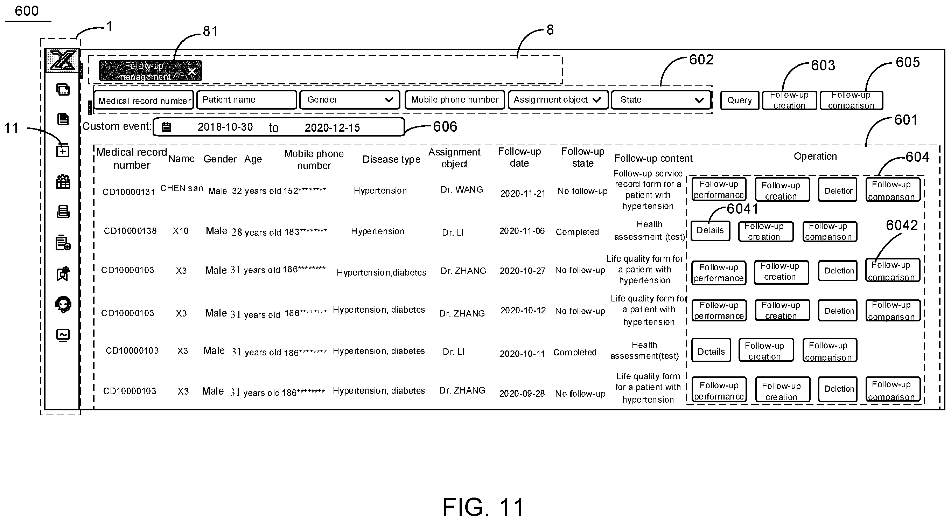

In S2D, as shown in FIG. 11, the PC displays a follow-up management interface 600 in response to a first operation of the user on the follow-up management tab, the follow-up task list 601 being presented in the follow-up management interface 600, the follow-up task list 601 including a plurality of lines of follow-up information, and each line of follow-up information including visit information of a patient, a follow-up time, follow-up content, and at least one second medical information operation item 604.

Herein, the first operation of the user on the follow-up management tab may be understood as that the user clicks on an icon representing the follow-up management tab in the navigation bar 1. Patients in the plurality of lines of follow-up information may be a same patient. For example, as shown in FIG. 11, the plurality of lines of follow-up information include four different pieces of follow-up information of the same patient X3.

In some embodiments, as shown in FIG. 11, each line of follow-up information further includes a follow-up state, which includes no follow-up or being completed. In a case where a follow-up state in a line of follow-up information is no follow-up, the at least one second medical information operation item 604 in the line of follow-up information includes a follow-up performance index item and a delete item. For example, a follow-up state in a first line of follow-up information in the follow-up task list 601 shown in FIG. 11 is no follow-up, and then a second medical information operation item 604 in the line of follow-up information includes a follow-up performance index item and a delete item. That is to say, in a case where a certain follow-up task is not performed, the user may delete the follow-up task.

Based on this, the medical information processing method further includes S61.

In S61, the PC displays a follow-up record form corresponding to follow-up content in response to an operation of the user on the follow-up performance index item.

For example, as shown in FIG. 12, the PC displays the follow-up task performance interface 700 in response to an operation of clicking on a follow-up performance index item in a fourth line of follow-up information in the follow-up task list 601, and a follow-up service record form for a patient with hypertension corresponding to follow-up content in the line of follow-up information is presented in the follow-up task performance interface 700.

In S62, as shown in FIG. 11, in response to an operation of saving content entered in the follow-up service record form for the patient with hypertension, the PC changes a follow-up state in the line of follow-up information from no follow-up to being completed, and changes the follow-up performance index to a third detail index item 6041, and hides a delete item. The third detail index item 6041 is configured to instruct to view a follow-up record form corresponding to a follow-up task. Content in the follow-up record form is content filled in by a medical worker during a follow-up process, and the content is in a non-edited state. That is to say, after the follow-up task is performed, the user may only view the content filled in in the follow-up record form during the follow-up process.

In some examples, as shown in FIG. 12, the follow-up service record form for the patient with hypertension includes "category of this follow-up", which includes options such as satisfactory control, unsatisfactory control, adverse reaction or complication. During the process of following up the patient by the medical worker, one or more of the options such as satisfactory control, unsatisfactory control, adverse reaction, and complication may be selected according to health condition of the patient, for example, satisfactory control is selected.

In some embodiments, the second medical information operation item 604 further includes a first follow-up comparison index item 6042. Based on this, the medical information processing method further includes S63.

In S63, in response to an operation of the user on the first follow-up comparison index item 6042, the PC acquires and displays comparison results of at least two follow-up contents matched with a same disease corresponding to the patient in the line of follow-up information.

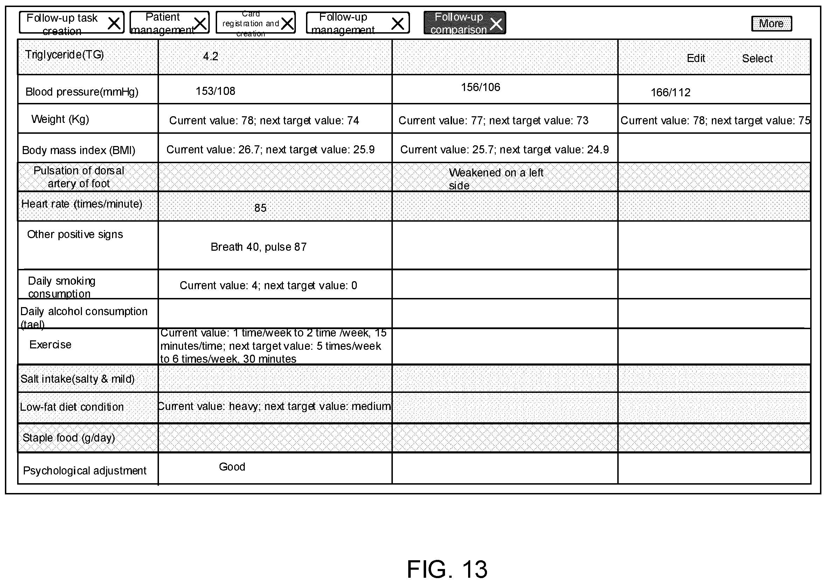

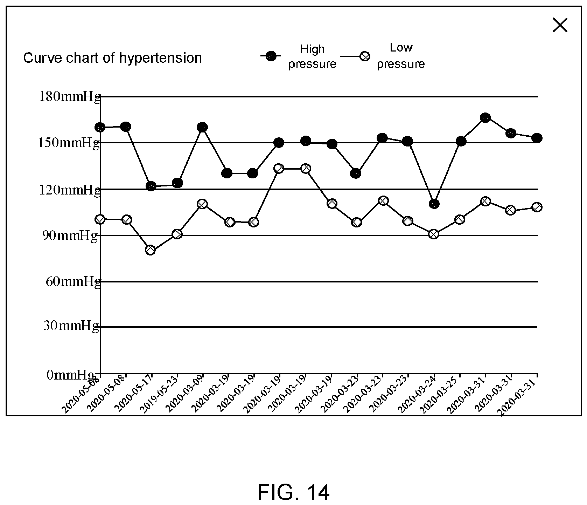

In some examples, as shown in FIG. 11, the follow-up content in the fourth line of follow-up information in the follow-up task list 601 is the follow-up service record form for the patient with hypertension, and the patient X3 in the line of follow-up information has completed the follow-up service record form for the patient with hypertension matched with hypertension more than two times before a current day. As shown in FIG. 12, the follow-up service record form for the patient with hypertension includes physical sign information such as blood pressure, weight, body mass index, and heart rate, as well as other information, such as daily smoking consumption and daily alcohol consumption. In this way, the PC acquires and displays comparison results of blood pressures, heart rates, and other information in a plurality of follow-up service record forms for the patient with hypertension in response to the operation of the user on the first follow-up comparison index item 6042, so that the medical worker may know control condition of the disease of the patient clearly and intuitively through the comparison results. For example, as shown in FIG. 13, in response to the operation of the user on the first follow-up comparison index item 6042, the PC acquires and displays comparison results of physical sign information such as blood pressures, weights, body mass indexes, and heart rates, and other information in a plurality of follow-up service record forms for the patient with hypertension, and displays the comparison results in a form of a form. For another example, as shown in FIG. 14, in response to the operation of the user on the first follow-up comparison index item 6042, the PC acquires and displays a comparison result of blood pressures in a plurality of follow-up record forms for the patient with hypertension, and displays the comparison result in a form of a trend chart. A presentation way of the comparison result(s) is not specifically limited in the embodiments of the present disclosure, and may be adjusted according to actual needs.

In some other embodiments, a second follow-up comparison index item 605 is further presented in the follow-up management interface 600. For example, the second follow-up comparison index item 605 is similar to the first follow-up comparison index item 6042, and what is different from the first follow-up comparison index item 6042 is that the second follow-up comparison index item 605 is configured to acquire and display comparison results of at least two follow-up contents matched with a same disease corresponding to a target patient. The target patient herein may be any patient for whom medical record card information has been created.

In some embodiments, the second medical information operation item 604 includes a follow-up creation index item. Herein, the follow-up creation index item is the same as the follow-up creation index item in the patient list 101 in the patient management interface 100, both of which are configured to instruct the user to create a follow-up task for a patient corresponding to the follow-up creation index item.

Based on this, the medical information processing method further includes S31 to S36. In addition, the third operation of the user in S36 may also be understood as that the user selects a preset follow-up task template 311 in the follow-up task template region 303 in the follow-up creation interface 300 (i.e., S361), or the user creates a follow-up task in the custom way in the custom region 310 in the follow-up creation interface 300 (i.e., S362 or S363).

In some other examples, after S36, the medical information processing method further includes S37.

In S37, in response to an operation of the user of saving a newly-created follow-up task, the PC adds the follow-up task and visit information of a corresponding patient into the follow-up task list 601.

In some embodiments, as shown in FIG. 11, a follow-up task search box 602 and a follow-up query item 607 are further presented in the follow-up management interface 600. The follow-up task search box 602 includes a medical record number input box, a name input box, a mobile phone number input box, a follow-up person selection item, and a state selection item. The follow-up task search box 602 is configured to present follow-up search information. The follow-up search information includes a medical record number, a patient name, a mobile phone number, a follow-up person, and a follow-up state. The follow-up query item 607 is configured to instruct search for the follow-up search information presented in the follow-up task search box 602.

In some other embodiments, as shown in FIG. 11, a custom time option 606 is further presented in the follow-up management interface 600, and the user may filter a follow-up date through the custom time option 606 to obtain a follow-up task with a follow-up date within a period of time.

In some embodiments, as shown in FIG. 11, a new follow-up creation index item 603 is further presented in the follow-up management interface 600. Based on this, the medical information processing method further includes S64 to S66.

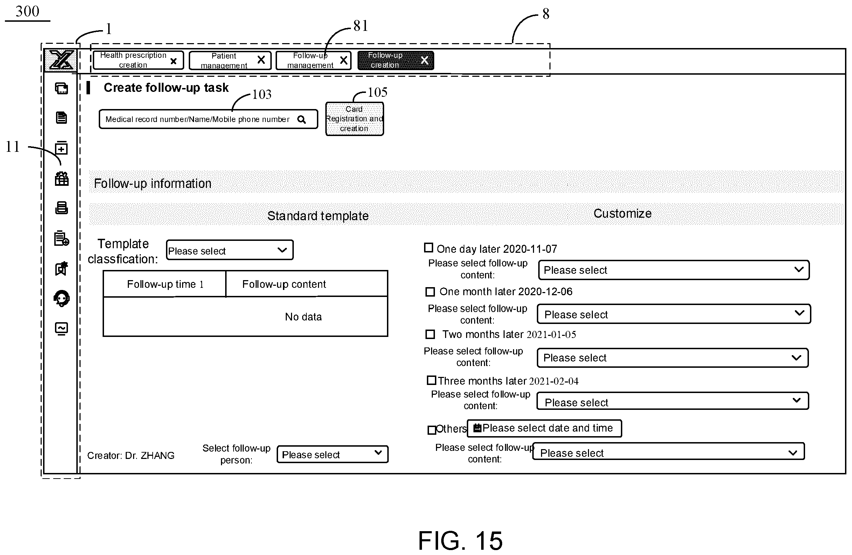

In S64, as shown in FIG. 15, the PC displays the follow-up creation interface 300 in response to an operation of the user on the new follow-up creation index item 603. What is different from the follow-up creation interface 300 displayed in S31 (i.e., the follow-up creation interface 300 shown in FIGS. 6 and 7) is that the follow-up creation interface 300 herein further includes a patient search box 103.

In S65, in response to an operation of querying information entered in the patient search box 103, the PC determines the target patient and displays the visit information of the target patient. For example, the user enters the name X3 and the medical record number CD10000103 in the patient search box 103, and in response to an operation of querying the name X3 and the medical record number CD10000103 entered in the patient search box 103, the PC determines that the target patient is X3, and displays the visit information of the target patient X3 in the health prescription creation interface 200 shown in FIG. 10.

In S66, the PC determines a new follow-up task matched with the disease of the target patient in response to the third operation of the user, the new follow-up task including follow-up content matched with the disease of the target patient and a follow-up time corresponding to the follow-up content.

Herein, S66 is the same as S36, and the third operation of the user may also be understood as that the user selects a preset follow-up task template 311 in the follow-up task template region 303 in the follow-up creation interface 300 (i.e., S361), or the user creates a follow-up task in the custom way in the custom region 310 in the follow-up creation interface 300 (i.e., S362 or S363), which will not be repeated herein.

In S67, in response to an operation of the user of saving the new follow-up task, the PC adds the visit information of the target patient and the new follow-up task into the follow-up task list 601.

Herein, S67 is the same as S37.

In some embodiments, the at least one navigation tab 11 includes a new follow-up creation tab. S2 includes S2E.

In S2E, the PC displays the follow-up creation interface 300 in response to a first operation of the user on the new follow-up creation tab (e.g., the user clicking on an icon representing the new follow-up creation tab in the navigation bar 1). The follow-up creation interface 300 displayed herein is the same as the follow-up creation interface 300 displayed in S64. Base on this, the medical information processing method further includes S64 to S66.

In some embodiments, referring to FIG. 15, the card registration and creation index item 105 is further presented in the follow-up creation interface 300. Herein, the card registration and creation index item 105 is the same as the card registration and creation index item 105 in the patient list interface 100 shown in FIG. 2, both of which are configured to instruct the user to create the medical record card for the new patient. Based on this, the medical information processing method further includes S41 and S42, which will not be repeated herein.

In some embodiments, if in S65, the PC does not determine the target patient in response to the operation of querying the information entered in the patient search box 103, the target patient is considered to be the new patient. Based on this, the PC first performs S41 and S42 to create the medical record card for the new patient, and then re-performs S65 to S67 in response to an operation of returning to the follow-up creation interface 300; or, the PC first performs S2A, in which case the information in the first line in the patient list 101 is the information of the new patient, and then the PC performs S31 and S36 to create a follow-up task for the new patient.

Herein, the operation of returning to the follow-up creation interface 300 may be understood as that the user clicks on an interface tab 81 appearing in the navigation pane 8 when the PC performs S65 in the interface tabs 81 in the navigation pane 8 when the PC performs S65, for example, a follow-up creation interface tab in the navigation pane 8 shown in FIG. 10; or, the user clicks on an icon representing the follow-up creation interface tab in the navigation bar 1. Of course, it may also be any other operation, which is not enumerated in the embodiments of the present disclosure, as long as through the operation, the follow-up creation interface 300 may be displayed, and the follow-up task may be created for the patient in the follow-up creation interface 300.

In some embodiments, the medical information processing method further includes S01 to S02.

In S01, the PC displays a login information box.

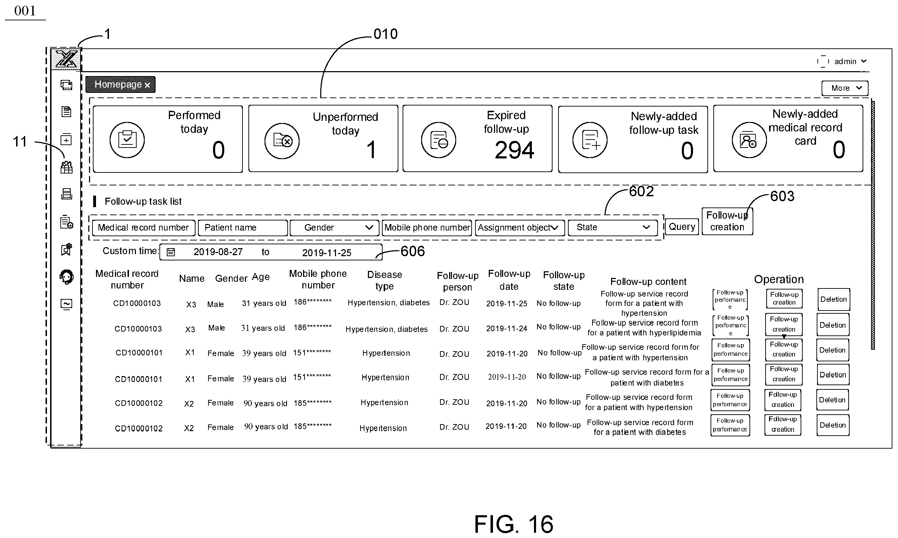

In S02, as shown in FIG. 16, the PC displays a homepage interface 001 in response to an operation of logging in login information entered in the login information box, work content and workload of the user on a current working day being presented in the homepage interface 001.

In some examples, login information of Dr. Zou as the user is entered in the login information box. In response to an operation of logging in the login information of Dr. Zou, the PC displays the homepage interface 001, and a workload reminder information column 010 of Dr. Zou on a current working day is presented in the homepage interface 001 to remind the user of the workload that needs to be completed today.

The workload reminder information column 010 includes workload that has not been performed today, for example, the number of follow-up tasks that have not been performed. Based on this, a follow-up task list 601 that has not been performed is further presented in the homepage interface 001 to facilitate the medical worker to view information of specific follow-up tasks.

It will be noted that, what is different from the follow-up task list 601 presented in the follow-up management interface 600 is that in the follow-up task list 601 presented in the follow-up management interface 600, all follow-up tasks of all patients are presented, follow-up persons of which may be a same doctor or different doctors, while in the follow-up task list 601 presented in the homepage interface 001, only follow-up tasks of a follow-up person who is a logged-in user (i.e., Dr. Zou) are displayed.

In some other examples, if the login information does not exist, that is, the user is a new user, the PC displays a registration information box in response to determining that the login information does not exist; and transmits registration information to a server in response to an operation of registering the registration information entered in the registration information box. The registration information includes login information, that is, part of the registration information is used as the login information of the user.

In some embodiments, the at least one navigation tab 11 includes a disease assessment tab. S2 includes S2F.

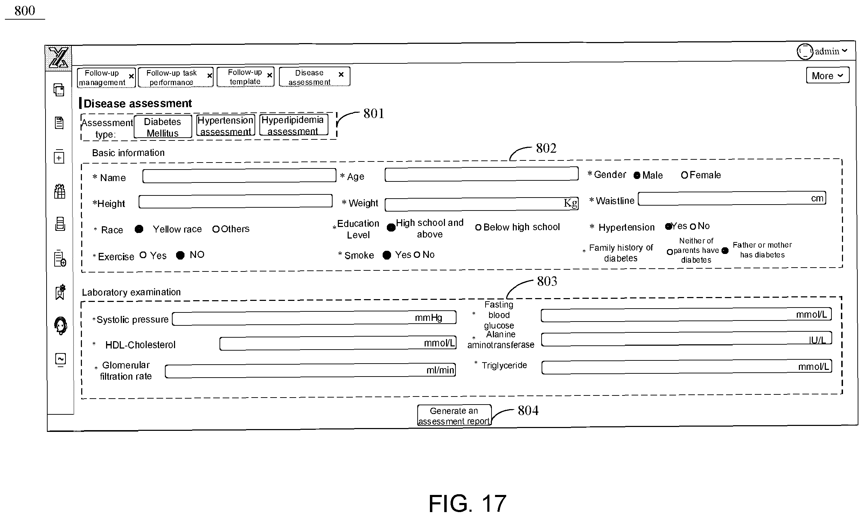

In S2F, as shown in FIG. 17, the PC displays a first disease assessment interface 800 in response to a first operation of the user on the disease assessment tab, a disease assessment information text box, which includes an assessment type option 801, a basic information text box 802, and a laboratory examination information text box 803, being presented in the first disease assessment interface 800. Herein, the assessment type option 801 includes a plurality of disease types, and part of information in the basic information text box 802 and examination items in the laboratory examination information text box 803 are matched with one of the diseases in the assessment type option.

Based on this, the medical information processing method further includes S71 to S72.

In S71, in response to a determined assessment type, the PC matches information in the basic information text box 802 and examination items in the laboratory examination information text box 803 with a disease corresponding to the determined assessment type.

In S72, in response to an operation of saving information entered in the disease assessment information text box, the PC automatically acquires and displays a disease assessment report corresponding to disease assessment information according to a knowledge base in the server.

For example, referring to FIG. 17, the assessment type option 801 presented in the first disease assessment interface 800 includes diabetes assessment, hypertension assessment, and hyperlipidemia assessment. Of course, the assessment type option 801 may further include other disease assessment. FIG. 17 only shows the diabetes assessment, hypertension assessment, and hyperlipidemia assessment as examples. If the user selects the diabetes assessment, in addition to information such as a name, an age, a gender, a height, a weight, a race, and an education level, information in the basic information text box 802 further includes information such as a waistline, family history of diabetes, exercise amount, and smoking or not; and examination items in the laboratory examination information text box 803 include fasting blood glucose, two hours postprandial blood glucose, systolic blood pressure, cholesterol, low-density lipoprotein, high-density lipoprotein and triglyceride, since the patient with diabetes may also have hypertension, etc. In this way, the assessment report may be made more accurate.

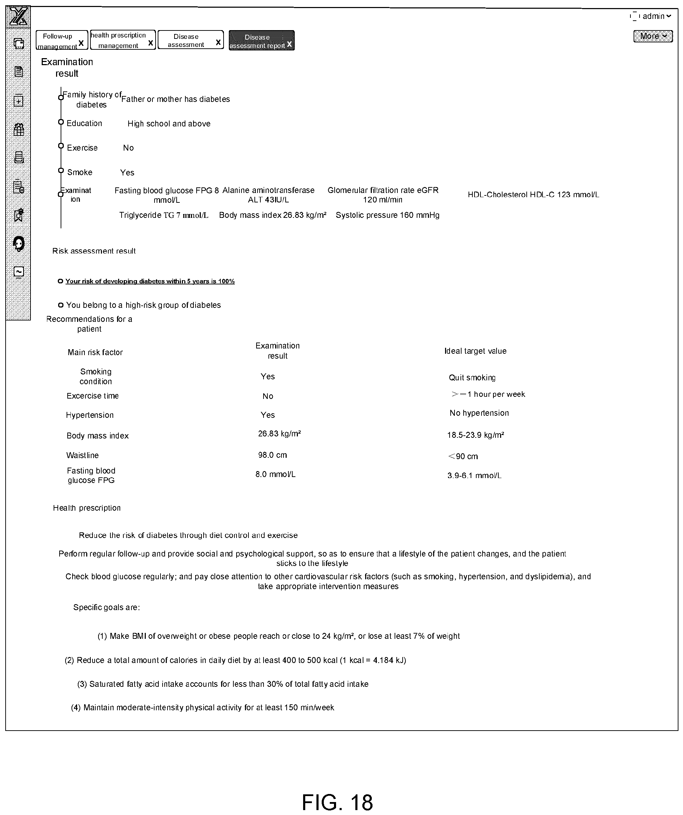

As shown in FIG. 18, the disease assessment report includes a risk assessment result and health advice for the patient, so that the patient may know his or her own health condition through the disease assessment result. If the risk assessment result shows that there is a small probability for the patient to suffer from diabetes, but there is stll a risk, the patient may recuperate on his or her own according to the health advice in the risk assessment result. In this way, a visit pressure of a medical institution may be relieved, and the patient visiting efficiency may be improved.

In some embodiments, the at least one navigation tab 11 includes a smart question and answer tab. S2 includes S2G.

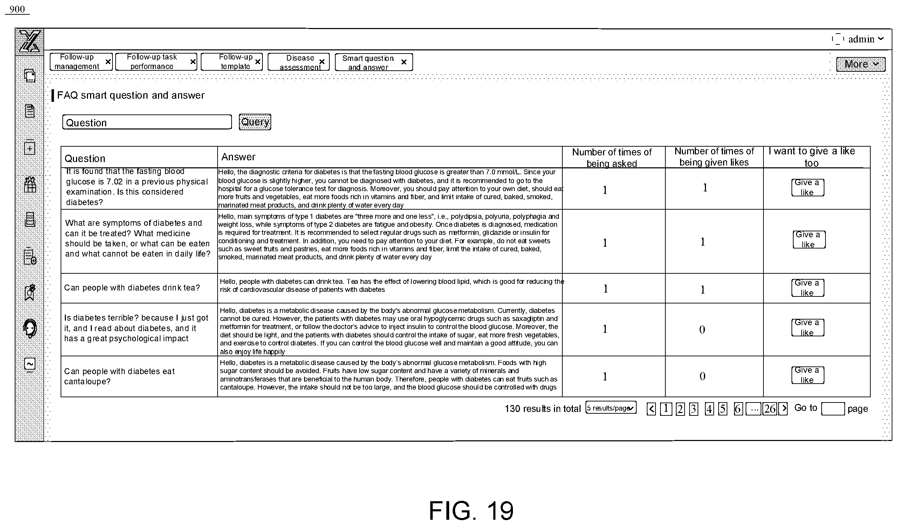

In S2G, as shown in FIG. 19, the PC displays a smart question and answer interface 900 in response to a first operation of the user on the smart question and answer tab, a question search box 901 configured to present a question to be searched being presented in the smart question and answer interface 900.

Based on this, the medical information processing method further includes S81.

In S81, in response to an operation of querying a question entered in the question search box 901, the PC acquires and displays a question whose similarity satisfies a similarity threshold condition and an answer matched with the question that are from a preset knowledge base in the server, which may facilitate a junior medical worker to consult related technical knowledge.

For example, there is one answer. For another example, there are a plurality of answers.

In some embodiments, the at least one navigation tab 11 includes a consultation tab. S2 includes S2H.

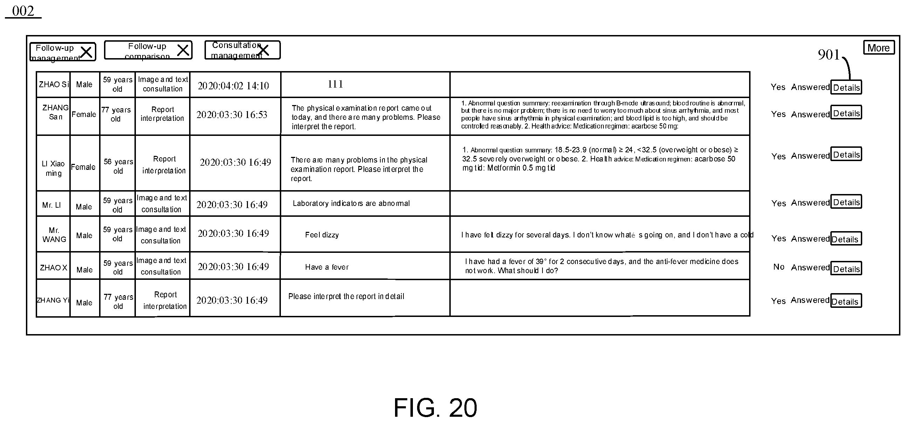

In S2H, as shown in FIG. 20, the PC displays a consultation management interface 002 in response to a first operation of the user on the consultation tab, a consultation list being presented in the consultation management interface 002, the consultation list including at least one line of consultation information from the server, and each line of consultation information including visit information of a patient, a question summary, a consultation type, whether there is an attachment, a reply state, and a fourth detail index item, in which the reply state includes being answered or being unanswered.

The medical information processing method further includes S90 and S94 to S95.

In S90, in a case where the reply state in a line of consultation information is being unanswered, the PC displays a consultation question corresponding to the line of consultation information and a reply text box matched with a consultation type in the line of consultation information in response to an operation of the user on the fourth detail index item 901.

In S94, the PC presents an answer matched with the consultation question in response to an operation of the user on the reply text box. Herein, the operation of the user on the reply text box may be understood as that the user enters the answer in the reply text box, or adopts a way of referencing a template in the knowledge base described below. It will be noted that, the "entry" may be understood as text entry, and may also be understood as voice entry or other entry ways, which is not limited in the embodiments of the present disclosure.

In S95, the PC changes the reply state in the line of consultation information from being unanswered to being answered in response to an operation of the user of saving the answer.

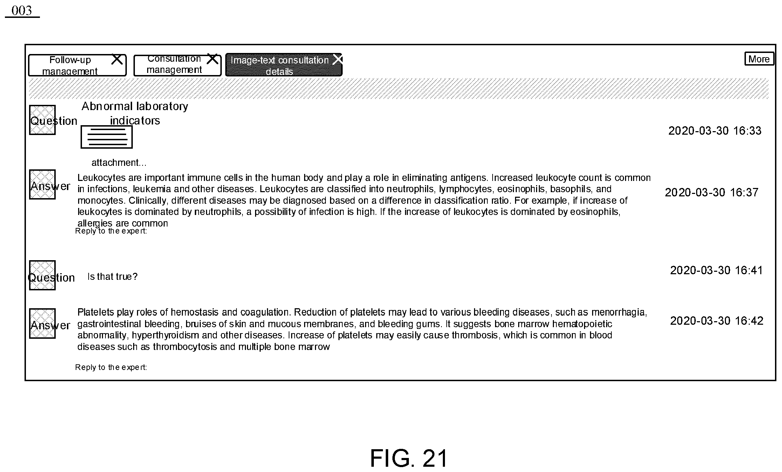

In some embodiments, as shown in FIG. 21, a consultation type in a line of consultation information is an image-text consultation, and the PC displays an image-text consultation detail interface 003 in response to an operation of the user of clicking on a fourth detail index item 901 corresponding to the line of consultation information. A question and a reply text box are displayed in the image-text consultation detail interface 003. In a case where a reply state in the line of consultation information is being unanswered, the PC presents an answer matched with the consultation question in response to an operation of the user on the reply text box. Herein, the operation of the user on the reply text box may be understood as that the user enters the answer in the reply text box.

In some other embodiments, a consultation type in a line of consultation information is report interpretation, and a reply text box includes an abnormal question summary text box and a health advice text box.

Based on this, the medical information processing method further includes S91.

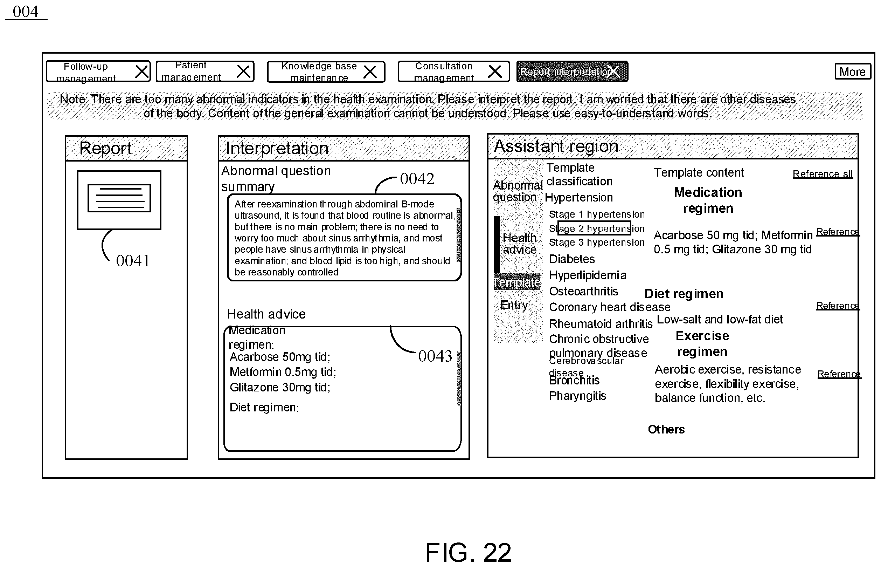

In S91, as shown in FIG. 22, the PC displays a report interpretation interface 004 in response to the operation of the user on the fourth detail index item 901, an examination report 0041, the abnormal qusetion summary text box 0042, and the health advice text box 0043 being presented in the report interpretation interface 004.

S94 includes S941 to S942.

In S941, the PC presents an abnormal question corresponding to the examination report and an interpretation text of the abnormal question in response to an operation of the user on the abnormal question summary text box 0042. Herein, the operation of the user on the abnormal question summary text box 0042 may be understood as that the user enters the abnormal question in the abnormal question summary text box 0042, or adopts a way of referencing an abnormal question template described below (i.e., S9411 to S9413 described below).

In S942, the PC presents a medical regimen matched with the abnormal question in response to an operation of the user on the health advice text box, the medical regimen including a medication regimen, a diet regimen, and an exercise regimen. Herein, the operation of the user on the health advice text box may be understood as that the user enters health advice in the health advice text box, or adopts a way of referencing a health advice template described below.

In some examples, as shown in FIG. 22, after S91, the medical information processing method further includes S92.

In S92, the PC displays an abnormal question template tab. For example, as shown in FIG. 22, the abnormal question template tab is presented in the report interpretation interface 004 in a form of words (e.g., abnormal question).

Based on this, S941 includes S9411 to S9413.

In S9411, the PC displays a third classification list in response to an operation of the user on the abnormal question template tab, the third classification list including a plurality of question types.

In S9412, in response to an operation of the user on a question type corresponding to the abnormal question in the third classification list, the PC displays the abnormal question corresponding to the question type and an interpretation text template corresponding to the abnormal question. For example, the abnormal question is sinus rhythm, and the third classification list includes a question type of electrocardiogram corresponding to sinus rhythm, and a corresponding interpretation text template is that there is no need to worry too much about sinus arrhythmia, and most people have sinus arrhythmia in physical examination. Then, in response to an operation of the user of clicking on electrocardiogram in the question types, the PC displays an interpretation text template with the words " there is no need to worry too much about sinus arrhythmia, and most people have sinus arrhythmia in physical examination".

In S9413, the PC presents content of the interpretation text template and the abnormal question in the abnormal question summary text box in response to an operation (e.g., reference) of the user on the interpretation text template.

Herein, the content of the interpretation text template is pre-stored. In this way, when summarizing the abnormal question in the report, the user may directly reference the pre-stored content of the interpretation text template, which improves the working efficiency of the doctor.

In some other examples, as shown in FIG. 22, after S91, the medical information processing method further includes S93.

In S93, the PC displays a health advice template tab. For example, as shown in FIG. 22, the health advice template tab is presented in the report interpretation interface 004 in a form of words (e.g., health advice).

Based on this, S942 includes S9421 to S9423.

In S9421, the PC displays an entry sub-tab in response to an operation of the user on the health advice template tab. For example, as shown in FIG. 22, the entry sub-tab is presented in the report interpretation interface 004 in a form of a word (e.g., entry). Herein, the entry sub-tab is the same as the entry sub-tab 202 in the health prescription creation interface 200.

In S9422, the PC displays a first classification list in response to an operation of the user on the entry sub-tab (e.g., clicking the text "entry"), the first classification list including a plurality of first-level items and at least one disease type included in each first-level item, and the plurality of first-level items including a medication regimen item, a diet regimen item, and an exercise regimen item. It will be noted that, S9422 is the same as S261, that is to say, the first-level items in the first classification list presented in the report interpretation interface 004 and the disease type included in each first-level item are the same as the first-level items in the first classification list presented in the health prescription creation interface 200 and the disease type included in each first-level item.

In S9423, in response to an operation of the user on a disease type corresponding to the abnormal question in a first-level item in the first classification list, the PC displays at least one entry text template corresponding to the disease type in the first-level item.

It will be noted that, not every abnormal question requires health advice from the medical worker. For example, if the abnormal question is sinus rhythm, there is no need to give health advice by the medical worker. If the abnormal question is hypertension, a disease type corresponding to the abnormal question is hypertension, and the PC presents at least one entry text template corresponding to hypertension in the report interpretation interface 004 in response to an operation of the user of clicking on hypertension in the first classification list. Herein, the entry text template corresponding to hypertension presented in the report interpretation interface 004 is the same as the entry text template corresponding to hypertension presented in the health prescription creation interface 200, both of which are templates pre-stored in the knowledge base.

In S9424, in response to an operation of the user on an entry text template, the PC presents content of the entry text template in the health advice text box 0043. Herein, the operation of the user on the entry text template may be understood as that the user clicks on a reference button corresponding to the entry text template (as shown in FIG. 22), or drags the entry text template into the health advice text box 0043.

In yet some other examples, as shown in FIG. 22, S942 includes S9425 to S9428.

In S9425, a health prescription template sub-tab is displayed in response to an operation of the user on the health advice template tab. For example, as shown in FIG. 22, the health prescription template sub-tab is presented in the report interpretation interface 004 in a form of a word (e.g., template). Herein, the health prescription template sub-tab is the same as the health prescription template sub-tab 206 in the health prescription creation interface 200.

In S9426, the PC displays a second classification list in response to an operation of the user on the health prescription template sub-tab, the second classification list including a plurality of disease types.

It will be noted that, S9426 is the same as S264, that is to say, the second classification list presented in the report interpretation interface 004 is the same as the second classification list 207 presented in the health prescription creation interface 200.

In S9427, in response to an operation of the user on a disease type corresponding to the abnormal question in the second classification list, the PC displays at least one medical regimen template corresponding to the disease type. Herein, the medical regimen template corresponding to the disease type (e.g., hypertension) presented in the report interpretation interface 004 is the same as the medical regimen template corresponding to hypertension presented in the health prescription creation interface 200, both of which are templates pre-stored in the knowledge base.

In S9528, in response to an operation of the user on a medical regimen template, the PC presents at least part of content of the medical regimen template in the health advice text box. Herein, the operation of the user on the medical regimen template may be understood as that the user clicks on all reference buttons corresponding to the medical regimen template (as shown in FIG. 22), or drags the medical regimen template into the health advice text box 0043, or clicks on a reference button corresponding to a medication regimen, an exercise regimen, a diet regimen or any other regimen in the medical regimen template.

For example, the interpretation text template, the entry text template and the medical regimen template presented in the report interpretation interface 004 are all in a non-edited state. That is to say, in the report interpretation interface 004, the user may only reference the interpretation text template, the entry text template, and the medical regimen template, and may not modify or delete the templates, or add a new template.

Of course, the above is only an example, that is to say, the user may also modify, delete or add the interpretation text template, the entry text template, and the medical regimen template presented in the report interpretation interface 004, which is not limited in the embodiments of the present disclosure.

In some embodiments, the abnormal question template tab, the health advice template tab, and the entry sub-tab and the health prescription template sub-tab in the health advice template tab may further be presented in any other interface in different forms, for example, they are presented in the navigation bar 1 in forms of icons.

In some embodiments, the medical information processing method further includes S3 to S6.

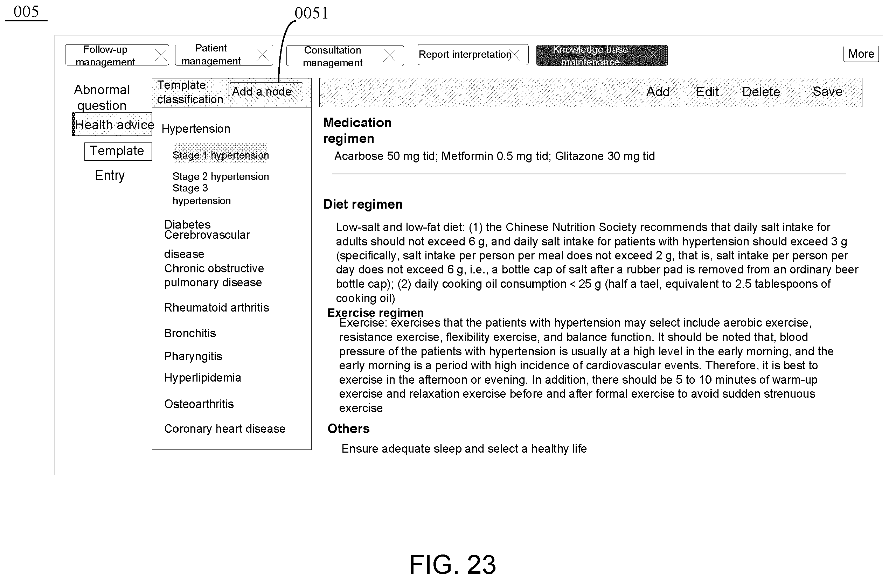

In S3, as shown in FIG. 23, the PC displays a knowledge base management interface 005 in response to an operation of the user on a knowledge base management tab, the abnormal question template tab and the health advice template tab being presented in the knowledge base management interface 005.

In S4, the PC displays a question node setup item and an interpretation text template setup item in response to an operation of the user on the abnormal question template tab, the question node setup item being configured to instruct to set question types, and the interpretation text template setup item being configured to instruct to add a new interpretation text template, instruct to edit the interpretation text template, and instruct to delete the interpretation text template.

Herein, the interpretation text template is the interpretation text template presented in the report interpretation interface 004 in S9412. That is to say, the interpretation text template presented in the report interpretation interface 004 in S9412 is from the interpretation text template stored by the user in the knowledge base management interface 005.

In S5, the PC displays an entry sub-tab in response to an operation of the user on the health advice template tab.

In S6, the PC displays a first node setup item and an entry text template setup item in response to an operation of the user on the entry sub-tab, the first node setup item being configured to instruct to set item types of health advice and a disease type matched with each item type, the item types of the health advice including a medication regimen item, a diet regimen item, an exercise regimen item, and other items, and the entry text template setup item being configured to instruct to add a new entry text template, instruct to edit the entry text template, and instruct to delete the entry text template.

Herein, the entry text template is the entry text template presented in the report interpretation interface 004 in S9422 and the entry text template presented in the health prescription creation interface 200 in S262. That is to say, the entry text template presented in the report interpretation interface 004 in S9422 and the entry text template presented in the health prescription creation interface 200 in S262 are both from the entry text template stored by the user in the knowledge base management interface 005.

In some examples, S6 includes S601' to S603.

A difference between S601' to S603' and S601 to S603 is only that in S601 to S603, the entry text template 205 is presented in the health prescription creation interface 200, while in S601' to S603', the entry text template is presented in the knowledge base management interface 005.

In this way, the user may pre-store the entry text template to directly reference it when creating a health prescription or creating health advice during interpretation of a report. It may also be possible to add a new entry text template, or to modify or delete the pre-stored entry text template during reference of the entry text template, which improves the working efficiency of the user.

In some other examples, after S4, the method further includes S7 and S8.

In S7, the PC displays a health prescription template sub-tab in response to an operation of the user on the health advice template tab.

In S8, as shown in FIG. 23, the PC displays a second node setup item and a health prescription template setup item in response to an operation of the user on the health prescription template sub-tab, the second node setup item being configured to instruct to set disease types, and the health prescription template setup item being configured to instruct to add a new medical regimen template, instruct to edit the medical regimen template, and instruct to delete the medical regimen template. For example, the health prescription template setup item includes an addition item, an editing item, a deletion item, and a save item.

Herein, the medical regimen template is the medical regimen template presented in the report interpretation interface 004 in S9427 and the medical regimen template presented in the health prescription creation interface 200 in S265. That is to say, the medical regimen template presented in the report interpretation interface 004 in S9427 and the medical regimen template presented in the health prescription creation interface 200 in S265 are both from the medical regimen template stored by the user in the knowledge base management interface 005.

In some examples, S8 includes S801' and S804 to S805.

A difference between S801' and S801 is only that the medical regimen template 208 in S801 is presented in the health prescription creation interface 200, while the entry text template in S801' is presented in the knowledge base management interface 005.

In S804, the PC presents a medical regimen template input box in the knowledge base management interface 005 in response to an operation of the user of clicking on the addition item; and the PC adds content input in the medical regimen template input box as a new medical regimen template in response to an operation of saving the content added in the entry text input box.

In S805, the PC deletes a medical regimen template in response to an operation of the user of clicking on the deletion item.

In some embodiments, the at least one navigation tab 11 includes a statistical tab. S2 includes S2J.

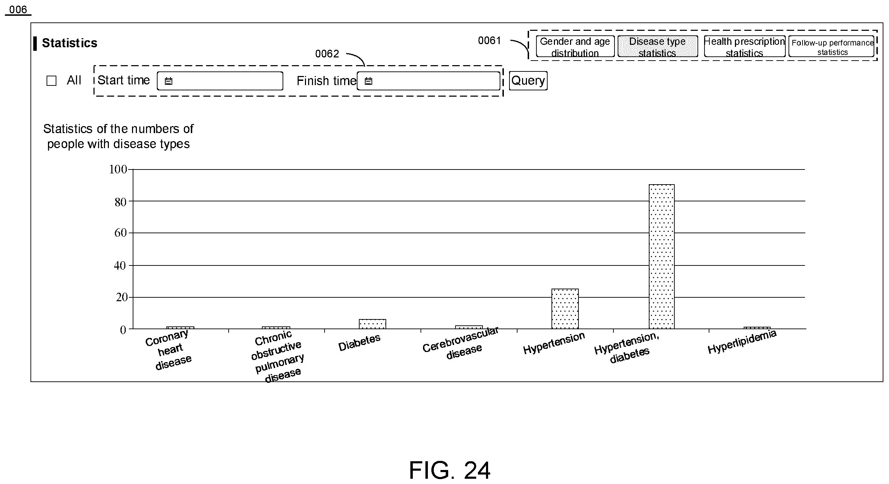

In S2J, as shown in FIGS. 24 to 28, the PC displays a statistical management interface 006 in response to a first operation of the user on the statistical tab (e.g., clicking on an icon representing the statistical tab in the navigation bar 1), the statistical management interface including a statistical category option 0061; and in response to an operation of the user on a statistical category in the statistical category option 0061, the PC acquires and displays statistical information corresponding to the statistical category, the statistical information being information obtained by performing classified statistics on management information of a plurality of patients and diseases of the plurality of patients. Herein, the acquisition may be understood as that the PC or the server performs an integrated analysis on data of the management information according to numerical values and field types in the data of the management information of the plurality of patients.

In some examples, as shown in FIG. 24, the statistical category option 0061 includes a disease type statistical option. The PC acquires and displays statistical information of the numbers of patients corresponding to different diseases in response to, for example, an operation of the user of clicking on the disease type statistical option. For example, a time period option 0062 is further presented in the statistical management interface 006, and the user may filter the numbers of patients corresponding to different diseases among patients whose medical record card information is created within a period of time through the time period option 0062.

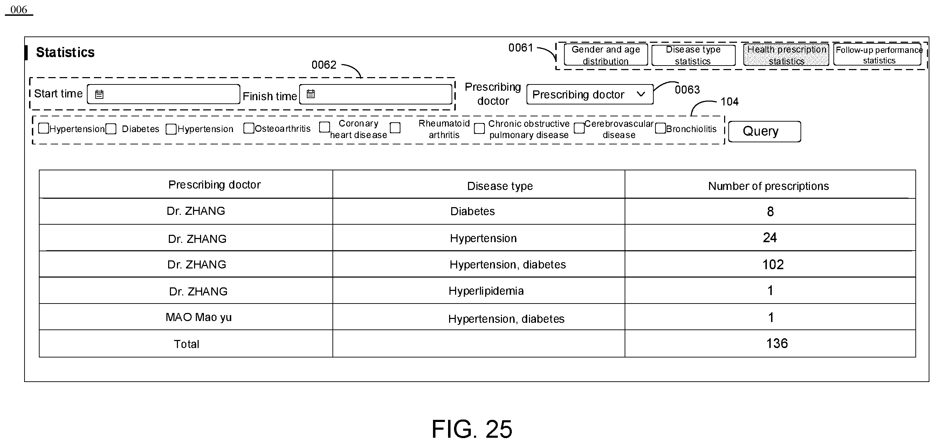

In some other examples, as shown in FIG. 25, the statistical category option 0061 includes a health prescription statistical option, and the PC acquires and displays the number of created health prescriptions in response to, for example, an operation of the user of clicking on the health prescription statistical option. For example, the time period option 0062 is further presented in the statistical management interface 006, and the user may filter the number of health prescriptions created within a period of time through the time period option 0062. For another example, a prescribing doctor selection item 0063 is further presented in the statistical management interface 006, and the user may filter the number of health prescriptions created by a certain doctor or a plurality of doctors through the prescribing doctor selection item 0063. For yet another example, a disease type option 104 is further presented in the statistical management interface 006, and the user may filter the number of health prescriptions matched with a certain disease type or a plurality of disease types through the disease type option 104.

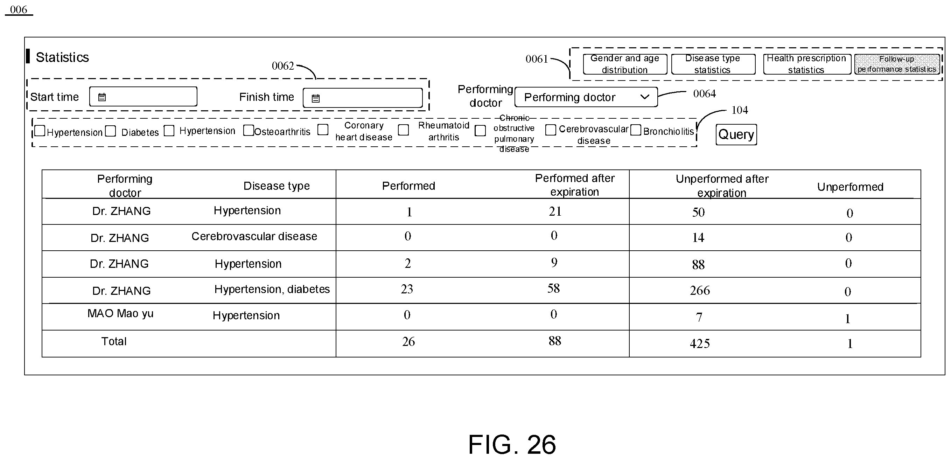

In yet some other examples, as shown in FIG. 26, the statistical category option 0061 includes a follow-up performance statistical option, and the PC acquires and displays the numbers of follow-up tasks in different states (e.g., performed, performed after expiration, unperformed after expiration, and unperformed) in response to, for example, an operation of the user of clicking on the follow-up performance statistical option. For example, the time period option 0062 is further presented in the statistical management interface 006, and the user may filter the numbers of follow-up tasks in different states within a period of time through the time period option 0062. For another example, a performing doctor option 0064 is further presented in the statistical management interface 006, and the user may filter the numbers of follow-up tasks in different states, performing doctor(s) of which are a certain doctor or a plurality of doctors, through the performing doctor option 0064. For yet another example, the disease type option 104 is further presented in the statistical management interface 006, and the user may filter the numbers of follow-up tasks in different states matched with a certain disease type or a plurality of disease types through the disease type option 104. Herein, the performing doctor is the follow-up person.

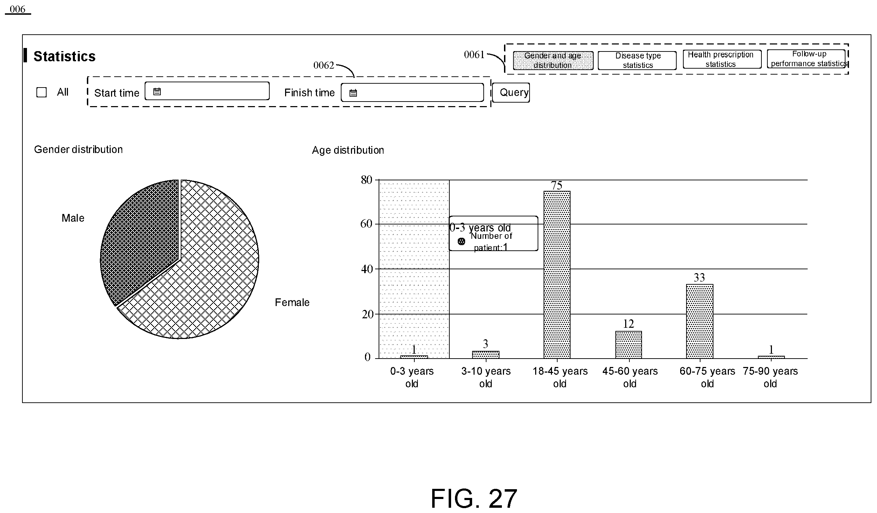

In yet some other examples, as shown in FIG. 27, the statistical category option 0061 includes a gender and age distribution option, and the PC acquires and displays a gender distribution result and an age distribution result of a plurality of patients (e.g., a gender ratio, an age ratio, the numbers of patients with different genders, or the numbers of patients with different ages) in response to, for example, an operation of the user of clicking on the gender and age distribution option. For example, the time period option 0062 is further presented in the statistical management interface 006, and the user may filter a gender distribution result and an age distribution result of a plurality of patients for whom medical record cards are created within a period of time through the time period option 0062.

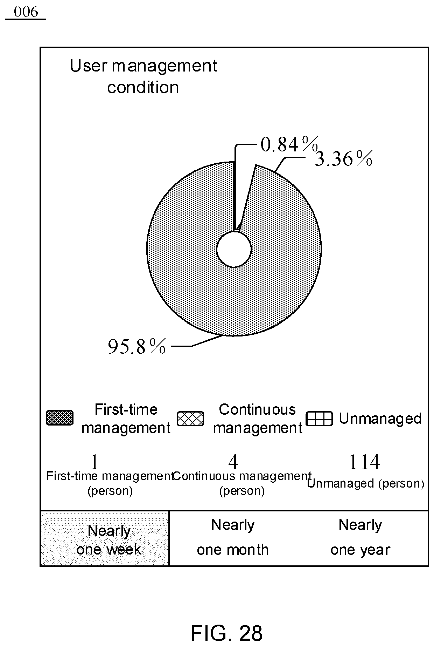

In yet some other examples, as shown in FIG. 28, the statistical category option 0061 includes a user management option, and the PC acquires and displays a statistical result of different management states of a plurality of patients in response to, for example, an operation of the user of clicking on the user management option. The management states are, for example, first-time management, continuous management, and being unmanaged. Herein, the first-time management may be understood as that from a date when medical record card information of a patient is created to a current statistical day, the medical worker has created a health prescription or a follow-up task for the patient once. The continuous management may be understood as that from the date when the medical record card information of the patient is created to the current statistical day, the medical worker has created, for example, three consecutive health prescriptions or three consecutive follow-up tasks for the patient, or has created health prescriptions or follow-up tasks for the patient for, for example, three consecutive times. Being unmanaged may be understood as that from the date when the medical record card information of the patient is created to the current statistical day, the medical worker has created no health prescription or follow-up task for the patient. The above management states are only described by taking an example in which a management item is creating a health prescription or a follow-up task. Of course, the management item may also be the medical worker interpreting a report for a patient or answering a question that the patient consults, etc. The embodiments of the present disclosure do not specifically limit the management item preset in the statistical process. The management item may be understood as an item related to storage of information except the medical record card information from the date when the medical record card information of the patient is created to the current statistical day.

Of course, in the above embodiments, other filtering options may also be presented in the statistical management interface 006 to filter statistical information, which is not limited in the embodiments of the present disclosure.

In some embodiments, the PC displays a plurality of navigation tabs, which include the patient management tab, health prescription management tab, follow-up management tab, disease assessment tab, smart question and answer tab, consultation tab, statistical tab, knowledge base management tab, new health prescription creation tab, new follow-up creation tab, card registration and creation tab, health advice template tab, abnormal question template tab, entry sub-tab, health prescription template sub-tab and historical prescription sub-tab. Of course, the plurality of navigation tabs may further include other tabs, which is not limited in the embodiments of the present disclosure.

It will be noted that, the above statistical categories may also be other categories, and may be set according to needs of the user, which is not limited in the embodiments of the present disclosure.

Some embodiments of the present disclosure provide a medical information acquisition method, and an implementation subject of the method is a second electronic device. The second electronic device is, for example, a mobile phone, a tablet computer, a handheld computer, a notebook computer, a personal computer (PC), or any other product or component with a display and a processor, and the embodiments of the present disclosure do not specifically limit a type of the second electronic device.

The medical information acquisition method will be described below by taking an example in which the implementation subject is the mobile phone.

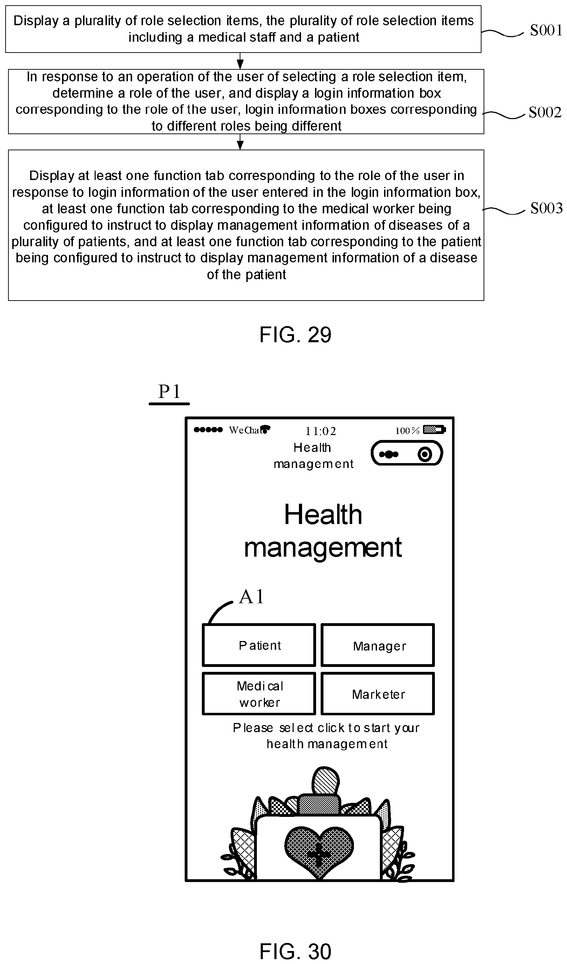

As shown in FIG. 29, the medical information acquisition method further includes S001 to S003.

In S001, as shown in FIG. 30, the mobile phone displays a homepage interface P1, a plurality of role selection items A1 being presented in the homepage interface P1, and the plurality of role selection items A1 including a medical worker and a patient. For example, as shown in FIG. 29, the role selection items A1 are displayed in forms of words. For another example, the role selection items A1 are displayed in forms of images or icons. Of course, the role selection items A1 may also be displayed in other forms, which is not limited in the embodiments of the present disclosure.

In S002, in response to an operation of the user of selecting a role selection item, the mobile phone determines a role of the user, and displays a login information box corresponding to the role of the user, login information boxes corresponding to different roles being different.

For example, if the user selects the medical worker, then in response to an operation of the user of selecting a role selection item representing the medical worker, the mobile phone determines that the role of the user is the medical worker, and displays a first login information box corresponding to the medical worker.

For another example, if the user selects the patent, then in response to an operation of the user of selecting a role selection item representing the patient, the mobile phone determines that the role of the user is the patent, and displays a second login information box corresponding to the patent.

Herein, the operation of the user of selecting the role selection item may be understood as touching, clicking, voice wakeup, etc. The embodiments of the present disclosure do not limit this, as long as in response to this operation, the mobile phone can determine the role of the user, and display the login information box corresponding to the role of the user.

Types of login information presented in the first login information box and the second login information box are different. For example, an ID card number and a password are presented in the first login information box, and a mobile phone number and a verification code are presented in the second login information box. Of course, other identity information may also be presented in the first login information box, as long as the identity information presented in the first login information box is matched with filled-in registration information when the patient makes a registration on the PC side. The embodiments of the present disclosure do not limit this.

In S003, the mobile phone displays at least one function tab corresponding to the role of the user in response to login information of the user entered in the login information box.

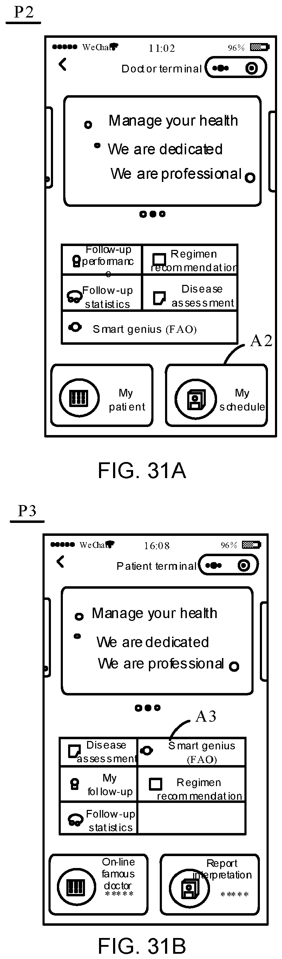

For example, as shown in FIG. 31A, in response to the login information box being the first login information box, and the ID card number and the password entered in the first login information box, the mobile phone displays a function navigation interface P2 corresponding to the medical worker, the function navigation interface P2 corresponding to the medical worker including a plurality of first function tabs A2.

For another example, as shown in FIG. 31B, in response to the login information box being the second login information box, and the mobile phone number and the verification code entered in the second login information box, the mobile phone displays a function navigation interface P3 corresponding to the patient, the function navigation interface P3 corresponding to the patient including a plurality of second function tabs A3.

It will be noted that, the first function tabs A2 and the second function tabs A3 may be displayed in forms of icons shown in FIG. 31A or 31B, or may be displayed in other forms. The embodiments of the present disclosure do not limit display manners of the first function tabs A2 and the second function tabs A3.

In a case where there are a plurality of first function tabs A2, the plurality of first function tabs A2 may be displayed simultaneously or may not be displayed simultaneously. Similarly, in a case where there are a plurality of second function tabs A3, the plurality of second function tabs A3 may be displayed simultaneously or may not be displayed simultaneously. The embodiments of the present disclosure do not limit a display order of the first function tabs A2 and the second function tabs A3.

In addition, in a case where the plurality of first function tabs A2 are not displayed simultaneously, the plurality of first function tabs A2 may be displayed in a same interface or in different interfaces. Similarly, in a case where the plurality of second function tabs A3 are not displayed simultaneously, the plurality of second function tabs A3 may be displayed in a same interface or in different interfaces. The embodiments of the present disclosure do not specifically limit display positions of the first function tabs A2 and the second function tabs A3.

The first function tabs A2 corresponding to the medical worker are configured to instruct to display management information of diseases of a plurality of patients. Herein, the management information of the diseases of the plurality of patients is the same as the management information of the diseases of the plurality of patients in the above medical information processing method, which will not be repeated herein.

The second function tabs A3 corresponding to the patient are configured to instruct to display management information of a disease of the patient. Herein, the management information of the disease of the patient may be understood as management information of a disease of one of the plurality of patients.

In some examples, the first login information box includes an ID card number input box and a password input box, and S003 further includes S0034 to S0035.

In S0034, the mobile phone sends the ID card number in the ID card number input box and the password in the password input box entered by the user to the server.

In S0035, the mobile phone displays the plurality of first function tabs A2 corresponding to the medical worker in response to received confirmation information from the server that the ID card number and the password are matched with the registration information.

In some other examples, the second login information box includes a mobile phone number input box and a verification code input box, and S003 further includes S0031 to S0033.

In S0031, the mobile phone sends the mobile phone number presented in the mobile phone number input box to the server.

In S0032, the mobile phone acquires the verification code from the server.

In S0033, the mobile phone determines whether the verification code entered by the user in the second login information box is matched with the verification code from the server, and displays the plurality of second function tabs A3 corresponding to the patient in response to determining that the verification code entered in the second login information box is matched with the verification code from the server.

Of course, S003 may also be implemented in other ways, which is not limited in the embodiments of the present disclosure.

In some embodiments, the role of the user is the medical worker, and the first function tabs A2 include a work schedule tab. Based on this, the medical information acquisition method further includes S004 to S005.

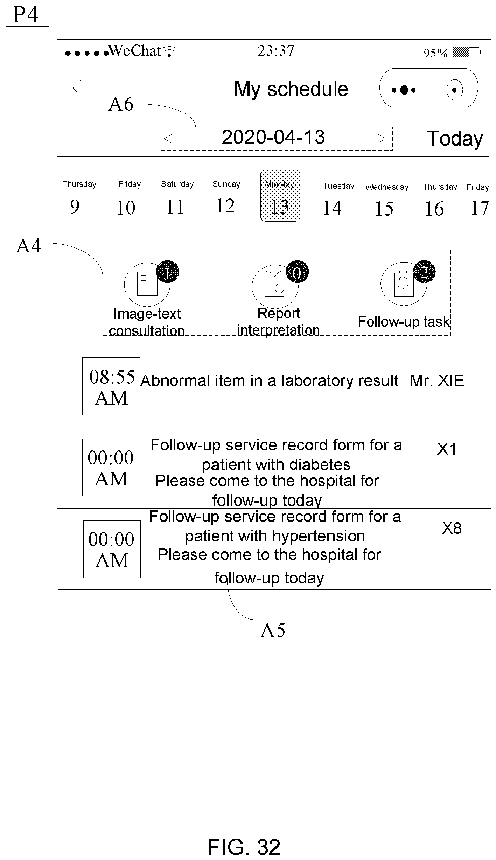

In S004, as shown in FIG. 32, the mobile phone displays a schedule interface P4 in response to an operation of the user on the work schedule tab, a plurality of work item index items A4 of the medical worker being presented in the schedule interface, and the work item index items A4 including an image-text consultation index item, a report interpretation index item, and a follow-up plan index item. Herein, the operation of the user on the work schedule tab may be understood as that the medical worker clicks on the work schedule tab or wakes up the work schedule tab with voice, etc. The embodiments of the present disclosure do not specifically limit this operation, as long as the mobile phone may display the work item index items A4 in response to this operation.

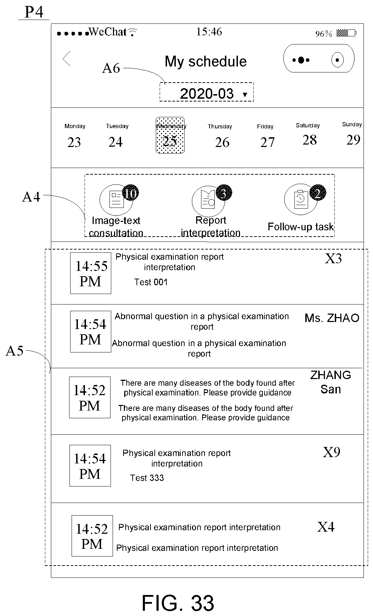

In S005, as shown in FIGS. 32 and 33, in response to an operation of the medical worker on a work item index item A4, the mobile phone acquires and displays work content corresponding to the work item index item of the medical worker from the server. Herein, the operation of the user on a work item index item A4 may be understood as that the medical worker clicks on the work item index item A4 or wakes up the work item index item A4 with voice, etc. The embodiments of the present disclosure do not specifically limit this operation.

For example, in response to an operation of the medical worker on the image-text consultation index item (e.g., clicking on the image-text consultation index item), the mobile phone acquires a work content list A5 corresponding to the image-text consultation index item of the medical worker from the server, and displays the work content list A5 in the schedule interface P4. As shown in FIG. 32, a summary of work content and a name of a patient corresponding to the work content are displayed in each line of information in the work content list A5. For example, a summary of work content of a first line of information in the work content list A5 in FIG. 33 is an abnormal item in a laboratory result, and a patient is Mr. Xie. That is to say, Mr. Xie consults the medical worker about the abnormal item in the laboratory result. Then work content of the medical worker is to answer questions about the abnormal item in the laboratory result to Mr. Xie. Based on this, the medical worker may also complete the work content corresponding to the image-text consultation index item through the image-text consultation index item, that is, the medical worker answers consultation questions raised by the patient.

For another example, in response to an operation of the medical worker on the follow-up plan index item (e.g., clicking on the follow-up plan index item), the mobile phone acquires a work content list A5 corresponding to the follow-up plan index item of the medical worker from the server, and displays the work content list A5 in the schedule interface P4. As shown in FIG. 32, for example, a summary of work content of a second line of information in the work content list A5 is a follow-up service record form for a patient with diabetes, and the patient is X1. That is to say, work content of the medical worker is to perform a follow-up task with follow-up content as the follow-up service record form for the patient with diabetes for the patient X1. Herein, the follow-up task is one of the follow-up tasks, the follow-up person of which is the medical worker, in the follow-up task list 601 displayed on the PC.

For example, information corresponding to only one work item index item A4 is displayed in the work content list A5.

For another example, as shown in FIGS. 32 and 33, information corresponding to the plurality of work item index items A4 is displayed in the work content list A5.

In some examples, the medical information acquisition method further includes S006.

In S006, the mobile phone acquires an amount of work content corresponding to a work item index item A4 of the medical worker on a target working day from the server, and displays the amount of the work content corresponding to the work item index item A4 of the medical worker on the work item index item. For example, as shown in FIG. 32, an amount of work content corresponding to the image-text consultation index item of the medical worker on a current working day from the server that is acquired by the mobile phone is 1, and then the amount 1 is displayed on the image-text consultation index item, so that the medical worker knows his or her own workload related to image-text consultation on the current working day.

For example, S006 and S004 are performed synchronously. Of course, S006 and S004 may also be performed asynchronously, which is not limited in the embodiments of the present disclosure.

In some other examples, the medical information acquisition method further includes S007 to S008.

In S007, as shown in FIGS. 32 and 33, the mobile phone further displays a working day option A6 in the schedule interface P4 to facilitate the medical worker to view work content on different working days.

In S008, the mobile phone determines the target working day in response to an operation of the medical worker on the working day option A6. For example, the target working day shown in FIG. 31 is the current working day, and the target working day shown in FIG. 33 is March 25, 2020.

It will be noted that, in a case where the amount of the work content is displayed, the mobile phone displays the amount of the work content of the medical worker on the current working day by default. In this way, the medical worker may know his or her work content on the current working day.

In some embodiments, the role of the user is the medical worker, and the first function tabs A2 include a statistical query tab. The medical information acquisition method further includes S009.

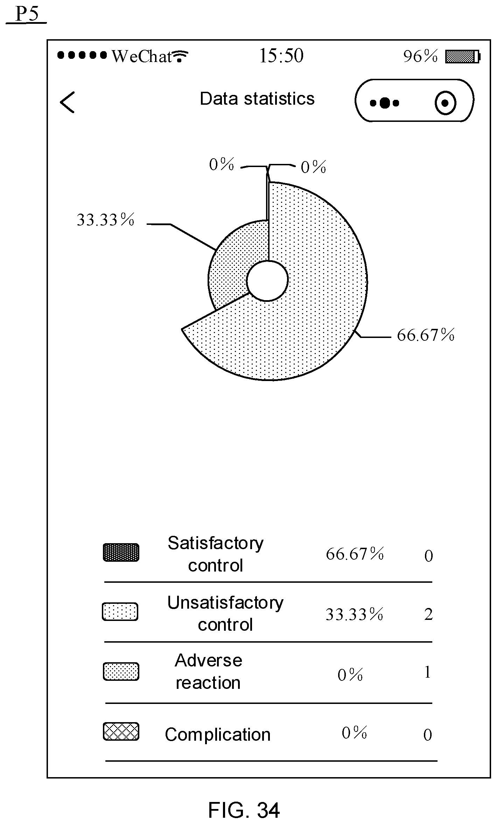

In S009, in response to an operation of the user on the statistical query tab, the mobile phone displays a statistical interface P5, and acquires and displays statistical information of follow-up condition of the plurality of patients from the server. Herein, as shown in FIG. 34, the statistical information of the follow-up condition of the plurality of patients is statistical information of health condition of the plurality of patients obtained after at least one follow-up is performed on the plurality of patients, i.e., statistical information of satisfactory control, unsatisfactory control, adverse reaction, or complication in the "category of this follow-up" in the follow-up service record form for the patient with hypertension shown in FIG. 12 of the plurality of patients. For example, as shown in FIG. 34, a proportion of the satisfactory control is 66.67%, and a proportion of the unsatisfactory control is 33.33%. The medical worker may know treatment condition of a disease of a patient through the statistical information, and may appropriately adjust a treatment method.

It will be noted that, in a case where the role of the user is the medical worker, the first function tabs A2 may further include other tabs, such as a tab of my patients, a regimen recommendation tab, and a follow-up performance tab in FIG. 30. The medical worker may view information of patients related to himself or herself through the tab of my patients, and may view medical regimens or health advice corresponding to a plurality of patients stored in the server through the regimen recommendation tab. The medical worker may view a plurality of follow-up tasks stored in the server and follow-up content corresponding to each follow-up task through the follow-up performance tab, i.e., the above follow-up record form. Of course, in a case where the follow-up person of the follow-up task is the medical worker, the follow-up person may also perform the follow-up task through the follow-up performance tab or the follow-up plan index item, i.e., filling in the follow-up record form. The embodiments of the present disclosure do not limit this.

In some embodiments, the role of the user is the patient, and the second function tabs A3 include a disease assessment tab. Based on this, the medical information acquisition method further includes S010 to S011.

In S010, the mobile phone displays a second disease assessment interface P6 in response to an operation of the patient on the disease assessment tab, at least one disease assessment question being presented in the second disease assessment interface P6.

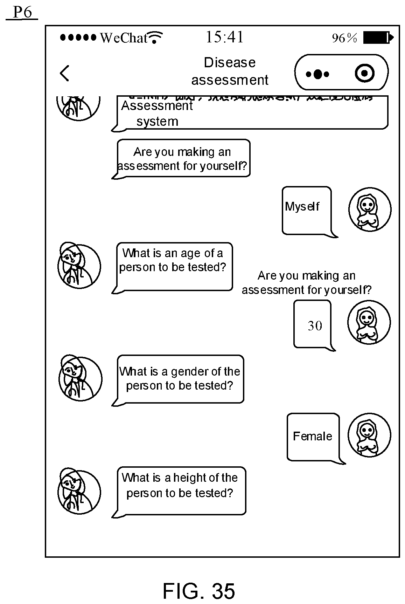

In S011, as shown in FIG. 35, in response to an answer of the user to each disease assessment question, the mobile phone acquires a disease assessment result of the patient from the server and displays the disease assessment result in a disease assessment result interface P7.

Herein, the disease assessment question is a question matched with each disease pre-stored in the server. According to an answer of the patient, the server may automatically match a disease assessment question and continue to ask the patient until an assessment result for the disease of the patient may be generated.

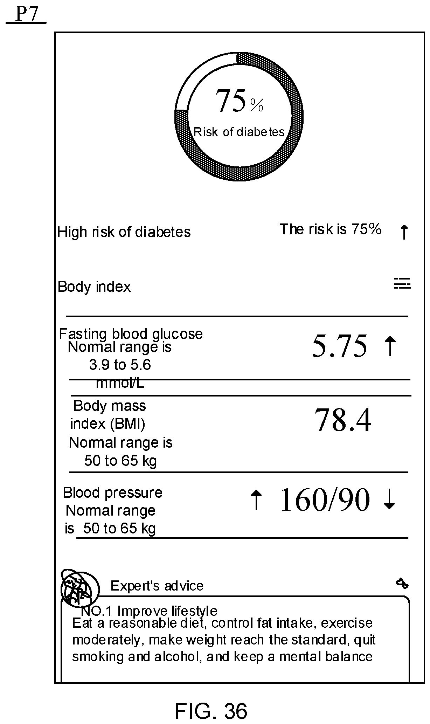

In some examples, as shown in FIG. 35, in response to the operation of the patient on the disease assessment tab, the mobile phone first displays a first question that "are you making an assessment for yourself?" in the second disease assessment interface P6. The mobile phone automatically displays a second question that "what is a gender of a person to be tested?" in response to an answer of the patient to the first question. The mobile phone automatically displays a third question in response to an answer of the patient to the second question, and so on. Finally, based on the questions and answers, an assessment result is automatically generated and displayed on the mobile phone (as shown in FIG. 36), so that the patient has an understanding of his or her own disease.

For example, the disease assessment question may be the same as the options in the disease assessment information text box presented in the first disease assessment interface 800.

In some embodiments, the role of the user is the patient, and the second function tabs A3 include a smart question and answer tab. Based on this, the medical information acquisition method further includes S012 to S013.

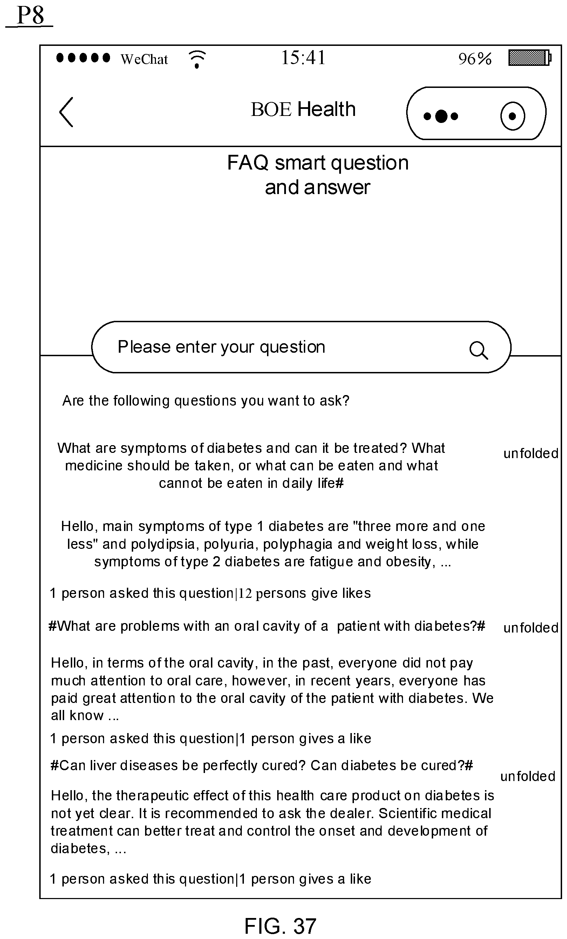

In S012, as shown in FIG. 37, the mobile phone displays an FAQ smart question and answer interface P8 in response to an operation of the patient on the smart question and answer tab, a question search box being presented in the FAQ smart question and answer interface P8.

In S013, in response to an operation of querying a question entered in the question search box by the patient, the mobile phone acquires and displays one or more answers associated with the question from the server. Herein, the answer(s) may be automatically matched by the server according to a pre-stored knowledge base.

In some embodiments, the role of the user is the patient, and the second function tabs A3 include a consultation tab. Based on this, the medical information acquisition method further includes S014 to S016.

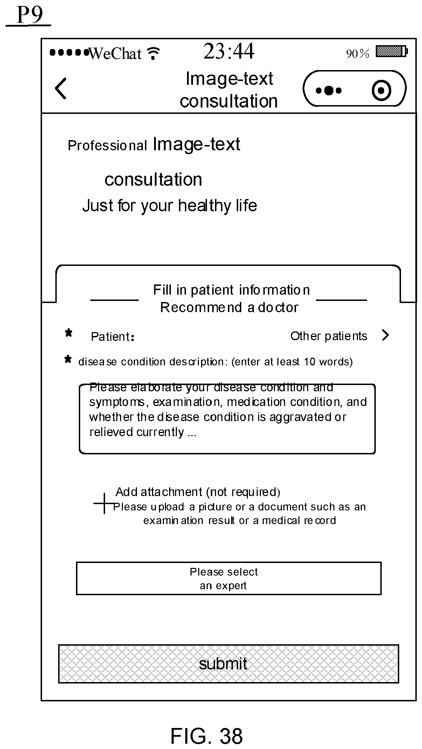

In S014, as shown in FIG. 38, the mobile phone displays a consultation interface P9 in response to an operation of the patient on the consultation tab, a basic information text box and a disease condition description text box being presented in the consultation interface P9, the basic information text box being configured to present basic information of the patient, e.g., a name, an age, a career and other information, and the disease condition description text box being configured to present information of disease condition of the patient, i.e., information of questions that the patient needs to consult.

In S015, in response to an operation of submitting information of the patient entered in the basic information text box and a consultation question entered in the disease condition description text box, the mobile phone transmits the information in the basic information text box and the consultation question in the disease condition description text box to the server.

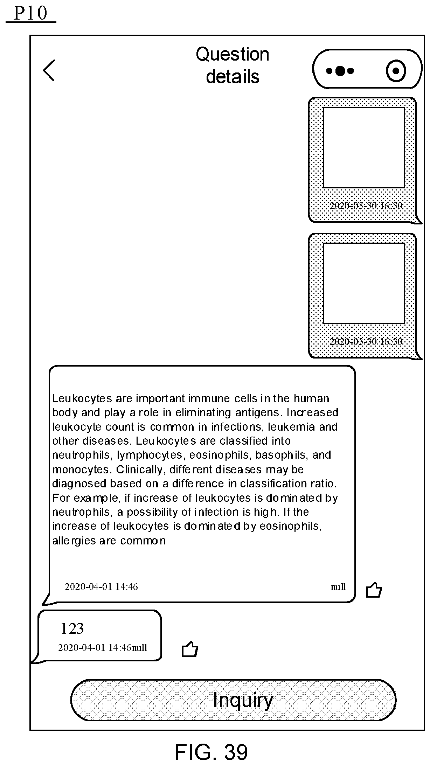

In S016, as shown in FIG. 39, the mobile phone acquires an answer matched with the consultation question from the server and displays the answer in a question detail interface P10.

For example, the mobile phone transmits the consultation question to the server, and the server transmits the consultation question to the PC, so that the medical worker may see the consultation question and answer the consultation question. The PC transmits the answer of the medical worker to the consultation question to the server, and the server transmits the answer to a mobile phone interface corresponding to the patient, so that the patient may view the answer.

As shown in FIG. 39, an inquiry item is presented in the question detail interface P10. The mobile phone displays a question input box in response to an operation of the patient on the inquiry item, and transmits content to the server in response to an operation of submitting the content entered in the question input box. In this way, the patient may continue to consult the medical worker about relevant questions according to the answer of the medical worker.

It will be noted that, the content presented in the basic information text box and the disease condition description text box in the consultation interface P9 is the consultation information in the consultation list in the consultation management interface 002 of the PC described above.

In some examples, the medical information acquisition method further includes S017 to S018.

In S017, as shown in FIG. 38, the mobile phone further displays an attachment addition item in the consultation interface P9.

In S018, in response to an operation of the patient on the attachment addition item, the mobile phone adds at least one attachment, such as a medical examination report or a check list.

Based on this, S015 includes S0151.

In S0151, in response to an operation of submitting the information of the patient entered in the basic information text box, the consultation question entered in the disease condition description text box, and the at least one attachment by the patient, the mobile phone transmits the information in the basic information text box, the consultation question in the disease condition description text box and the at least one attachment to the server. In this way, the medical worker may see, for example, the medical examination report or the check list, and may intuitively understand the health condition of the patient.

In some examples, the medical information acquisition method further includes S019 to S020.

In S019, as shown in FIG. 38, the mobile phone further displays a doctor selector in the consultation interface P9.

In S020, the mobile phone determines a doctor to be consulted in response to an operation of the patient on the doctor selector.

Based on this, S015 includes S0152.

In S0152, in response to the operation of submitting the information of the patient entered in the basic information text box and the consultation question entered in the disease condition description text box, the mobile phone transmits the information in the basic information text box, the consultation question in the disease condition description text box, and the doctor to be consulted corresponding to the consultation question to the server.

In this way, the patient may select an appropriate doctor according to his or her own disease condition, an experience of the patient of seeing a doctor may be improved, and the working efficiency of the doctor may also be improved. For example, if the consultation question of the patient is about digestion, a doctor who specializes in digestive diseases may be selected.

In some embodiments, the plurality of role selection items A1 further include a manager, and the at least one function tab includes a statistical query tab. Based on this, the method further includes S021.

In S021, the mobile phone displays a query item in response to an operation of the user on the statistical query tab, the query item being configured to instruct to query a classified statistical result, and the classified statistical result being obtained according to classified statistics of data related to the patient and the doctor stored in the server.

Herein, a login method of the manager is the same as the login method of the medical worker described above.

In some examples, the classified statistical result is the same as the statistical result displayed in the statistical management interface 006 on the PC side (e.g., FIGS. 24 to 28).

Some embodiments of the present disclosure provide a medical information interaction method, which includes S1001 to S1004.

In S1001, a medical information processing apparatus displays at least one navigation tab11, and in response to a first operation of a user, the medical information processing apparatus determines a target navigation tab 11, and displays management information of a disease of a patient matched with the target navigation tab 11.

In S1002, the medical information processing apparatus transmits the management information of the disease of the patient to a server.

In S1003, a medical information acquisition apparatus acquires the management information of the disease of the patient from the server.

In S1004, the medical information acquisition apparatus displays a plurality of role selection items, the plurality of role selection items including a medical worker and a patient; in response to an operation of the user of selecting a role selection item, the medical information acquisition apparatus determines a role of the user, and displays a login information box corresponding to the role of the user, login information boxes corresponding to different roles being different; and in response to login information of the user entered in the login information box, the medical information acquisition apparatus displays at least one function tab corresponding to the role of the user, first function tabs A2 corresponding to the medical worker being configured to instruct to display management information of diseases of a plurality of patients, and second function tabs A3 corresponding to the patient being configured to instruct to display the management information of the disease of the patient.

For example, in a case where the management information of the disease of the patient includes medical record card information of the patient, the medical information processing apparatus transmits medical record card information of each patient to the server, and the medical information acquisition apparatus acquires the medical record card information of each patient from the server; and in a case where the user of the medical information acquisition apparatus is the medical worker, the medical information acquisition apparatus acquires medical record card information of a plurality of patients related to the medical worker from the server, and displays the information in an interface of the medical information acquisition apparatus, the user of which is the medical worker.

For another example, in a case where the management information of the disease of the patient includes consultation information of the patient, and the consultation information of the patient includes basic information of the patient, a consultation question and an answer, the medical information processing apparatus transmits the answer to the server in response to an operation of submitting the answer corresponding to the consultation information by the medical worker; and in a case where the user of the medical information acquisition apparatus is the patient, the medical information acquisition apparatus acquires the answer corresponding to the consultation information of the patient from the server, and displays the answer in the interface of the medical information acquisition apparatus, the user of which is the patient.

Through the medical information interaction method in the embodiments of the present disclosure, the medical information is interacted in different terminal devices, so that the medical information is processed and displayed according to needs of a user of each terminal device, thereby improving the working efficiency of medical worker, and solving a problem of difficult medical treatment for the patient.

In some embodiments, the medical information interaction method further includes S1005 to S1008.

In S1005, the medical information acquisition apparatus displays a consultation tab corresponding to the patient in response to the login information of the patient entered in the login information box, the consultation tab being configured to instruct the patient to submit a consultation question.

In S1006, the medical information acquisition apparatus transmits the consultation question to the server.

In S1007, the medical information processing apparatus acquires and displays the consultation question from the server.

In S1008, the medical information processing apparatus transmits an answer to the server in response to an operation of submitting the answer matched with the consultation question.

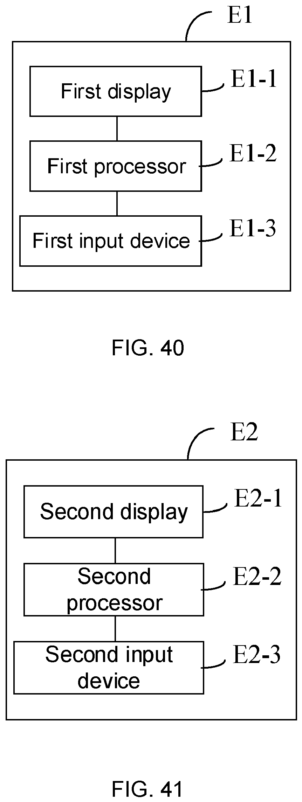

Some embodiments of the present disclosure provide a medical information processing apparatus. As shown in FIG. 40, the medical information processing apparatus includes a first display E1-1 and a first processor E1-2. The first processor E1-2 is coupled to the first display E1-1. The first processor E1-2 is configured to: control the first display E1-1 to display at least one navigation tab 11; and in response to a first operation of a user, to determine a target navigation tab 11, and to control the first display E1-1 to display management information of a disease of a patient matched with the target navigation tab 11. The target navigation tab 11 is one of the at least one navigation tab 11.

In some examples, as shown in FIG. 40, the medical information processing apparatus further includes a first input device E1-3. The first input device E1-3 is coupled to the first processor E1-2, and the first input device E1-3 is configured to enter the first operation of the user.

Some embodiments of the present disclosure provide a medical information acquisition apparatus. As shown in FIG. 41, the medical information acquisition apparatus E2 includes a second display E2-1 and a second processor E2-2. The second processor E2-2 is configured to: control the second display E2-1 to display a plurality of role selection items, the plurality of role selection items including a medical worker and a patient; in response to an operation of selecting a role selection item by a user, to determine a role of the user, and to control the second display E2-1 to display a login information box corresponding to the role of the user, login information boxes corresponding to different roles being different; and in response to login information of the user entered in the login information box, to control the second display E2-1 to display at least one function tab corresponding to the role of the user, at least one function tab corresponding to the medical worker being configured to instruct to display management information of diseases of a plurality of patients, and at least one function tab corresponding to the patient being configured to instruct to display management information of a disease of the patient.

In some embodiments, as shown in FIG. 41, the medical information acquisition apparatus E2 further includes a second input device E2-3 coupled to the second processor E2-2. The second input device E2-3 is configured to enter the operation of the user of selecting a role selection item, and enters the login information of the user.

Some embodiments of the present disclosure provide a computer equipment, which includes a memory and a processor. The memory stores computer program instructions that, when executed on the processor, cause the processor to implement the medical information processing method, the medical information acquisition method or the medical information interaction method described above. The computer equipment may be the medical information processing apparatus or the medical information acquisition apparatus described above.

Some embodiments of the present disclosure provide a computer-readable storage medium (i.e., a non-transitory computer-readable storage medium). The computer-readable storage medium stores computer program instructions that, when executed on the processor, cause the processor to perform one or more steps in the medical information processing method described in any one of the above embodiments, to perform one or more steps in the medical information acquisition method described in any one of the above embodiments, or to perform the medical information interaction method described in any one of the above embodiments.

For example, the computer-readable storage medium may include, but is not limited to, a magnetic storage device (e.g., a hard disk, a floppy disk or a magnetic tape), an optical disk (e.g., a compact disk (CD)), a digital versatile disk (DVD)), a smart card or a flash memory device (e.g., an erasable programmable read-only memory (EPROM), a card, a stick or a key driver). Various computer-readable storage media described in the present disclosure may represent one or more devices and/or other machine-readable storage media for storing information. The term "machine-readable storage media" may include, but is not limited to, wireless channels and other various media capable of storing, containing and/or carrying instructions and/or data.

The foregoing descriptions are merely some specific implementations of the present disclosure, but the protection scope of the present disclosure is not limited thereto. Any changes or replacements that a person skilled in the art could conceive of within the technical scope of the present disclosure should be included in the protection scope of the present disclosure. Therefore, the protection scope of the present disclosure shall be subject to the protection scope of the claims.

**Claims**

1. A medical information processing method, comprising:

    displaying at least one navigation tab; and
    in response to a first operation of a user, determining a target navigation tab, and displaying management information of a disease of a patient matched with the target navigation tab, the target navigation tab being one of the at least one navigation tab.

2. The medical information processing method according to claim 1, wherein the at least one navigation tab includes a patient management tab; and
    in response to the first operation of the user, determining the target navigation tab, and displaying the management information of the disease of the patient matched with the target navigation tab include: displaying at least a patient list in response to the first operation of the user on the patient management tab, the patient list including visit information of each patient of at least one patient and at least one medical operation index item corresponding to the patient, and the at least one medical operation index item including an index item for creating a treatment method of the disease of the patient.

3. The medical information processing method according to claim 2, wherein the at least one medical operation index item includes a health prescription creation index item; and
    the method further comprises:

    displaying at least a health prescription text box in response to an operation of the user on a health prescription creation index item corresponding to a target patient, the health prescription text box being a blank box; and
    presenting a medical regimen matched with a disease of the target patient in the health prescription text box in response to a second operation of the user, the medical regimen including at least one of a medication regimen, a diet regimen or an exercise regimen.

4. The medical information processing method according to claim 3, further comprising:

    displaying an entry sub-tab, wherein presenting the medical regimen matched with the disease of the target patient in the health prescription text box in response to the second operation of the user includes:

    presenting a first classification list in response to an operation of the user on the entry sub-tab, the first classification list including a plurality of first-level items and at least one disease type included in each first-level item, and the plurality of first-level items including a medication regimen item, a diet regimen item, and an exercise regimen item;

in response to an operation of the user on a disease type corresponding to the disease of the target patient in a first-level item in the first classification list, presenting at least one entry text template corresponding to the disease type in the first-level item; and

in response to an operation of the user on an entry text template, presenting content of the entry text template in the health prescription text box.

5. The medical information processing method according to claim 4, further comprising:
displaying at least one entry template setup item, the at least one entry template setup item including at least one of an addition item, an editing item, and a deletion item, the addition item being configured to instruct to add a new entry text template, the editing item being configured to instruct to edit at least one entry text template, and the deletion item being configured to instruct to delete at least one entry text template.

6. The medical information processing method according to any one of claims 3 to 5, further comprising:

displaying a health prescription template sub-tab; wherein
presenting the medical regimen matched with the disease of the target patient in the health prescription text box in response to the second operation of the user includes:

presenting a second classification list in response to an operation of the user on the health prescription template sub-tab, the second classification list including at least one disease type;
in response to an operation of the user on a disease type corresponding to the disease of the target patient in the second classification list, further presenting at least one medical regimen template corresponding to the disease type; and
in response to an operation of the user on a medical regimen template, presenting at least part of content of the medical regimen template in the health prescription text box.

7. The medical information processing method according to claim 6, further comprising:
displaying a health prescription template setup item, the health prescription template setup item being configured to instruct to edit at least one medical regimen template, and to add content of the edited medical regimen template as a new medical regimen template, or to update content of the medical regimen template before editing to the content of the edited medical regimen template.

8. The medical information processing method according to any one of claims 3 to 7, further comprising:

displaying a historical prescription sub-tab; wherein
presenting the medical regimen matched with the disease of the target patient in the health prescription text box in response to the second operation of the user includes:

presenting a historical prescription list associated with the disease of the target patient in response to an operation of the user on the historical prescription sub-tab;
presenting content of a historical prescription in the historical prescription list in response to an operation of the user on the historical prescription list; and
presenting the content of the historical prescription in the health prescription text box in response to an operation of the user on the historical prescription.

9. The medical information processing method according to any one of claims 3 to 8, further comprising:
further displaying an item of saving as a template, the item of saving as a template being configured to save the medical regimen presented in the health prescription text box as a new medical regimen template.

10. The medical information processing method according to any one of claims 2 to 9, wherein the at least one medical operation index item includes a follow-up creation index item; and
the method further comprises:
determining a follow-up task matched with a disease type in response to an operation of the user on a follow-up creation index item corresponding to a target patient and a third operation of the user, the follow-up task including follow-up content matched with a disease of the target patient and a follow-up time corresponding to the follow-up content.

11. The medical information processing method according to claim 10, wherein the follow-up content is a follow-up record form; and
the method further comprises:

in response to an operation of opening a scale making tool interface, displaying the scale making tool interface, the scale making tool interface including a field display region and an editing region, the field display region including a plurality of fields, and the editing region being used to make the follow-up record form, wherein a follow-up record form to be made includes a plurality of target elements, and each target element is generated by using a field; and

making the follow-up record form in the editing region in response to an operation of making the follow-up record form.

12. The medical information processing method according to claim 11, wherein making the follow-up record form in the editing region in response to the operation of making the follow-up record form includes:

displaying a selected field in the editing region in response to a field selection operation of the user in the scale making tool interface; and repeatedly responding to the field selection operation until fields corresponding to a plurality of target elements required by the follow-up record form are all displayed in the editing region to generate the follow-up record form.

13. The medical information processing method according to claim 12, wherein the plurality of fields include at least one basic field, and the basic field includes at least one attribute to be edited; and in a case where the field selected through the field selection operation is a basic field, after the selected field is displayed in the editing region, the method further comprises:

displaying an attribute menu of the basic field in the editing region in response to the field selection operation, the attribute menu including at least one attribute editing box; and displaying an edited attribute in each attribute editing box to generate a target element in response to a basic field attribute editing operation entered by the user in the at least one attribute editing box.

14. The medical information processing method according to claim 13, wherein the attribute menu further includes a button of saving as a frequently-used field; and the method further comprises:
displaying the generated target element in the field display region as a frequently-used field in response to an operation of the user of selecting the button of saving as a frequently-used field.

15. The medical information processing method according to claim 13 or 14, wherein the plurality of fields include at least two basic fields, the at least two basic fields include first basic fields and second basic fields, a first basic field is configured to generate a first target element, a second basic field is configured to generate a second target element, and there is a corresponding relationship between the first target element and the second target element; and at least one attribute editing box of the first basic field includes a data source attribute editing box;
displaying the edited attribute in each attribute editing box in response to the basic field attribute editing operation entered by the user in the at least one attribute editing box includes:

displaying a data source attribute representing the corresponding relationship in the data source attribute editing box of the first basic field in response to the basic field attribute editing operation; and
the method further comprises:

displaying a follow-up task performance interface in response to an operation of opening follow-up task performance, the follow-up task performance interface including the follow-up record form included in the follow-up task created for the target patient, the follow-up record form including the first target element and the second target element, and the first target element and the second target element each having an editing box; and
receiving a thirteenth operation entered in an editing box of the second target element, the thirteenth operation being used to:
in response to an operation of entering target content in the editing box of the second target element, display the target content in the editing box of the second target element, and automatically display content obtained according to the corresponding relationship represented by the data source attribute in an editing box of the first target element.

16. The medical information processing method according to claim 13 or 14, wherein the plurality of fields include third basic fields, a third basic field is configured to generate a third target element, and at least one attribute editing box of the third basic field includes a data source attribute editing box;
displaying the edited attribute in each attribute editing box in response to the basic field attribute editing operation entered by the user in the at least one attribute editing box includes:

displaying a data source attribute linked to a background database in the data source attribute editing box of the third basic field in response to the basic field attribute editing operation; and

the method further comprises:

    displaying a follow-up task performance interface in response to an operation of performing the follow-up task, the follow-up task performance interface including the follow-up record form included in the follow-up task created for the target patient, the follow-up record form including the third target element, and the third target element having an editing box; and
    automatically displaying content in the background database linked to the data source attribute of the third target element in the editing box of the third target element in response to an operation of the user of selecting the editing box of the third target element.

17. The medical information processing method according to claim 11 or 13, wherein the plurality of fields include at least one frequently-used field, and the frequently-used field includes at least one edited attribute; and
in a case where a field selected through a field selection operation is a frequently-used field, after the selected field is displayed in the editing region, the method further comprises:
using the frequently-used field displayed in the editing region as a target element in response to the field selection operation of the user.

18. The medical information processing method according to claim 17, wherein the plurality of fields include a plurality of frequently-used fields, and the plurality of frequently-used fields include at least one public frequently-used field and at least one private frequently-used field; and
the public frequently-used field may be used as a target element in at least two follow-up record forms to be made, and the private frequently-used field may be used as a target element in one follow-up record form to be made.

19. The medical information processing method according to any one of claims 11 to 18, wherein the scale making tool interface further includes a form region and a submission column; and the form region is configured to display a name of a generated follow-up record form, and the submission column includes a form generation button; and
the method further comprises:
displaying a name of a saved follow-up record form in the form region in response to an operation of the user of selecting the form generation button.

20. The medical information processing method according to claim 19, wherein the submission column further includes a preview button; and
before the name of the saved follow-up record form is displayed in the form region in response to the operation of the user of selecting the form generation button, the method further comprises:
displaying a generated form in the editing region in response to an operation of selecting the preview button.

21. The medical information processing method according to claim 20, wherein the operation of selecting the preview button is further configured to select a preview effect of a form, and the preview effect includes a paper effect and an effect displayed on a screen of a terminal; and
displaying the generated form in the editing region in response to the operation of the user of selecting the preview button includes:
in response to the operation of selecting the preview button, displaying the generated follow-up record form in the editing region in the paper effect, or displaying the generated follow-up record form in the editing region in the effect displayed on a screen of a mobile terminal.

22. The method according to any one of claims 19 to 21, wherein the form region includes a list sub-region and an operation sub-region; the list sub-region includes a name of at least one saved follow-up record form, and the saved form includes a plurality of set elements; and the operation sub-region includes at least one import reference option, and each saved form corresponds to an import reference option; and the method further comprises:

    in response to an operation of selecting an import reference option, displaying a saved follow-up record form corresponding to the selected import reference option in the editing region, and using the follow-up record form as a basic form;
    in response to a modification operation of the user, setting modified at least one set element to generate a target element, the modification operation being configured to modify at least one set element of a plurality of set elements in the basic form, and the at least one set element being not the same as the target element in the follow-up record form to be made; and
    repeatedly responding to the modification operation until the plurality of set elements in the basic form are the same as the plurality of target elements in the follow-up record form to be made to generate a new follow-up record form.

23. The medical information processing method according to claim 10, further comprising:

    displaying at least one follow-up task template;

wherein

determining the follow-up task matched with the disease type in response to the third operation of the user includes:

in response to an operation of the user on a follow-up task template, determining the follow-up task template as the follow-up task matched with the disease type.

24. The medical information processing method according to claim 23, further comprising:

creating a follow-up template, the follow-up template including the at least one follow-up task template.

25. The medical information processing method according to claim 24, wherein creating the follow-up template includes:

displaying a follow-up template interface, the follow-up template interface including a template selection box, and the template selection box including a plurality of disease options;
displaying a disease option menu in response to an operation of creating the follow-up template; and
in response to an operation of the user of selecting a disease option, determining a target disease, and creating a follow-up template matched with the target disease, the follow-up template including a plurality of follow-up record forms and a time interval corresponding to each follow-up record form, each of the plurality of follow-up record forms being a follow-up record form corresponding to the target disease, and the time interval being a time interval between a follow-up date set for the target disease and a date when a follow-up task is created.

26. The medical information processing method according to claim 25, wherein the follow-up template interface further includes a template content column, the template content column includes a new button, and the template content column is configured to display a created follow-up template; and
creating the follow-up template matched with the target disease includes:

displaying a follow-up template creation dialog box in the follow-up template interface in response to an operation of selecting the new button, the follow-up template creation dialog box including a follow-up record form editing box, a time interval editing box, an OK button and a cancel button, the follow-up record form editing box having at least one form option related to the target disease, and each form option corresponding to a preset follow-up record form;
in response to an operation of the user of selecting a form option, displaying a name of a follow-up record form corresponding to the selected form option in the follow-up record form editing box;
displaying a set time interval in the time interval editing box in response to an operation of the user of entering a time interval; and
displaying the follow-up template in the template content column in response to an operation of the user of selecting the OK button.

27. The medical information processing method according to claim 10, further comprising:

displaying at least one preset time option and a follow-up content selector corresponding to each preset time option; wherein
determining the follow-up task matched with the disease type in response to the third operation of the user includes:
in response to selection of the user of a preset time option and selection of the user of a follow-up content selector corresponding to the preset time option, determining follow-up content in the selected follow-up content selector and a follow-up time in the preset time option as the follow-up task matched with the disease type.

28. The medical information processing method according to claim 10, further comprising:

displaying a time selector and a follow-up content selector corresponding to the time selector; wherein
determining the follow-up task matched with the disease type in response to the third operation of the user includes:
in response to selection of the user of the time selector and the follow-up content selector corresponding to the time selector, determining follow-up content in the selected follow-up content selector and a follow-up time in the time selector as the follow-up task matched with the disease type.

29. The medical information processing method according to any one of claims 10 to 28, further comprising:

displaying a disease type selector; and
determining the disease type corresponding to the disease of the target patient in response to an operation of the user on the disease type selector.

30. The medical information processing method according to any one of claims 10 to 29, further comprising:

displaying a follow-up person selector; and

associating a follow-up person with the follow-up task in response to an operation of the user on the follow-up person selector.

**31.** The medical information processing method according to any one of claims 2 to 30, further comprising:

displaying a card registration and creation index item;

displaying at least basic information blank text boxes and a diagnosis information blank text box in response to an operation of the user on the card registration and creation index item, the basic information blank text boxes being configured to present basic information of a new patient, and the diagnosis information blank text box being configured to present diagnosis information of the new patient; and

in response to an operation of the user of saving information presented in the basic information blank text boxes and the diagnosis information blank text box, adding part of the information presented in the basic information blank text boxes and the diagnosis information blank text box as visit information into the patient list.

**32.** The medical information processing method according to claim 1, wherein the at least one navigation tab includes a health prescription management tab; in response to the first operation of the user, determining the target navigation tab, and displaying the management information of the disease of the patient matched with the target navigation tab include:

displaying a health prescription list in response to the first operation of the user on the health prescription management tab, the health prescription list including at least one line of prescription information, each line of prescription information including visit information of a patient and at least one first medical information operation item, and the at least one first medical information operation item including a second detail index item; and

the method further comprises:

displaying a health prescription text box in response to an operation of the user on the second detail index item, a medical regimen corresponding to the line of prescription information being presented in the health prescription text box, and the medical regimen including at least one of a medication regimen, a diet regimen or an exercise regimen.

**33.** The medical information processing method according to claim 32, wherein each line of prescription information further includes state information, and the state information includes one of being valid, being deactivated, or being invalid;

in a case where state information in a line of prescription information is being valid, at least one first medical information operation item in the line of prescription information further includes a deactivation item and an invalidation item; and

the method further comprises:

in response to an operation of the user on the deactivation item or the invalidation item, changing the state information in the line of prescription information from being valid to being deactivated or being invalid, and hiding the deactivation item and the invalidation item.

**34.** The medical information processing method according to claim 32 or 33, further comprising:

displaying a new health prescription creation index item;

displaying a patient search box and a health prescription text box in response to an operation of the user on the new health prescription creation index item, the health prescription text box being a blank box;

in response to an operation of querying information entered in the patient search box, determining a target patient, and displaying visit information of the target patient;

presenting a medical regimen matched with a disease of the target patient in the health prescription text box in response to a second operation of the user; and

in response to an operation of the user of saving the medical regimen, adding the visit information of the target patient as part of information in a new line of prescription information into the health prescription list, and associating the medical regimen of the target patient in a second detail index item in the new line of prescription information.

**35.** The medical information processing method according to any one of claims 32 to 34, further comprising:

displaying a card registration and creation index item;

displaying basic information blank text boxes and a diagnosis information blank text box in response to an operation of the user on the card registration and creation index item, the basic information blank text boxes being configured to present basic information of a new patient, and the diagnosis information blank text box being configured to present diagnosis information of the new patient; and

in response to an operation of the user of saving information presented in the basic information blank text boxes and the diagnosis information blank text box, adding part of the information presented in the basic information blank text boxes and the diagnosis information blank text box as visit information into the patient list.

36. The medical information processing method according to claim 1, wherein the at least one navigation tab includes a follow-up management tab; and in response to the first operation of the user, determining the target navigation tab, and displaying the management information of the disease of the patient matched with the target navigation tab include: displaying a follow-up task list in response to the first operation of the user on the follow-up management tab, the follow-up task list including at least one line of follow-up information, and each line of follow-up information including visit information of a patient, a follow-up time, follow-up content, and at least one second medical information operation item.

37. The medical information processing method according to claim 36, wherein each line of follow-up information further includes a follow-up state, and the follow-up state includes one of no follow-up and being completed;

   in a case where a follow-up state in a line of follow-up information is no follow-up, the at least one second medical information operation item in the line of follow-up information further includes a follow-up performance index item; and the method further comprises:

      displaying a follow-up record form corresponding to follow-up content in response to an operation of the user on the follow-up performance index item; and in response to an operation of saving content entered in the follow-up record form, changing the follow-up state in the line of follow-up information from no follow-up to being completed, and changing the follow-up performance index item to a third detail index item.

38. The medical information processing method according to claim 36 or 37, wherein the at least one second medical information operation item includes a follow-up comparison index item; and the method further comprises: in response to an operation of the user on the follow-up comparison index item, acquiring and displaying comparison results of at least two follow-up contents matched with a same disease corresponding to the patient in the line of follow-up information.

39. The medical information processing method according to any one of claims 36 to 38, wherein at least one second medical information operation item further includes a follow-up creation index item; and the method further comprises:

      determining a new follow-up task matched with a disease type corresponding to a disease of a target patient in response to an operation of the user on the follow-up creation index item and a third operation of the user, the new follow-up task including follow-up content matched with the disease of the patient and a follow-up time corresponding to the follow-up content; and adding the new follow-up task into the follow-up task list in response to an operation of the user of saving the new follow-up task.

40. The medical information processing method according to any one of claim 36 to 39, further comprising:

      displaying a new follow-up creation index item; displaying a patient search box and a disease type selector in response to an operation of the user on the new follow-up creation index item; in response to an operation of querying information entered in the patient search box, determining a target patient, and displaying visit information of the target patient; determining a new follow-up task matched with the disease of the target patient in response to a third operation of the user, the new follow-up task including follow-up content matched with the disease of the target patient and a follow-up time corresponding to the follow-up content; and adding the visit information of the target patient and the new follow-up task into the follow-up task list in response to an operation of the user of saving the new follow-up task.

41. The medical information processing method according to claim 40, further comprising:

      displaying a card registration and creation index item; displaying basic information blank text boxes and a diagnosis information blank text box in response to an operation of the user on the card registration and creation index item, the basic information blank text boxes being configured to present basic information of a new patient, and the diagnosis information blank text box being configured to present diagnosis information of the new patient; and in response to an operation of the user of saving information presented in the basic information blank text boxes and the diagnosis information blank text box, adding part of the information presented in the basic information blank text boxes and the diagnosis information blank text box as visit information into a patient list.

42. The medical information processing method according to claim 1, further comprising:

    displaying a login information box; and
    displaying work content and workload of the user on a current working day in response to an operation of logging in login information entered in the login information box.

43. The medical information processing method according to any one of claims 1 to 42, wherein the at least one navigation tab includes a disease assessment tab; and
    in response to the first operation of the user, determining the target navigation tab, and displaying the management information of the disease of the patient matched with the target navigation tab include:

    displaying a disease assessment information text box in response to an operation of the user on the disease assessment tab; and
    displaying a disease assessment report corresponding to disease assessment information in response to an operation of saving information entered in the disease assessment information text box.

44. The medical information processing method according to any one of claims 1 to 43, wherein the at least one navigation tab includes a smart question and answer tab; and
    in response to the first operation of the user, determining the target navigation tab, and displaying the management information of the disease of the patient matched with the target navigation tab include:

    displaying a question search box in response to an operation of the user on the smart question and answer tab; and
    in response to an operation of querying a question entered in the question search box, displaying at least one answer associated with the question.

45. The medical information processing method according to any one of claims 1 to 44, wherein the at least one navigation tab further includes a consultation tab; and
    in response to the first operation of the user, determining the target navigation tab, and displaying the management information of the disease of the patient matched with the target navigation tab include:

    displaying a consultation list in response to an operation of the user on the consultation tab, the consultation list including at least one line of consultation information from a server, and each line of consultation information including visit information of a patient, a question summary, a consultation type, and a fourth detail index item; and displaying at least a consultation question corresponding to the line of consultation information in response to an operation of the user on the fourth detail index item.

46. The medical information processing method according to claim 45, wherein each line of consultation information further includes a reply state, and the reply state includes one of being answered and being unanswered; and
    in a case where a reply state in a line of consultation information is being unanswered, the method further comprises:

    further displaying a reply text box matched with the consultation type in the line of consultation information in response to the operation of the user on the fourth detail index item;
    presenting an answer matched with the consultation question in response to an operation of the user on the reply text box; and
    changing the reply state in the line of consultation information from being unanswered to being answered in response to an operation of the user of saving the answer.

47. The medical information processing method according to claim 46, wherein the consultation type includes report interpretation; and the reply text box includes an abnormal question summary text box and a health advice text box; and
    the method further comprises:

    further displaying at least one examination report in response to the operation of the user on the fourth detail index item; wherein
    presenting the answer matched with the consultation question in response to the operation of the user on the reply text box includes:

    presenting an abnormal question corresponding to the at least one examination report and an interpretation text of the abnormal question in response to an operation of the user on the abnormal question summary text box; and
    presenting a medical regimen matched with the abnormal question in response to an operation of the user on the health advice text box, the medical regimen including at least one of a medication regimen, a diet regimen, or an exercise regimen.

**48.** The medical information processing method according to claim 47, further comprising:

> displaying an abnormal question template tab; wherein
> presenting the abnormal question corresponding to the at least one examination report and the interpretation text of the abnormal question in response to the operation of the user on the abnormal question summary text box includes:
>
> > displaying a third classification list in response to an operation of the user on the abnormal question template tab, the third classification list including at least one question type;
> > in response to an operation of the user on a question type corresponding to the abnormal question in the third classification list, displaying the abnormal question corresponding to the question type and at least one interpretation text template corresponding to the abnormal question; and
> > in response to an operation of the user on an interpretation text template, presenting content of the interpretation text template and the abnormal question in the abnormal question summary text box.

**49.** The medical information processing method according to claim 48, further comprising:

> displaying a health advice template tab; wherein presenting the medical regimen matched with the abnormal question in response to the operation of the user on the health advice text box includes:
>
> > displaying an entry sub-tab in response to an operation of the user on the health advice template tab;
> > displaying a first classification list in response to an operation of the user on the entry sub-tab, the first classification list including a plurality of first-level items and at least one disease type included in each first-level item, and the plurality of first-level items including a medication regimen item, a diet regimen item, and an exercise regimen item;
> > in response to an operation of the user on a disease type corresponding to the abnormal question in a first-level item in the first classification list, displaying at least one entry text template corresponding to the disease type in the first-level item; and
> > in response to an operation of the user on an entry text template, presenting content of the entry text template in the health advice text box;
and/or
presenting the medical regimen matched with the abnormal question in response to the operation of the user on the health advice text box includes:

> > displaying a health prescription template sub-tab in response to the operation of the user on the health advice template tab;
> > displaying a second classification list in response to an operation of the user on the health prescription template sub-tab, the second classification list including at least one disease type;
> > displaying at least one medical regimen template corresponding to the disease type in response to an operation of the user on the disease type corresponding to the abnormal question in the second classification list; and
> > in response to an operation of the user on a medical regimen template, presenting at least part of content of the medical regimen template in the health advice text box.

**50.** The medical information processing method according to any one of claims 1 to 49, wherein the at least one navigation tab further includes a statistical tab; and
in response to the first operation of the user, determining the target navigation tab, and displaying the management information of the disease of the patient matched with the target navigation tab include: displaying statistical information in response to an operation of the user on the statistical tab, the statistical information being information obtained by performing classified statistics on the patient and the management information of the disease of the patient.

**51.** The medical information processing method according to claim 1, further comprising:

> displaying a knowledge base management tab;
> displaying an abnormal question template tab and a health advice template tab in response to an operation of the user on the knowledge base management tab;
> displaying a question node setup item and an interpretation text template setup item in response to an operation of the user on the abnormal question template tab, the question node setup item being configured to instruct to set question types, the interpretation text template setup item being configured to instruct to add a new interpretation text template, to instruct to edit the interpretation text template, and/or to instruct to delete the interpretation text template; displaying an entry sub-tab in response to an operation of the user on the health advice template tab; and

displaying a first node setup item and an entry text template setup item in response to an operation of the user on the entry sub-tab, the first node setup item being configured to instruct to set item types of health advice and a disease type matched with each item type, the item types of the health advice including a medication regimen item, a diet regimen item, and an exercise regimen item, and the entry text template setup item being configured to instruct to add a new entry text template, to instruct to edit the entry text template, and/or to instruct to delete the entry text template;

and/or

displaying a health prescription template sub-tab in response to the operation of the user on the health advice template tab; and

displaying a second node setup item and a health prescription template setup item in response to an operation of the user on the health prescription template sub-tab, the second node setup item being configured to instruct to set disease types, and the health prescription template setup item being configured to instruct to add a new medical regimen template, to instruct to edit the medical regimen template, and/or to instruct to delete the medical regimen template.

52. A medical information acquisition method, comprising:

displaying a plurality of role selection items, the plurality of role selection items including a medical worker and a patient;

in response to an operation of a user of selecting a role selection item, determining a role of the user, and displaying a login information box corresponding to the role of the user, login information boxes corresponding to different roles being different; and

displaying at least one function tab corresponding to the role of the user in response to login information of the user entered in the login information box, at least one function tab corresponding to the medical worker being configured to instruct to display management information of diseases of a plurality of patients, and at least one function tab corresponding to the patient being configured to instruct to display management information of a disease of the patient.

53. The medical information acquisition method according to claim 52, wherein the role of the user is the medical worker, and the at least one function tab includes a work schedule tab; and

the method further comprises:

displaying at least one work item index item of the user in response to an operation of the user on the work schedule tab, the work item index item including at least one of a question consultation index item, a report interpretation index item, and a follow-up plan index item; and in response to an operation of the user on a work item index item, acquiring and displaying work content corresponding to the work item index item of the user from a server.

54. The medical information acquisition method according to claim 53, further comprising:

acquiring an amount of work content corresponding to the work item index item of the user on a target working day from the server, and displaying the amount of the work content corresponding to the work item index item on the work item index item.

55. The medical information acquisition method according to claim 54, further comprising:

displaying a working day option; and determining the target working day in response to an operation of the user on the working day option.

56. The medical information acquisition method according to any one of claims 53 to 55, wherein the at least one function tab further include a statistical query tab; and

the method further comprises:

acquiring and displaying at least statistical information of follow-up condition of the plurality of patients from the server in response to an operation of the user on the statistical query tab, the statistical information of the follow-up condition of the plurality of patients being statistical information of health condition of the patients obtained after at least one follow-up is performed on the patients.

57. The medical information acquisition method according to claim 52, wherein the role of the user is the patient, and the at least one function tab includes a disease assessment tab; and

the method further comprises:

displaying at least one disease assessment question in response to an operation of the user on the disease assessment tab; and acquiring and displaying a disease assessment result of the user from a server in response to an answer of the user to each disease assessment question.

**58.** The medical information acquisition method according to claim 52 or 57, wherein the role of the user is the patient, and the at least one function tab includes a smart question and answer tab; and
the method further comprises:

displaying a question search box in response to an operation of the user on the smart question and answer tab; and
in response to an operation of the user of querying a question entered in the question search box, acquiring and displaying at least one answer associated with the question from a server.

**59.** The medical information acquisition method according to any one of claims 52 or 57-58, wherein the role of the user is the patient, and the at least one function tab includes a consultation tab; and
the method further comprises:

displaying a basic information text box and a disease condition description text box in response to an operation of the user on the consultation tab;
in response to an operation of the user of submitting information of the patient entered in the basic information text box and a consultation question entered in the disease condition description text box, transmitting the information in the basic information text box and the consultation question in the disease condition description text box to a server; and
acquiring and displaying an answer matched with the consultation question from the server.

**60.** The medical information acquisition method according to claim 59, further comprising:

displaying an attachment addition item; and
adding at least one attachment in response to an operation of the user on the attachment addition item; wherein
in response to the operation of the user of submitting the information of the patient entered in the basic information text box and the consultation question entered in the disease condition description text box, transmitting the information in the basic information text box and the consultation question in the disease condition description text box to the server includes:
in response to an operation of the user of submitting the information of the patient entered in the basic information text box, the consultation question entered in the disease condition description text box, and the at least one attachment, transmitting the information in the basic information text box, the consultation question in the disease condition description text box, and the at least one attachment to the server.

**61.** The medical information acquisition method according to claim 59 or 60, further comprising:

displaying a doctor selector; and
determining a doctor to be consulted in response to an operation of the user on the doctor selector; wherein
in response to the operation of the user of submitting the information of the patient entered in the basic information text box and the consultation question entered in the disease condition description text box, transmitting the information in the basic information text box and the consultation question in the disease condition description text box to the server includes:
in response to the operation of the user of submitting the information of the patient entered in the basic information text box and the consultation question entered in the disease condition description text box, transmitting the information in the basic information text box, the consultation question in the disease condition description text box, and a doctor to be consulted corresponding to the consultation question to the server.

**62.** The medical information acquisition method according to any one of claims 52 or 57-61, wherein the role of the user is the patient, and the at least one function tab includes a health data statistical tab; and
the method further comprises:
acquiring and displaying a classified statistical result of the management information of the disease of the patient from the server in response to an operation of the user on the health data statistical tab.

**63.** The medical information acquisition method according to claim 62, wherein the plurality of role selection items further include a manager, and the at least one function tab includes a statistical query tab; and
the method further comprises:
displaying a query item in response to an operation of the user on the statistical query tab, the query item being configured to instruct to query the classified statistical result, and the classified statistical result being obtained according to classified statistics of data related to the patient and a doctor stored in the server.

**64.** A medical information interaction method, comprising:

displaying, by a medical information processing apparatus, at least one navigation tab, and in response to a first operation of a user, determining a target navigation tab, and displaying management information of a disease of a patient matched with the target navigation tab;
transmitting, by the medical information processing apparatus, the management information of the disease of the patient to a server;
acquiring, by a medical information acquisition apparatus, the management information of the disease of the patient from the server; and
displaying, by the medical information acquisition apparatus, a plurality of role selection items, the plurality of role selection items including a medical worker and a patient; in response to an operation of the user of selecting a role selection item, determining, by the medical information acquisition apparatus, a role of the user, and displaying, by the medical information acquisition apparatus, a login information box corresponding to the role of the user, login information boxes corresponding to different roles being different; and displaying, by the medical information acquisition apparatus, at least one function tab corresponding to the role of the user in response to login information of the user entered in the login information box, at least one function tab corresponding to the medical worker being configured to instruct to display management information of diseases of a plurality of patients, and at least one function tab corresponding to the patient being configured to instruct to display management information of a disease of the patient.

65. A medical information processing apparatus, comprising:

a first display; and
a first processor coupled to the first display, the first processor being configured to: control the first display to display at least one navigation tab; and in response to a first operation of a user, determine a target navigation tab, and control the first display to display management information of a disease of a patient matched with the target navigation tab, the target navigation tab being one of the at least one navigation tab.

66. The medical information processing apparatus according to claim 65, further comprising:
a first input device coupled to the first processor, the first input device being configured to enter the first operation of the user.

67. A medical information acquisition apparatus, comprising:

a second display; and
a second processor coupled to the second display, the second processor being configured to: control the second display to display a plurality of role selection items, the plurality of role selection items including a medical worker and a patient; in response to an operation of a user of selecting a role selection item, determine a role of the user, and control the second display to display a login information box corresponding to the role of the user, login information boxes corresponding to different roles being different; and control the second display to display at least one function tab corresponding to the role of the user in response to login information of the user entered in the login information box, at least one function tab corresponding to the medical worker being configured to instruct to display management information of diseases of a plurality of patients, and at least one function tab corresponding to the patient being configured to instruct to display management information of a disease of the patient.

68. The medical information acquisition apparatus according to claim 67, further comprising:
a second input device coupled to the second processor, the second input device being configured to enter the operation of the user of selecting a role selection item, and to enter the login information of the user.

69. A computer equipment, comprising a memory and a processor, the memory storing computer programs that, when executed on the processor, cause the processor to implement the medical information processing method according to any one of claims 1 to 51, the medical information acquisition method according to any one of claims 52 to 63, and/or the medical information interaction method according to claim 64.

70. A non-transitory computer-readable storage medium, storing computer programs that, when executed on a processor, cause the processor to perform one or more steps in the medical information processing method according to any one of claims 1 to 51, one or more steps in the medical information acquisition method according to any one of claims 52 to 63, and/or one or more steps in the medical information interaction method according to claim 64.

Display at least one navigation tab — S1

In response to a first operation of a user, determine a target navigation tab, and display management information of a disease of a patient matched with the target navigation tab, the target navigation tab being one of the at least one navigation tab — S2

FIG. 1

EP 4 068 292 A1

100

1

81    8

Patient management ✕    More⌄

Patient list

11

103    104

| Medical record number | Name | Gender ⌄ | Mobile phone number | Query | Card Registration and creation | 105 |

☐ Hypertension ☐ Diabetes ☐ Hyperlipidemia ☐ Osteoarthritis ☐ Coronary heart disease ☐ Rheumatoid arthritis ☐ Chronic obstructive pulmonary disease ☐ Cerebrovascular disease ☐ Bronchitis

101    102    1021

| Medical record number | Name | Gender | Age | Mobile phone number | Disease type | Operation | | |
|---|---|---|---|---|---|---|---|---|
| CD10000101 | X1 | Female | 39 years old | 151******** | Hypertension | Health prescription creation | Follow-up creation | Details |
| CD10000102 | X2 | Female | 60 years old | 185******** | Hypertension | Health prescription creation | Follow-up creation | Details |
| CD10000103 | X3 | Male | 31 years old | 186******** | Hypertension, diabetes | Health prescription creation | Follow-up creation | Details |
| CD10000104 | X4 | Female | 50 years old | 150******** | Hypertension, diabetes | Health prescription creation | Follow-up creation | Details |
| CD10000105 | X5 | Male | 35 years old | 133******** | Hypertension | Health prescription creation | Follow-up creation | Details |
| CD10000001 | X6 | Male | 31 years old | 131******** | Diabetes | Health prescription creation | Follow-up creation | Details |
| CD10000002 | X7 | Male | 31 years old | 186******** | Diabetes | Health prescription creation | Follow-up creation | Details |

FIG. 2

200

EP 4 068 292 A1

FIG. 3

200

FIG. 4

EP 4 068 292 A1

200

Create a health prescription

Basic information—Hypertension, diabetes

ID number: 411*************** 　　Name: X3

Medical record number: CD10000103 　　Mobile phone number: 186*********

Date of birth: 1988-01-08 　　Age: 31 years old 　　Gender: Male

Health prescription 　　201 　　213 　　Save as a template 　　Save 　　211

**Medication regimen** 　　Save an entry

Please input ...

**Diet regimen** 　　Save an entry

Please input ...

**Exercise regimen** 　　Save an entry

Please input ...

Follow-up management ✕ 　　Patient management ✕ 　　Health prescription creation ✕ 　　81

admin ⌄ 　　More⌄

Historical prescription 　　My template 　　Entry 　　8 　　209 　　210

Prescribing date: 2019-11-19 Prescribing doctor: ZOU** 　　Reuse

Prescribing date: 2019-11-19 Prescribing doctor: ZOU** 　　Reuse

Prescribing date: 2019-05-08 Prescribing doctor: ZOU** 　　Reuse

Medication regimen

Metformin Hydrochloride Tablets (Glucophage) 0.5 mg po tid;
Gliquidone Tablets 30 mg po tid

Diet regimen

Low-salt and low-fat diet: (1) the Chinese Nutrition Society recommends that daily salt intake for adults should not exceed 6 g, and daily salt intake for patients with hypertension should exceed 3 g (specifically, salt intake per person per meal does not exceed 2 g, that is, salt intake per person per day does not exceed 6 g, i.e., a bottle cap of salt after a rubber pad is removed from an ordinary beer bottle cap); (2) daily cooking oil consumption<25 g (half a tael, equivalent to 2.5 tablespoons of cooking oil); (3) it is recommended that the patients with hypertension consume 400~500 g (8 taels to 1 catty) of fresh vegetables per day

Exercise regimen

Exercise: exercises that the patients with hypertension may select include aerobic exercise, resistance exercise, flexibility exercise, and balance function. It should be noted that, blood pressure of the patients with hypertension is usually at a high level in the early morning, and the early morning is a period with high incidence of cardiovascular events. Therefore, it is best to exercise in the afternoon or evening. In addition, there should be 5 to 10 minutes of warm-up exercise and relaxation exercise before and after formal exercise to avoid sudden strenuous exercise and stop without transition

Others
Ensure adequate sleep

FIG. 5

EP 4 068 292 A1

300

admin ˅

Follow-up management ✕ | Patient management ✕ | Health prescription creation ✕ | **Follow-up creation ✕** | More˅

**Create a follow-up task**

Basic information—hypertension, diabetes

Name: X3      Gender: Male      Date of birth: 1988-01-08      Age: 31 years old
Mobile phone number: 186********    Medical record number: CD10000103    ID number: 411**************

Follow-up information

| Standard template | Customize |
|---|---|

Template classification:

Please select

During follow-up | Hypertension > | Stage 1 hypertension
 | Diabetes > | Stage 2 hypertension
 | Hyperlipidemia | Stage 3 hypertension

☐ One day later 2019-11-26
Please select follow-up content: Please select
☐ One month later 2019-12-25
Please select follow-up content: Please select
☐ Two months later 2020-01-24
Please select follow-up content: Please select
☐ Three months later 2020-02-23
Please select follow-up content: Please select
☐ Others 📅 Select date and time
Please select follow-up content: Please select

Creator: Dr. ZHANG    Select an assignment object: Please select

301 · 3011 · 3021 · 302 · 307 · 306 · 304 · 3011 · 305

1 · 81 · 8 · 11

FIG. 6

300

EP 4 068 292 A1

admin ▾

Follow-up management ✕   Patient management ✕   Health prescription creation ✕   Follow-up creation ✕

More▾

**Create a follow-up task**

Basic information– hypertension, diabetes

Name: X3    Gender: Male    Date of birth: 1988-01-08    Age: 31 years old

Mobile phone number: 186*******    Medical record number: CD10000103    ID number: 411***************

Follow-up information

301  3011   Standard template    309   Customize   310

Template classification:   Hypertension/Stage 1 hypertension   311

| Follow-up time | Follow-up content |
| --- | --- |
| ☐ 2020-01-24 | Follow-up service record form for a patient with diabetes |
| ☐ 2019-11-26 | Follow-up service record form for a patient with hypertension |
| ☐ 2019-12-02 | Follow-up service record form for a patient with hypertension |
| ☐ 2019-12-09 | Follow-up service record form for a patient with hypertension |
| ☐ 2019-11-26 | Follow-up service record form for a patient with diabetes |
| ☐ 2019-12-02 | Follow-up service record form for a patient with diabetes |
| ☐ 2019-12-25 | Follow-up service record form for a patient with hypertension |

303   307   309

Creator: Dr. ZHANG    Select an assignment object⸸    Please select   3011

☐ One day later 2019-11-26   306
Please select follow-up content:   Please select ▾
☐ One month later 2019-12-25
Please select follow-up content:   Please select ▾
☐ Two months later 2020-01-24
Please select follow-up content:   Please select ▾
☐ Three months later 2020-02-23
Please select follow-up content:   Please select ▾
☑ Others  📅  2019-11-29
Please select follow-up content:   Follow-up service record form for a patient with diabetes   308

309   305   304   3011

Add

FIG. 7

FIG. 8

FIG. 9

EP 4 068 292 A1

200

1　81　8

Patient management ×　Health prescription management ×　Health prescription creation ×　More⌄

11

**Create a health prescription** — 103 — 105

Medical record number/Name/Mobile phone number 🔍

Card registration and creation — 213

211

Save as a template　Save

Health prescription — 201

Save an entry

**Medication regimen**

Please input...

Save an entry

**Diet regimen**

Please input...

Save an entry

**Exercise regimen**

Please input...

Historical prescription

My template　　Entry

Template classification　Add a top node

Template content　Edit Reference all　Save

▶ Hypertension

▶ Diabetes　＋ ✎ 🗑

Please select a template category!

Osteoarthritis

Hyperlipidemia

Pharyngitis

Chronic obstructive pulmonary disease

Cerebrovascular

Bronchiolitis

Hyperlipidemia

Pharyngitis

203　204

FIG. 10

EP 4 068 292 A1

600

1 — 81 — 8 — 602 — 603 — 605

Follow-up management ✕

| Medical record number | Patient name | Gender ▾ | Mobile phone number | Assignment object ▾ | State ▾ |

Query | Follow-up creation | Follow-up comparison

11

Custom event: 📅 2018-10-30 to 2020-12-15 — 606

601

| Medical record number | Name | Gender | Age | Mobile phone number | Disease type | Assignment object | Follow-up date | Follow-up state | Follow-up content | Operation |
|---|---|---|---|---|---|---|---|---|---|---|
| CD10000131 | CHEN san | Male | 32 years old | 152******** | Hypertension | Dr. WANG | 2020-11-21 | No follow-up | Follow-up service record form for a patient with hypertension | Follow-up performance \| Follow-up creation \| Deletion \| Follow-up comparison — 604 |
| CD10000138 | X10 | Male | 28 years old | 183******** | Hypertension | Dr. LI | 2020-11-06 | Completed | Health assessment (test) | Details \| Follow-up creation \| Follow-up comparison — 6041 / 6042 |
| CD10000103 | X3 | Male | 31 years old | 186******** | Hypertension, diabetes | Dr. ZHANG | 2020-10-27 | No follow-up | Life quality form for a patient with hypertension | Follow-up performance \| Follow-up creation \| Deletion \| Follow-up comparison |
| CD10000103 | X3 | Male | 31 years old | 186******** | Hypertension, diabetes | Dr. ZHANG | 2020-10-12 | No follow-up | Life quality form for a patient with hypertension | Follow-up performance \| Follow-up creation \| Deletion \| Follow-up comparison |
| CD10000103 | X3 | Male | 31 years old | 186******** | Hypertension, diabetes | Dr. LI | 2020-10-11 | Completed | Health assessment(test) | Details \| Follow-up creation \| Follow-up comparison |
| CD10000103 | X3 | Male | 31 years old | 186******** | Hypertension, diabetes | Dr. ZHANG | 2020-09-28 | No follow-up | Life quality form for a patient with hypertension | Follow-up performance \| Follow-up creation \| Deletion \| Follow-up comparison |

FIG. 11

700

81       8

admin ⌄

Follow-up management ✕ | Follow-up task performance ✕    More ⌄

**Follow-up task performance**

Basic information

Name: X3     Gender: Male     Date of birth: 1988-01-08     Age: 31 years old
Mobile phone number: 186********    Medical record number: CD10000103    ID number: 411**************

Start date  to Finish date | Query     Follow-up service record form for a patient with hypertension     Save | Submit

visit history    Follow-up record

| 2019-02-21 12：30：28 Details |
| Clinic A   General clinic |
| Diabetes   Dr. ZOU |

| 2019-02-21 12：30：28 Details |
| Clinic B   General clinic |
| Diabetes   Dr. ZOU |

| 2019-02-21 12：30：28 Details |
| Clinic A   General clinic |
| Diabetes   Dr. ZOU |

| 2019-02-21 12：30：28 Details |
| Hospital D   General clinic |
| Diabetes   Dr. ZOU |

| 2019-02-21 12：30：28 Details |
| Clinic A   General clinic |
| Diabetes   Dr. ZOU |

Follow-up date: 2019-11-25    Follow-up way: Please select a follow-up way

**Symptom**
- ☐ No symptom   ☐ Headache and dizziness   ☐ Nausea and vomiting
- ☐ Giddiness and tinnitus   ☐ Difficult breathing   ☐ Palpitations and chest tightness
- ☐ Nasal bleeding   ☐ Numbness of limbs   ☐ Lower limb edema
- Others: Please enter other symptoms

**Sign**

| | Current value | Target value to be adjusted during next follow-up |
|---|---|---|
| Blood pressure (mmHg) | | |
| Weight (kg) | | |
| Body mass index (BMI) kg/m² | | |
| Heart rate (times/minute) | | |
| Others | Please enter other signs   B2a | |

**Lifestyle guidance**

| | Current value | Target value to be adjusted during next follow-up |
|---|---|---|
| Daily smoking consumption | | |
| Daily alcohol consumption (ael) | | |
| Exercise | ⌄ (minutes/time) | ⌄ (minutes/time) |
| Salt intake condition (salty & mild) | ○ Light ○ Medium ○ Heavy | ○ Light ○ Medium ○ Heavy |
| Psychological adjustment | ○ Good ○ Fair ○ Poor | |
| Treatment compliance | ○ Good ○ Fair ○ Poor | |

B100

| Auxiliary examination | Please enter auxiliary examination |
| Medication compliance | ○ Regular ○ Intermittent ○ Not taking medicine |
| Adverse drug reaction | ○ No ○ Yes Please enter adverse reaction |
| Category of this follow-up | ○ Satisfactory control ○ Unsatisfactory control ○ Adverse reaction ○ Complication Note: |

**Medication condition**

| | Drug name | Usage | Frequency | Dosage per time | Operation |
|---|---|---|---|---|---|
| This prescription | | | | | 🗑 ⊕ |
| Prescriptions from other institutions | Prescriptions from other institutions | | | | |

**Referral**

| Reason | Please enter a reason | | |
|---|---|---|---|
| Referral institution | Please enter a referral institution | Referral department | Please enter a referral department |

## FIG. 12

| Follow-up task creation ✕ | Patient management ✕ | Card registration and creation ✕ | Follow-up management ✕ | Follow-up comparison ✕ | | More |
|---|---|---|---|---|---|---|
| Triglyceride(TG) | 4.2 | | | | Edit    Select |
| Blood pressure(mmHg) | 153/108 | 156/106 | 166/112 | |
| Weight (Kg) | Current value: 78; next target value: 74 | Current value: 77; next target value: 73 | Current value: 78; next target value: 75 | |
| Body mass index (BMI) | Current value: 26.7; next target value: 25.9 | Current value: 25.7; next target value: 24.9 | | |
| Pulsation of dorsal artery of foot | | Weakened on a left side | | |
| Heart rate (times/minute) | 85 | | | |
| Other positive signs | Breath 40, pulse 87 | | | |
| Daily smoking consumption | Current value: 4; next target value: 0 | | | |
| Daily alcohol consumption (tael) | | | | |
| Exercise | Current value: 1 time/week to 2 time /week, 15 minutes/time; next target value: 5 times/week to 6 times/week, 30 minutes | | | |
| Salt intake(salty & mild) | | | | |
| Low-fat diet condition | Current value: heavy; next target value: medium | | | |
| Staple food (g/day) | | | | |
| Psychological adjustment | Good | | | |

FIG. 13

FIG. 14

FIG. 15

FIG. 16

EP 4 068 292 A1

FIG. 17

EP 4 068 292 A1

Follow-up management ✕ | health prescription management ✕ | Disease assessment ✕ | Disease assessment report ✕

admin ⌄

More ⌄

**Examination result**

Family history of diabetes — Father or mother has diabetes

Education — High school and above

Exercise — No

Smoke — Yes

Examination — Fasting blood glucose FPG 8 mmol/L    Alanine aminotransferase ALT 43IU/L    Glomerular filtration rate eGFR 120 ml/min    HDL-Cholesterol HDL-C 123 mmol/L

Triglyceride TG 7 mmol/L    Body mass index 26.83 kg/m²    Systolic pressure 160 mmHg

**Risk assessment result**

○ **Your risk of developing diabetes within 5 years is 100%**

○ You belong to a high-risk group of diabetes

**Recommendations for a patient**

| Main risk factor | Examination result | Ideal target value |
|---|---|---|
| Smoking condition | Yes | Quit smoking |
| Excercise time | No | >=1 hour per week |
| Hypertension | Yes | No hypertension |
| Body mass index | 26.83 kg/m² | 18.5-23.9 kg/m² |
| Waistline | 98.0 cm | <90 cm |
| Fasting blood glucose FPG | 8.0 mmol/L | 3.9-6.1 mmol/L |

**Health prescription**

Reduce the risk of diabetes through diet control and exercise

Perform regular follow-up and provide social and psychological support, so as to ensure that a lifestyle of the patient changes, and the patient sticks to the lifestyle

Check blood glucose regularly; and pay close attention to other cardiovascular risk factors (such as smoking, hypertension, and dyslipidemia), and take appropriate intervention measures

Specific goals are:

(1) Make BMI of overweight or obese people reach or close to 24 kg/m², or lose at least 7% of weight

(2) Reduce a total amount of calories in daily diet by at least 400 to 500 kcal (1 kcal = 4.184 kJ)

(3) Saturated fatty acid intake accounts for less than 30% of total fatty acid intake

(4) Maintain moderate-intensity physical activity for at least 150 min/week

## FIG. 18

900

admin ∨

| Follow-up management ✕ | Follow-up task performance ✕ | Follow-up template ✕ | Disease assessment ✕ | Smart question and answer ✕ | More ∨ |

**▌FAQ smart question and answer**

[ Question ]  [Query]

| Question | Answer | Number of times of being asked | Number of times of being given likes | I want to give a like too |
|---|---|---|---|---|
| It is found that the fasting blood glucose is 7.02 in a previous physical examination. Is this considered diabetes? | Hello, the diagnostic criteria for diabetes is that the fasting blood glucose is greater than 7.0 mmol/L. Since your blood glucose is slightly higher, you cannot be diagnosed with diabetes, and it is recommended to go to the hospital for a glucose tolerance test for diagnosis. Moreover, you should pay attention to your own diet, should eat more fruits and vegetables, eat more foods rich in vitamins and fiber, and limit intake of cured, baked, smoked, marinated meat products, and drink plenty of water every day | 1 | 1 | Give a like |
| What are symptoms of diabetes and can it be treated? What medicine should be taken, or what can be eaten and what cannot be eaten in daily life? | Hello, main symptoms of type 1 diabetes are "three more and one less", i.e., polydipsia, polyuria, polyphagia and weight loss, while symptoms of type 2 diabetes are fatigue and obesity. Once diabetes is diagnosed, medication is required for treatment. It is recommended to select regular drugs such as metformin, glidazide or insulin for conditioning and treatment. In addition, you need to pay attention to your diet. For example, do not eat sweets such as sweet fruits and pastries, eat more foods rich in vitamins and fiber, limit the intake of cured, baked, smoked, marinated meat products, and drink plenty of water every day | 1 | 1 | Give a like |
| Can people with diabetes drink tea? | Hello, people with diabetes can drink tea. Tea has the effect of lowering blood lipid, which is good for reducing the risk of cardiovascular disease of patients with diabetes | 1 | 1 | Give a like |
| Is diabetes terrible? because I just got it, and I read about diabetes, and it has a great psychological impact | Hello, diabetes is a metabolic disease caused by the body's abnormal glucose metabolism. Currently, diabetes cannot be cured. However, the patients with diabetes may use oral hypoglycemic drugs such as saxagliptin and metformin for treatment, or follow the doctor's advice to inject insulin to control the blood glucose. Moreover, the diet should be light, and the patients with diabetes should control the intake of sugar, eat more fresh vegetables, and exercise to control diabetes. If you can control the blood glucose well and maintain a good attitude, you can also enjoy life happily | 1 | 0 | Give a like |
| Can people with diabetes eat cantaloupe? | Hello, diabetes is a metabolic disease caused by the body's abnormal glucose metabolism. Foods with high sugar content should be avoided. Fruits have low sugar content and have a variety of minerals and aminotransferases that are beneficial to the human body. Therefore, people with diabetes can eat fruits such as cantaloupe. However, the intake should not be too large, and the blood glucose should be controlled with drugs | 1 | 0 | Give a like |

130 results in total  [5 results/page∨]   [<] [1] [2] [3] [4] [5] [6] [...][26][>]  Go to [    ] page

FIG. 19

002

| Follow-up management ✕ | | Follow-up comparison ✕ | Consultation management ✕ | | | | | 901 | More |
|---|---|---|---|---|---|---|---|---|---|
| ZHAO Si | Male | 59 years old | Image and text consultation | 2020:04:02 14:10 | 111 | | Yes | Answered | Details |
| ZHANG San | Female | 77 years old | Report interpretation | 2020:03:30 16:53 | The physical examination report came out today, and there are many problems. Please interpret the report. | 1. Abnormal question summary: reexamination through B-mode ultrasound; blood routine is abnormal, but there is no major problem; there is no need to worry too much about sinus arrhythmia, and most people have sinus arrhythmia in physical examination; and blood lipid is too high, and should be controlled reasonably. 2. Health advice: Medication regimen: acarbose 50 mg: | Yes | Answered | Details |
| LI Xiao ming | Female | 56 years old | Report interpretation | 2020:03:30 16:49 | There are many problems in the physical examination report. Please interpret the report. | 1. Abnormal question summary: 18.5-23.9 (normal) ≥ 24, <32.5 (overweight or obese) ≥ 32.5 severely overweight or obese. 2. Health advice: Medication regimen: acarbose 50 mg tid: Metformin 0.5 mg tid | Yes | Answered | Details |
| Mr. LI | Male | 59 years old | Image and text consultation | 2020:03:30 16:49 | Laboratory indicators are abnormal | | Yes | Answered | Details |
| Mr. WANG | Male | 59 years old | Image and text consultation | 2020:03:30 16:49 | Feel dizzy | I have felt dizzy for several days. I don't know what's going on, and I don't have a cold | Yes | Answered | Details |
| ZHAO X | Male | 59 years old | Image and text consultation | 2020:03:30 16:49 | Have a fever | I have had a fever of 39° for 2 consecutive days, and the anti-fever medicine does not work. What should I do? | No | Answered | Details |
| ZHANG Yi | Male | 77 years old | Report interpretation | 2020:03:30 16:49 | Please interpret the report in detail | | Yes | Answered | Details |

FIG. 20

003

<table>
<tr><td colspan="3">☐ Follow-up management ✕ | Consultation management ✕ | Image-text consultation details ✕ | [More]</td></tr>
</table>

Question  Abnormal laboratory indicators

attachment...                                                2020-03-30 16:33

Answer  Leukocytes are important immune cells in the human body and play a role in eliminating antigens. Increased leukocyte count is common in infections, leukemia and other diseases. Leukocytes are classified into neutrophils, lymphocytes, eosinophils, basophils, and monocytes. Clinically, different diseases may be diagnosed based on a difference in classification ratio. For example, if increase of leukocytes is dominated by neutrophils, a possibility of infection is high. If the increase of leukocytes is dominated by eosinophils, allergies are common

Reply to the expert:                                        2020-03-30 16:37

Question  Is that true?                                     2020-03-30 16:41

Answer  Platelets play roles of hemostasis and coagulation. Reduction of platelets may lead to various bleeding diseases, such as menorrhagia, gastrointestinal bleeding, bruises of skin and mucous membranes, and bleeding gums. It suggests bone marrow hematopoietic abnormality, hyperthyroidism and other diseases. Increase of platelets may easily cause thrombosis, which is common in blood diseases such as thrombocytosis and multiple bone marrow

Reply to the expert:                                        2020-03-30 16:42

FIG. 21

| Follow-up management ✕ | Patient management ✕ | Knowledge base maintenance ✕ | Consultation management ✕ | Report interpretation ✕ | | More |

Note: There are too many abnormal indicators in the health examination. Please interpret the report. I am worried that there are other diseases of the body. Content of the general examination cannot be understood. Please use easy-to-understand words.

### Report

0041

### Interpretation

**Abnormal question summary** — 0042

After reexamination through abdominal B-mode ultrasound, it is found that blood routine is abnormal, but there is no main problem; there is no need to worry too much about sinus arrhythmia, and most people have sinus arrhythmia in physical examination; and blood lipid is too high, and should be reasonably controlled

**Health advice**

Medication regimen:
Acarbose 50mg tid;
Metformin 0.5mg tid;
Glitazone 30mg tid;

Diet regimen: — 0043

### Assistant region

| Abnormal question | Template classification | Template content | Reference all |

Hypertension
Stage 1 hypertension
Stage 2 hypertension
Stage 3 hypertension
Diabetes
Hyperlipidemia
Osteoarthritis
Coronary heart disease
Rheumatoid arthritis
Chronic obstructive pulmonary disease
Cerebrovascular disease
Bronchitis
Pharyngitis

Health advice

Template Entry

**Medication regimen**

Acarbose 50 mg tid; Metformin 0.5 mg tid; Glitazone 30 mg tid    Reference

**Diet regimen**

Low-salt and low-fat diet    Reference

**Exercise regimen**

Aerobic exercise, resistance exercise, flexibility exercise, balance function, etc.    Reference

**Others**

## FIG. 22

EP 4 068 292 A1

005    0051



| Follow-up management | Patient management | Consultation management | Report interpretation | Knowledge base maintenance | More |

**Abnormal question**

**Health advice**

Template classification | Add a node

| Add | Edit | Delete | Save |

Hypertension

Stage 1 hypertension

Stage 2 hypertension

Stage 3 hypertension

Template

Entry

Diabetes

Cerebrovascular disease

Chronic obstructive pulmonary disease

Rheumatoid arthritis

Bronchitis

Pharyngitis

Hyperlipidemia

Osteoarthritis

Coronary heart disease

**Medication regimen**

Acarbose 50 mg tid; Metformin 0.5 mg tid; Glitazone 30 mg tid

**Diet regimen**

Low-salt and low-fat diet: (1) the Chinese Nutrition Society recommends that daily salt intake for adults should not exceed 6 g, and daily salt intake for patients with hypertension should exceed 3 g (specifically, salt intake per person per meal does not exceed 2 g, that is, salt intake per person per day does not exceed 6 g, i.e., a bottle cap of salt after a rubber pad is removed from an ordinary beer bottle cap); (2) daily cooking oil consumption < 25 g (half a tael, equivalent to 2.5 tablespoons of cooking oil)

**Exercise regimen**

Exercise: exercises that the patients with hypertension may select include aerobic exercise, resistance exercise, flexibility exercise, and balance function. It should be noted that, blood pressure of the patients with hypertension is usually at a high level in the early morning, and the early morning is a period with high incidence of cardiovascular events. Therefore, it is best to exercise in the afternoon or evening. In addition, there should be 5 to 10 minutes of warm-up exercise and relaxation exercise before and after formal exercise to avoid sudden strenuous exercise

**Others**

Ensure adequate sleep and select a healthy life

FIG. 23

006

**Statistics**

0061

| Gender and age distribution | Disease type statistics | Health prescription statistics | Follow-up performance statistics |

0062

☐ All | Start time 🗓 | Finish time 🗓 | Query

Statistics of the numbers of
people with disease types

100
80
60
40
20
0

Coronary heart disease | Chronic obstructive pulmonary disease | Diabetes | Cerebrovascular disease | Hypertension | Hypertension, diabetes | Hyperlipidemia

FIG. 24

**Statistics**

0061 — | Gender and age distribution | Disease type statistics | Health prescription statistics | Follow-up performance statistics |

0062 —

| Start time 📅 | Finish time 📅 | Prescribing doctor | Prescribing doctor ▾ | 0063 |

104 —

☐Hypertension ☐ Diabetes ☐Hypertension ☐Osteoarthritis ☐ Coronary heart disease ☐ Rheumatoid arthritis ☐ Chronic obstructive pulmonary disease ☐Cerebrovascular disease ☐Bronchiolitis | Query |

| Prescribing doctor | Disease type | Number of prescriptions |
|---|---|---|
| Dr. ZHANG | Diabetes | 8 |
| Dr. ZHANG | Hypertension | 24 |
| Dr. ZHANG | Hypertension, diabetes | 102 |
| Dr. ZHANG | Hyperlipidemia | 1 |
| MAO Mao yu | Hypertension, diabetes | 1 |
| Total | | 136 |

FIG. 25

| Statistics | | | 0061 | Gender and age distribution | Disease type statistics | Health prescription statistics | Follow-up performance statistics |
|---|---|---|---|---|---|---|---|

0062

Start time 📅     Finish time 📅     Performing doctor [ Performing doctor ▾ ] — 0064

— 104

☐ Hypertension ☐ Diabetes ☐ Hypertension ☐ Osteoarthritis ☐ Coronary heart disease ☐ Rheumatoid arthritis ☐ Chronic obstructive pulmonary disease ☐ Cerebrovascular disease ☐ Bronchiolitis   [ Query ]

| Performing doctor | Disease type | Performed | Performed after expiration | Unperformed after expiration | Unperformed |
|---|---|---|---|---|---|
| Dr. ZHANG | Hypertension | 1 | 21 | 50 | 0 |
| Dr. ZHANG | Cerebrovascular disease | 0 | 0 | 14 | 0 |
| Dr. ZHANG | Hypertension | 2 | 9 | 88 | 0 |
| Dr. ZHANG | Hypertension, diabetes | 23 | 58 | 266 | 0 |
| MAO Mao yu | Hypertension | 0 | 0 | 7 | 1 |
| Total | | 26 | 88 | 425 | 1 |

FIG. 26

**Statistics**

0061 — Gender and age distribution | Disease type statistics | Health prescription statistics | Follow-up performance statistics

0062 —

☐ All | Start time 🗓 | Finish time 🗓 | Query

Gender distribution

Age distribution

Male

Female

0-3 years old
Number of patient:1

| | 0-3 years old | 3-10 years old | 18-45 years old | 45-60 years old | 60-75 years old | 75-90 years old |
|---|---|---|---|---|---|---|
| | 1 | 3 | 75 | 12 | 33 | 1 |

FIG. 27

EP 4 068 292 A1

87

006

User management condition

0.84%
3.36%
95.8%

| First-time management | Continuous management | Unmanaged |
|---|---|---|
| 1 First-time management (person) | 4 Continuous management (person) | 114 Unmanaged (person) |
| Nearly one week | Nearly one month | Nearly one year |

FIG. 28

Display a plurality of role selection items, the plurality of role selection items including a medical staff and a patient ⎯⎯ S001

In response to an operation of the user of selecting a role selection item, determine a role of the user, and display a login information box corresponding to the role of the user, login information boxes corresponding to different roles being different ⎯⎯ S002

Display at least one function tab corresponding to the role of the user in response to login information of the user entered in the login information box, at least one function tab corresponding to the medical worker being configured to instruct to display management information of diseases of a plurality of patients, and at least one function tab corresponding to the patient being configured to instruct to display management information of a disease of the patient ⎯⎯ S003

FIG. 29

P1

FIG. 30

P2

●●●●● WeChat 11:02 96%

‹ Doctor terminal ●●● ◎

○ Manage your health
● We are dedicated
We are professional ○

● ● ●

| Follow-up performanc | Regimen recommendation |
|---|---|
| Follow-up statistics | Disease assessment |
| Smart genius (FAO) | |

A2

| My patient | My schedule |

FIG. 31A

P3

●●●●● WeChat 16:08 96%

‹ Patient terminal ●●● ◎

○ Manage your health
● We are dedicated
We are professional ○

● ● ●

A3

| Disease assessment | Smart genius (FAO) |
|---|---|
| My follow-up | Regimen recommendation |
| Follow-up statistics | |

| On-line famous doctor ***** | Report interpretation ***** |

FIG. 31B

P4

•••••WeChat 📶     23:37     95% 🔋

<    **My schedule**    •• •  •

A6

<    2020-04-13    >    Today

| Thursday | Friday | Saturday | Sunday | Monday | Tuesday | Wednesday | Thursday | Friday |
|----------|--------|----------|--------|--------|---------|-----------|----------|--------|
| 9 | 10 | 11 | 12 | 13 | 14 | 15 | 16 | 17 |

A4

| Image-text consultation ① | Report interpretation ⓪ | Follow-up task ② |

| 08:55 AM | Abnormal item in a laboratory result   Mr. XIE |

| 00:00 AM | Follow-up service record form for a patient with diabetes Please come to the hospital for follow-up today | X1 |

| 00:00 AM | Follow-up service record form for a patient with hypertension Please come to the hospital for follow-up today | X8 |

A5

FIG. 32

P4

FIG. 33

P5

●●●●● WeChat 🛜　　　15:50　　　96% ▮▮▮

< 　　　Data statistics　　　⊙

0% ⎯ ⎤ ⎡ ⎯ 0%

33.33% ⎯

66.67%

| | Satisfactory control | 66.67% | 0 |
|---|---|---|---|
| | Unsatisfactory control | 33.33% | 2 |
| | Adverse reaction | 0% | 1 |
| | Complication | 0% | 0 |

FIG. 34

P6

•••••WeChat📶      15:41      96% ▮▶

<      Disease
assessment     ••• ◉

Assessment
system

Are you making an
assessment for yourself?

Myself

What is an age of a
person to be tested?

Are you making an
assessment for yourself?

30

What is a gender of the
person to be tested?

Female

What is a height of the
person to be tested?

FIG. 35

P7

**75**%
Risk of diabetes

High risk of diabetes          The risk is 75%    ↑

Body index                                    ☰

Fasting blood glucose
 Normal range is              **5.75** ↑
  3.9 to 5.6
   mmol/L

 Body mass
 index (BMI)                  **78.4**
 Normal range is
  50 to 65 kg

Blood pressure             ↑ **160/90** ↓
Normal range
is  50 to 65 kg

Expert's advice                              ✂

NO.1 Improve lifestyle
Eat a reasonable diet, control fat intake, exercise
moderately, make weight reach the standard, quit
smoking and alcohol, and keep a mental balance

FIG. 36

P8

●●●●● WeChat    📶    15:41      96% ▭

⟨      BOE Health      •●•   ⊙

## FAQ smart question and answer

Please enter your question    🔍

Are the following questions you want to ask?

What are symptoms of diabetes and can it be treated? What medicine should be taken, or what can be eaten and what cannot be eaten in daily life#      unfolded

Hello, main symptoms of type 1 diabetes are "three more and one less" and polydipsia, polyuria, polyphagia and weight loss, while symptoms of type 2 diabetes are fatigue and obesity, ...

1 person asked this question|12 persons give likes

#What are problems with an oral cavity of a patient with diabetes?#      unfolded

Hello, in terms of the oral cavity, in the past, everyone did not pay much attention to oral care, however, in recent years, everyone has paid great attention to the oral cavity of the patient with diabetes. We all know ...

1 person asked this question|1 person gives a like

#Can liver diseases be perfectly cured? Can diabetes be cured?#      unfolded

Hello, the therapeutic effect of this health care product on diabetes is not yet clear. It is recommended to ask the dealer. Scientific medical treatment can better treat and control the onset and development of diabetes, ...

1 person asked this question|1 person gives a like

FIG. 37

## P9

●●●●●WeChat 📶     23:44     90% 🔋

‹     Image-text
consultation     ( ●●●   ⊙ )

Professional **Image-text**

**consultation**
Just for your healthy life

Fill in patient information
Recommend a doctor

★   Patient:        Other patients   ›

★ disease condition description: (enter at least 10 words)

Please elaborate your disease condition and symptoms, examination, medication condition, and whether the disease condition is aggravated or relieved currently ...

Add attachment (not required)
Please upload a picture or a document such as an examination result or a medical record

Please select
an expert

submit

FIG. 38

P10

Question
details

<     •●••   ◉

2020-03-30 16:30

2020-03-30 16:30

Leukocytes are important immune cells in the human body and play a role in eliminating antigens. Increased leukocyte count is common in infections, leukemia and other diseases. Leukocytes are classified into neutrophils, lymphocytes, eosinophils, basophils, and monocytes. Clinically, different diseases may be diagnosed based on a difference in classification ratio. For example, if increase of leukocytes is dominated by neutrophils, a possibility of infection is high. If the increase of leukocytes is dominated by eosinophils, allergies are common

2020-04-01 14:46       null

123
2020-04-01 14:46null

Inquiry

FIG. 39

E1

First display — E1-1

First processor — E1-2

First input device — E1-3

FIG. 40

E2

Second display — E2-1

Second processor — E2-2

Second input device — E2-3

FIG. 41

B100

B2

B2

B1

**Follow-up service record form for a patient with hypertension**

| Follow-up date: | Select a date and time 📅 | | Follow-up way: | Please select |
|---|---|---|---|---|

B2

| Symptom | | | Please select | ∨ |
|---|---|---|---|---|
| ☐ No symptom | ☐ Headache and dizziness | | ☐ Nausea and vomiting | |
| ☐ Giddiness and tinnitus | ☐ Difficult breathing | | ☐ Palpitations and chest tightness | |
| ☐ Epistaxis | ☐ Numbness of limbs | | ☐ Lower limb edema | |
| Others: | | | | |

| Sign | | | ∨ |
|---|---|---|---|
| | Current value | Target value to be adjusted during next follow-up | |
| Height (m) | | | |
| Weight(kg) | | | |

B2

| | | | |
|---|---|---|---|
| Body mass index (BMI) (kg/m²) | | | |
| Heart rate(times/minute) | | | |
| Others | Please fill in other positive signs | | |

| Lifestyle guidance | | ∨ |
|---|---|---|
| | Current condition | Target value to be adjusted during next follow-up |
| Daily smoking consumption | | |
| Daily alcohol consumption (tael) | | |
| Exercise | Please select ▾ _____ minutes/time | Please select ▾ _____ minutes/time |
| Salt intake condition (salty & mild) | ○ Light  ○ Medium  ○ Heavy | Light  ○ Medium  ○ Heavy |
| Psychological adjustment | ○ Good  ○ Fair  ○ Poor | |
| Treatment compliance | ○ Good  ○ Fair  ○ Poor | |
| Auxiliary examination | | |
| Medication compliance | ○ Regular  ○ Intermittent  ○ Not taking medicine | |
| Adverse drug reaction | ○ No  ○ Yes  Please enter adverse reaction | |
| Category of this follow-up | ○ Satisfactory control  ○ Unsatisfactory control  ○ Adverse reaction  ○ Complication<br>Note: | |

| Medication condition | | | | | ∨ |
|---|---|---|---|---|---|
| | Drug name | Usage | Frequency | Dosage per time | Operation |
| This prescription | Drug name 1 | | | | 🗑 |
| | Drug name2 | | | | 🗑 |
| | Drug name 3 | | | | 🗑 ⊕ |
| Prescriptions from other institutions | | | | | |

| Referral | | | ∨ |
|---|---|---|---|
| Reason | | | |
| Referral institution | | Referral department | |
| Next follow-up date | Please select 📅 | Signature of a follow-up doctor | |

FIG. 42A

B100

B2

B2

B1

**Follow-up service record form for a patient with hypertension**

| Follow-up date: | 2018-12-04 11:34:25 📅 | Follow-up way: | Outpatient clinic ▾ |
|---|---|---|---|

B2

| Symptom | | | ⌄ |
|---|---|---|---|
| ☐ No symptom | ☑ Headache and dizziness | ☑ Nausea and vomiting | |
| ☐ Giddiness and tinnitus | ☐ Difficult breathing | ☐ Palpitations and chest tightness | |
| ☐ Epistaxis | ☐ Numbness of limbs | ☐ Lower limb edema | |
| Others: | | | |

B2

| Sign | | ⌄ |
|---|---|---|
| | Current value | Target value to be adjusted during next follow-up |
| Height (m) | 1.76 | |
| Weight(kg) | 90 | 87 |
| Body mass index (BMI) (kg/m²) | 28.9 | 26.7 |
| Heart rate(times/ minute) | 78 | |
| Others | Please fill in other positive signs | |

| Lifestyle guidance | | ⌄ |
|---|---|---|
| | Current condition | Target value to be adjusted during next follow-up |
| Daily smoking consumpotion | 7 | 3 |
| Daily alcohol consumption (tael) | 3 | 1 |
| Exercise | 5 times/week to 6 times/week ▾ _____ minutes/time | 5 times/week to 6 times/week ▾ _____ minutes/time |
| Salt intake condition (salty & mild) | ○ Light  ○ Medium  ⦿ Heavy | ○ Light  ⦿ Medium  ○ Heavy |
| Psychological adjustment | ○ Good  ○ Fair  ⦿ Poor | |
| Treatment compliance | ○ Good  ○ Fair  ⦿ Poor | |
| Auxiliary examination | | |
| Medication compliance | ○ Regular  ○ Intermittent  ⦿ Not taking medicine | |
| Adverse drug reaction | ○ No  ⦿ Yes  Please enter adverse reaction | |
| Category of this follow-up | ○ Satisfactory control  ○ Unsatisfactory control  ⦿ Adverse reaction  ○ Complication | Note: |

| Medication condition | | | | | ⌄ |
|---|---|---|---|---|---|
| This prescription | Drug name | Usage | Frequency | Dosage per time | Operation |
| | Drug name 1 | | QD | | 🗑 |
| | Drug name 2 | | QD | | 🗑 |
| | Drug name 3 | | BID | | 🗑 ⊝ |
| Prescriptions from other institutions | | | | | |

| Referral | | | ⌄ |
|---|---|---|---|
| Reason | | | |
| Referral institution | | Referral department | |
| Next follow-up date | 2019-01-04 📅 | Signature of a follow-up doctor | XXX |

FIG. 42B

B100

B2        B2        B1

### Follow-up service record form for a patient with diabetes

| Follow-up date: | 2018-12-04 11:34:25 📅 | Follow-up way: | Outpatient clinic |

**Symptom** ⌄

| ☐ No symptom | ☑ Polydipsia | ☑ Polyphagia |
| ☐ Polydipsia | ☐ Blurring of vision | ☐ Infection |
| ☐ Numbness of limbs | ☐ Lower limb edema | ☐ Significant weight loss |

Others: _____

B2

**Sign** ⌄

| | Current value | Target value to be adjusted during next follow-up |
|---|---|---|
| Blood pressure (mmHg) | 160 / 105 | |
| Weight(kg) | 180 | 174 |
| Body mass index (BMI) (kg/m²) | 28.9 | 26.7 |
| Pulsation of dorsal artery of foot | ○ Normal  ○ Weaken [Bilateral / Left side / Right side] | ○ Disappear [Bilateral / Left side / Right side] |
| Others | Please fill in other positive signs | |

**Lifestyle guidance** ⌄

| | Current condition | Target value to be adjusted during next follow-up |
|---|---|---|
| Daily smoking consumption | 7 | 3 |
| Daily alcohol consumption (tael) | 3 | 1 |
| Exercise | 5 times/week to 6 times/week ____ minutes/time | 5 times/week to 6 times/week ____ minutes/time |
| Staple food (g/day) | 1000 | 800 |
| Psychological adjustment | ○ Good  ○ Fair  ◉ Poor | |
| Treatment compliance | ○ Good  ○ Fair  ◉ Poor | |
| Auxiliary examination | | |
| Medication compliance | ○ Regular  ○ Intermittent  ◉ Not taking medicine | |
| Adverse drug reaction | ○ No  ◉ Yes  Please enter adverse reaction | |
| Category of this follow-up | ○ Satisfactory control  ○ Unsatisfactory control  ◉ Adverse reaction  ○ Complication  Note: ____ | |

**Medication condition** ⌄

| | Drug name | Usage | Frequency | Dosage per time | Operation |
|---|---|---|---|---|---|
| This prescription | Drug name1 | | QD | | 🗑 |
| | Drug name 2 | | QD | | 🗑 |
| | Drug name 3 | | BID | | 🗑 ⊕ |
| Prescriptions from other institutions | | | | | |

**Referral** ⌄

| Reason | |
|---|---|
| Referral institution | | Referral department | |
| Next follow-up date | 2019-01-04 📅 | Signature of a follow-up doctor | XXX |

## FIG. 42C

B200

B1

B8

B81

Follow-up
management ×

Follow-up task
performance ×

Scale making tool ×

Patient
management ×

Follow-up
creation ×

Follow-up
template ×

Scale making tool

Frequently-used field

Basic field

B31a(31)

Single-line text box | Multi-line text box

Counter | Single-selection box group

Multi-selection box group | Time selector

Date selector | Score

Color selector | Drop-down selection box

Switch | Slider

B3

⊞ View | 🔒 Empty | ✏ Preview | ⊞ Generate a form | B7

More∨

B4

FIG. 43A

FIG. 43B

B200

B1

B8

Follow-up management | Follow-up task performance | Scale making tool × | Patient management × | Follow-up creation × | Follow-up template ×

Scale making tool

View | Empty | Preview | Generate a form — B7

Field attribute | Form attribute — B51

Frequently-used field B31b(31)
Public frequently-used field
Follow-up date | Follow-up way
Symptom
Private frequently-used field
Blood pressure

Basic field B31a(31)
Single-line text box | Multi-line text box
Counter | Single-selection box group
Multi-selection box group | Time selector
Date selector | Score
Color selector | Drop-down selection box
Switch | Slider

B3

Follow-up date

Drop-down selection box

Title
Follow-up way
Description

Default prompt text
Please select

Option content
Outpatient clinic, telephone, network, short message, and others | Generated element
Public element

Filling-in setup
Required item | Private element
Limiting the number of words
Using regular check

Data source

Save as a frequently used field

Save

B5

B4

104

FIG. 43C

FIG. 43D

B1  B200  B8

| | Follow-up management × | Follow-up task performance × | Scale making tool × | Patient management × | Follow-up creation × | Follow-up template × | | More ∨ |

| Editor | Cascade selector |
|---|---|
| Layout field | |
| Grid layout | |

Weight (kg):

Body mass index (BMI):

Others:

Next target value:

Next target amount:

Vertical arrangement

Save

Made form    B3          B4                          B6              B62a        B5

| Serial number | Form name | Form type | Making time | Operation |
|---|---|---|---|---|
| 123456789456789bvc | Follow-up service record form 6 for a patient with hypertension | | 2020-05-20 17:32:53 | Import and reference  Edit  Delete |
| 123452389456789yhn | Follow-up service record form 7 for a patient with hypertension | 111 | 2020-05-27 14:23:53 | Import and reference  Edit  Delete |
| 123456789236789thn | Coronary heart disease follow-up form | 22 | 2020-06-10 10:24:34 | Import and reference  Edit  Delete |
| 123223678945678 9mjh | Stroke follow-up form | 1 | 2020-06-10 17:32:53 | Import and reference  Edit  Delete |
| 123456789452589bvc | Prenatal follow-up form | 222 | 2020-06-11 10:19:14 | Import and reference  Edit  Delete |
| 123456787556789bvc | Diabetes follow-up form | 222 | 2020-06-18 14:00:47 | Import and reference  Edit  Delete |
| 12345989456784bvc | Health assessment form | 222 | 2020-06-23 16:47:06 | Import and reference  Edit  Delete |
| 123456473256789bvc | Diabetes follow-up form | c | 2020-06-24 17:28:40 | Import and reference  Edit  Delete |

B61                                                                      B62

FIG. 43E

FIG. 43F

FIG. 43G

EP 4 068 292 A1

EP 4 068 292 A1

B500

B81   B8

admincdy

B1

Follow-up management ✕ | Follow-up task performance ✕ | Scale making took | Patient management ✕ | Follow-up creation ✕ | Follow-up template ✕

B11

Follow-up template   B13

B14

Creation

Template classification   Add a top node

B14b

Hyperlipidemia

* Time interval   21

New

Diabetes

* Follow-up form   Life quality form for a patient with hypertension

Hypertension   B13a

B14a   Cancel   OK

Operation

Stage 1 hypertension

Edit  Dele

Stage 2 hypertension

Edit  Dele

Stage 3 hypertension

7 | Follow-up service record form for a patient with hypertension | Edit  Dele

Coronary heart disease

14 | Follow-up service record form for a patient with hypertension | Edit  Dele

1 | Follow-up service record form for a patient with hypertension | Edit  Dele

7 | Follow-up service record form for a patient with diabetes | Edit  Dele

14 | Follow-up service record form for a patient with hypertension | Edit  Dele

B15

FIG. 44A

B500
B1
B81
B8

admincdyy

Follow-up management✕ | Follow-up task performance✕ | Scale making tool ✕ | Patient management✕ | Follow-up creation ✕ | Follow-up template ✕

Follow-up template B13

Template classification | Add a top node

Hyperlipidemia

Diabetes

Hypertension ⌄

Stage 1 hypertension

Stage 2 hypertension

Stage 3 hypertension

Coronary heart disease

B13a

Template content | New

| Time interval | Follow-up form | Operation | |
|---|---|---|---|
| 1 | Follow-up service record form for a patient with diabetes | Edit | Delete |
| 1 | Follow-up service record form for a patient with hypertension | Edit | Delete |
| 7 | Follow-up service record form for a patient with hypertension | Edit | Delete |
| 14 | Follow-up service record form for a patient with hypertension | Edit | Delete |
| 1 | Follow-up service record form for a patient with hypertension | Edit | Delete |
| 7 | Follow-up service record form for a patient with diabetes | Edit | Delete |
| 14 | Follow-up service record form for a patient with hypertension | Edit | Delete |
| 21 | Follow-up service record form for a patient with hypertension | Edit | Delete |

B15

FIG. 44B

Follow-up service record
form for a patient with
hypertension

Name

[                              ]

- - - - - - - - - - - - - - - -
Date of birth

Select a date 📅

- - - - - - - - - - - - - - - -
Follow-up date

Select a date 📅

- - - - - - - - - - - - - - - -
Follow-up way

FIG. 45

## INTERNATIONAL SEARCH REPORT

International application No.

**PCT/CN2020/131561**

**A. CLASSIFICATION OF SUBJECT MATTER**

G16H 10/00(2018.01)i

According to International Patent Classification (IPC) or to both national classification and IPC

**B. FIELDS SEARCHED**

Minimum documentation searched (classification system followed by classification symbols)

G06Q;G06F

Documentation searched other than minimum documentation to the extent that such documents are included in the fields searched

Electronic data base consulted during the international search (name of data base and, where practicable, search terms used)

CNABS; SIPOABS; DWPI; CNTXT; WOTXT; EPTXT; USTXT; CJFD; CNKI: 导航, 检索, 搜索, 搜寻, 索引, 页签, 列表, 医护, 医疗, 患者, 医院, 诊断, 疾病, 治疗, 编辑, 显示, 展示, 级别, 等级, 分类, 子, 操作, 输入, 匹配, 比较, 模板, 字段, 目标, 表单, navigate, search, retrieve, index, page, tab, list, doctor, nurse, medical, treatment, hospital, diagnose, disease, edit, display, show, level, grad, type, son, sub, operate, input, match, compare, model, field, object, menu, form, table

**C. DOCUMENTS CONSIDERED TO BE RELEVANT**

| Category* | Citation of document, with indication, where appropriate, of the relevant passages | Relevant to claim No. |
|---|---|---|
| X | CN 102576376 A (COHN, STEVEN, CHARLES) 11 July 2012 (2012-07-11) description, paragraphs [0071]-[0079], [0088]-[0091], [0108]-[0109], [0150]-[0151] | 1-70 |
| X | CN 109461476 A (NANJING MEDICAL UNIVERSITY SIR RUN RUN HOSPITAL) 12 March 2019 (2019-03-12) description, paragraphs [0431]-[0441] | 1-70 |
| A | CN 107066812 A (SHENZHEN QIANHAI HALENTS LIFE-INFO TECHNOLOGY CO., LTD.) 18 August 2017 (2017-08-18) entire document | 1-70 |
| A | US 2017116373 A1 (GINSBURG LEONARD et al.) 27 April 2017 (2017-04-27) entire document | 1-70 |
| A | US 2014249855 A1 (AIRSTRIP IP HOLDINGS LLC.) 04 September 2014 (2014-09-04) entire document | 1-70 |
| A | US 2014337051 A1 (KARPF RONALD STEVEN) 13 November 2014 (2014-11-13) entire document | 1-70 |

☐ Further documents are listed in the continuation of Box C.  ☑ See patent family annex.

\* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **23 February 2021** | **03 March 2021** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088 China** | |
| Facsimile No. **(86-10)62019451** | Telephone No. |

Form PCT/ISA/210 (second sheet) (January 2015)

INTERNATIONAL SEARCH REPORT
Information on patent family members

International application No.

**PCT/CN2020/131561**

| Patent document cited in search report | | | Publication date (day/month/year) | Patent family member(s) | | | Publication date (day/month/year) |
|---|---|---|---|---|---|---|---|
| CN | 102576376 | A | 11 July 2012 | SG | 177547 | A1 | 28 February 2012 |
| | | | | US | 2014108048 | A1 | 17 April 2014 |
| | | | | US | 2011010195 | A1 | 13 January 2011 |
| | | | | JP | 2012533117 | A | 20 December 2012 |
| | | | | WO | 2011005632 | A1 | 13 January 2011 |
| | | | | BR | 112012000516 | A2 | 24 September 2019 |
| | | | | KR | 20120058510 | A | 07 June 2012 |
| | | | | AU | 2010270855 | A1 | 01 March 2012 |
| | | | | CA | 2767237 | A1 | 13 January 2011 |
| | | | | EP | 2452285 | A1 | 16 May 2012 |
| | | | | ZA | 201200947 | A | 29 May 2013 |
| | | | | MX | 2012000349 | A | 30 April 2012 |
| | | | | IN | 201201191 | P1 | 02 November 2012 |
| | | | | IL | 217398 | A | 28 February 2013 |
| CN | 109461476 | A | 12 March 2019 | None | | | |
| CN | 107066812 | A | 18 August 2017 | None | | | |
| US | 2017116373 | A1 | 27 April 2017 | US | 10685743 | B2 | 16 June 2020 |
| | | | | CA | 3076349 | A1 | 29 March 2018 |
| | | | | WO | 2018057918 | A1 | 29 March 2018 |
| US | 2014249855 | A1 | 04 September 2014 | None | | | |
| US | 2014337051 | A1 | 13 November 2014 | None | | | |

Form PCT/ISA/210 (patent family annex) (January 2015)

**REFERENCES CITED IN THE DESCRIPTION**

**Patent documents cited in the description**

- CN 201911169008X
- CN 202011063268
- CN 202011331863